# EUROPEAN PATENT SPECIFICATION

(11) **EP 3 237 377 B1**
(45) Date of publication and mention of the grant of the patent: **26.02.2025**
(21) Application number: 15872293.4
(22) Date of filing: 24.12.2015
(51) Int. Cl.: C07D 211/46, C07C 401/00, C07D 495/04, C07D 209/48, A61P 43/00, A61P 35/00, A61P 3/04, A61P 1/16, A61K 31/593, A61K 31/59, C07D 295/125, A61P 3/10, A61P 9/00

(54) **VITAMIN D3 DERIVATIVES AND PHARMACEUTICAL USE THEREOF**
VITAMIN-D3-DERIVATE UND RE PHARMAZEUTISCHE VERWENDUNG DAVON
DÉRIVÉS DE LA VITAMINE D3 ET LEUR UTILISATION PHARMACEUTIQUE

(30) Priority: 24.12.2014 US 201462096575 P
(43) Date of publication of application: 01.11.2017
(73) Proprietor: Kyoto University, Kyoto-shi, Kyoto 606-8501 (JP); National University Corporation Tokyo University of Agriculture and Technology, Fuchu-shi, Tokyo 183-8538 (JP)
(72) Inventor: WATANABE, Mizuki, Kyoto-shi Kyoto 606-8501 (JP); ASANO, Risa, Kyoto-shi Kyoto 6068501 (JP); NAGASAWA, Kazuo, Fuchu-shi Tokyo 183-8538 (JP); UESUGI, Motonari, Kyoto-shi Kyoto 606-8501 (JP)
(74) Representative: Hoffmann Eitle
(86) International application number: PCT/JP2015/006462
(87) International publication number: WO 2016/103722

(56) References cited:
- WO-A1-2013/154752
- WO-A1-90/10620
- CN-A- 103 585 632
- JP-A- S6 064 924
- GLEBOCKA AGNIESZKA ET AL: "A-ring analogs of 1,25-dihydroxyvitamin D3", ARCHIVES OF BIOCHEMISTRY AND BIOPHYSICS, vol. 523, no. 1, 2012, pages 48 - 57, XP028924119, ISSN: 0003-9861, DOI: 10.1016/J.ABB.2011.11.010
- DANIEL OVES ET AL: "Efficient Synthesis of Novel 1[alpha]-Amino and 3[beta]-Amino Analogues of 1[alpha],25-Dihydroxyvitamin D 3", JOURNAL OF ORGANIC CHEMISTRY, vol. 68, no. 3, 1 February 2003 (2003-02-01), US, pages 1154 - 1157, XP055491192, ISSN: 0022-3263, DOI: 10.1021/jo026474t
- YI YIN ET AL: "Vitamin D attenuates high fat diet-induced hepatic steatosis in rats by modulating lipid metabolism : VITAMIN D ATTENUATES HIGH FAT DIET-INDUCED HEPATIC STEATOSIS IN RATS", EUROPEAN JOURNAL OF CLINICAL INVESTIGATION, vol. 42, no. 11, 8 September 2012 (2012-09-08), GB, pages 1189 - 1196, XP055490943, ISSN: 0014-2972, DOI: 10.1111/j.1365-2362.2012.02706.x
- MING KONG ET AL: "Vitamin D deficiency promotes nonalcoholic steatohepatitis through impaired enterohepatic circulation in animal model", AMERICAN JOURNAL OF PHYSIOLOGY - GASTROINTESTINAL AND LIVER PHYSIOLOGY, vol. 307, no. 9, 1 November 2014 (2014-11-01), US, pages G883 - G893, XP055490934, ISSN: 0193-1857, DOI: 10.1152/ajpgi.00427.2013
- JUAN KONG ET AL: "Molecular mechanism of 1,25-dihydroxyvitamin D 3 inhibition of adipogenesis in 3T3-L1 cells", AMERICAN JOURNAL OF PHYSIOLOGY: ENDOCRINOLOGY AND METABOLISM., vol. 290, no. 5, 1 May 2006 (2006-05-01), US, pages E916 - E924, XP055490979, ISSN: 0193-1849, DOI: 10.1152/ajpendo.00410.2005
- CHIANG KUN-CHUN ET AL: "The Anti-cancer Actions of Vitamin D", ANTI-CANCER AGENTS IN MEDICINAL CHEMISTRY, vol. 13, no. 1, 1 January 2013 (2013-01-01), NL, pages 126 - 139, XP055776080, ISSN: 1871-5206, DOI: 10.2174/187152013804487443
- SLOMINSKI ANDRZEJ T. ET AL: "Products of Vitamin D3 or 7-Dehydrocholesterol Metabolism by Cytochrome P450scc Show Anti-Leukemia Effects, Having Low or Absent Calcemic Activity", PLOS ONE - ARTICLE E9907, vol. 5, no. 3, 26 March 2010 (2010-03-26), pages 1 - 13, XP055776095, DOI: 10.1371/journal.pone.0009907
- WANG, X. X. ET AL.: "Vitamin D receptor agonist doxercalciferol modulates dietary fat-induced renal disease and renal lipid metabolism", AMERICAN JOURNAL OF PHYSIOLOGY RENAL PHYSIOLOGY, vol. 300, no. 3, 2011, pages F801 - F810, XP055457502
- MARCZAK, S. ET AL.: "Synthesis and biological activity of the la,25-dihydroxyvitamin D3 diastereomer with unnatural configuration at the rings C/D side-chain moiety", BIOORGANIC & MEDICINAL CHEMISTRY LETTERS, vol. 11, no. 1, 2001, pages 63 - 66, XP004225323, doi:10.1016/S0960-894X(00)00598-9
- MICHALAK, K. ET AL.: "Total Synthesis of a CD -Ring: Side-Chain Building Block for Preparing 17-epi-Calcitriol Derivatives from the Hajos-Parrish Dione", THE JOURNAL OF ORGANIC CHEMISTRY, vol. 76, no. 16, 2011, pages 6906 - 6911, XP055457510
- FUJISHIMA, T. ET AL.: "Efficient synthesis and biological evaluation of all A-ring diastereomers of la,25-dihydroxyvitamin D3 and its 20-epimer", BIOORGANIC & MEDICINAL CHEMISTRY, vol. 8, no. 1, 2000, pages 123 - 134, XP055457507
- OVES, D. ET AL.: "Versatile synthesis and biological evaluation of 1,3-diamino- substituted 1alpha,25-dihydroxyvitamin D3 analogues", BIOORGANIC & MEDICINAL CHEMISTRY, vol. 14, no. 4, 2006, pages 928 - 937, XP005237546, doi:10.1016/j.bmc.2005.09.009
- SHIMAZAKI, M. ET AL.: "Analogs of la,25-dihydroxyvitamin D3 with high potency in induction of osteoclastogenesis and prevention of dendritic cell differentiation: Synthesis and biological evaluation of 2-substituted 19-norvitamin D analogs", BIOORGANIC & MEDICINAL CHEMISTRY, vol. 14, no. 13, 2006, pages 4645 - 4656, XP005445103, doi:10.1016/j.bmc.2006.02.015
- RYODEN, Y. ET AL.: "Endogenous compounds which inhibit SREBP activation", PROCEEDINGS OF THE 133TH ANNUAL MEETING OF THE PHARMACEUTICAL SOCIETY OF JAPAN , 133TH, THE PHARMACEUTICAL SOCIETY OF JAPAN , 2013, 29K-PM09S, ALL, 2013, XP009503989, Retrieved from the Internet <URL:http://nenkai.pharm.or.jp/133/pc/ipdfview.asp?i=2288>

## Description

### [Technical Field]

The present invention is directed to novel vitamin D₃ derivatives and these compounds for the pharmaceutical or medical use thereof for treating a disease selected from metabolic disease, liver disease, diabetes, cancer, obesity or cardiovascular disease in a subject in need thereof.

### [Background Art]

Sterol regulatory element-binding proteins (SREBPs) are one of families of transcription factors involved in lipid homeostasis. SREBPs control lipid metabolism in all tissues, by regulating expression of the genes related to biosynthesis and uptake of fatty acids, triglycerides, cholesterol, and phospholipids. Because of their central roles in lipid metabolism, SREBPs are strongly linked to metabolic syndromes. For example, high insulin levels, induced by high calorie diets or obesity, hyper-activate SREBPs, causing triglyceride accumulation and inducing fatty liver diseases. Hyper-activation of SREBPs also increases cholesterol levels and suppresses insulin receptor substrate-2, leading to hyperlipidemia, arteriosclerosis, and insulin resistance. Furthermore, activation of SREBPs is often correlated with the growth of cancers and the ability of hepatitis virus to cause fatty liver diseases (Non Patent Literature 1). The involvement of SREBP activation in multiple diseases has made these transcription factors attractive pharmaceutical targets. To date, the only known "endogenous" molecules that directly inhibit the SREBP activation pathway are sterols. Further, reference is made in the literature to 1,25-dihydroxyvitamin D₃'s preventing effect against high fat diet-induced hepatic steatosis in rats as being related to the inhibition of lipogenesis and the promotion of fatty acid oxidation in rat liver (Non Patent Literature 2), vitamin D deficiency as promoting nonalcoholic steatohepatitis through impaired enterohepatic circulation in an animal model (Non Patent Literature 3), and the molecular mechanism of 1,25-dihydroxyvitamin D3 inhibition of adipogenesis in 3T3-L1 cells (Non Patent Literature 4).

### [Citation List]

### [Non Patent Literature]

[NPL 1] J. A. Menendez and R. Lupu, Nat. Rev. Cancer, 2007, 7, 763-777; A. J. Brown, Biochem. J., 2008, 416, e15-e17
[NPL 2] Eur. J. Clin. Invest. Vol 42(11) pp 1189-1196 (2012))
[NPL 3] Am. J. Physiol. Gastrointest. Liver Physiol. Vol 307(9) pp G883-G893 (11-2014)
[NPL 4] Am. J. Physiol. Endocrinol. Metab. Vol 290(5) pp E916-E924 (2006)

### [Summary of Invention]

### [Technical Problem]

It is an object of the present invention to provide compounds useful as a SREBP inhibitor and useful for treating a disease such as metabolic disease including non-alcoholic steatohepatitis (NASH); liver disease including fatty liver; diabetes; cancer; obesity; cardiovascular disease; and the like.

### [Solution to Problem]

The inventors have found novel vitamin D₃ derivatives as a SREBP inhibitor. The vitamin D₃ derivatives in the present invention are useful for treating a disease such as metabolic disease including non-alcoholic steatohepatitis (NASH); liver disease including fatty liver; diabetes; cancer; obesity; cardiovascular disease; and the like.

In one aspect, the present invention is directed to a compound of the following general formula (I): 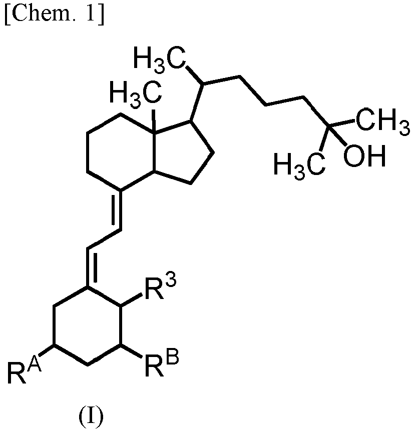 wherein one of R^{A} and R^{B} is hydroxyl and the other is NR¹R²;
R¹ and R² are each independently selected from hydrogen; C₁₋₄ alkyl; C₁₋₄ alkylcarbonyl optionally substituted with at least one halogen which are the same or different; C₁₋₄ alkylsulfonyl; benzyloxycarbonyl; 3 to 6-membered cycloalkyl-C₁₋₄ alkyl; C₆₋₁₀ arylcarbonyl optionally substituted with at least one group independently selected from the group consisting of halogen, halo-C₁₋₄ alkyl, -S-halo-C₁₋₄ alkyl, C₁₋₄ alkoxy, halo-C₁₋₄ alkoxy, nitro, cyano, C₁₋₄ alkoxycarbonyl and C₆₋₁₀ aryl; C₆₋₁₀ arylsulfonyl optionally substituted with at least one group independently selected from the group consisting of C₁₋₄ alkyl, nitro, and di-(C₁₋₄ alkyl)amino; 5 to 6-membered saturated heterocyclyl-C₁₋₄ alkyl optionally substituted with at least one group independently selected from the group consisting of halogen and hydroxyl; 5 to 6-membered heteroaryl; and a group of the following formula: 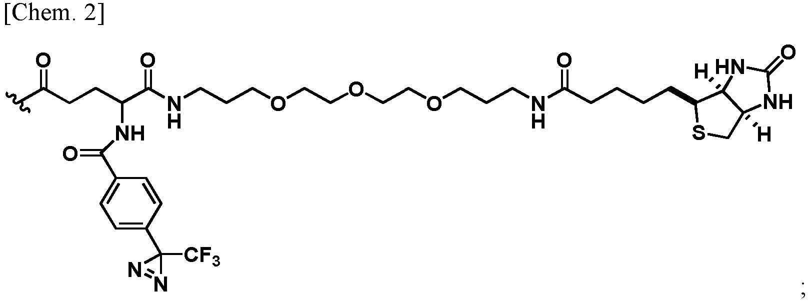 or
R¹ and R² may optionally combine together with the nitrogen atom to which they attach to form a nitrogen-containing oxo-substituted saturated 5- to 6-membered heterocyclic ring which may be optionally fused with a C₆₋₁₀ aryl ring; and
R³ is hydrogen or =CH₂; or a pharmaceutically acceptable salt thereof;
provided that R¹ and R² are not concurrently hydrogen.

In another aspect, the present invention is also directed to the compound of Formula (I) or a pharmaceutically acceptable salt thereof for use in a method for treating a disease selected from metabolic disease, liver disease, obesity, diabetes, cardiovascular disease, or cancer in a subject.

In another aspect, the present invention is also directed to the compound of Formula (I) or a pharmaceutically acceptable salt thereof for use in a method for inhibiting SREBPs in a subject.

In still another aspect, the present invention is also directed to a pharmaceutical composition, comprising as the active ingredient the compound of Formula (I) or a pharmaceutically acceptable salt thereof and a pharmaceutically acceptable carrier.

In still another aspect, the present invention is also directed to the compound of Formula (I) or a pharmaceutically acceptable salt thereof for use in the treatment of metabolic disease including non-alcoholic steatohepatitis; liver disease including fatty liver; diabetes; cancer; obesity; or cardiovascular disease.

In still another aspect, the present invention is also directed to the compound of Formula (I) or a pharmaceutically acceptable salt thereof for use in the treatment of obesity, non-alcoholic steatohepatitis (NASH); fatty liver; or cancer.

### [Advantageous Effects of Invention]

The compound of Formula (I) or a pharmaceutically acceptable salt thereof has SREBP inhibitory activity and may be useful for treating a disease such as metabolic disease including non-alcoholic steatohepatitis (NASH), liver disease including fatty liver; diabetes; cancer; obesity; cardiovascular disease or the like.

### [Brief Description of Drawings]

[Fig. 1]

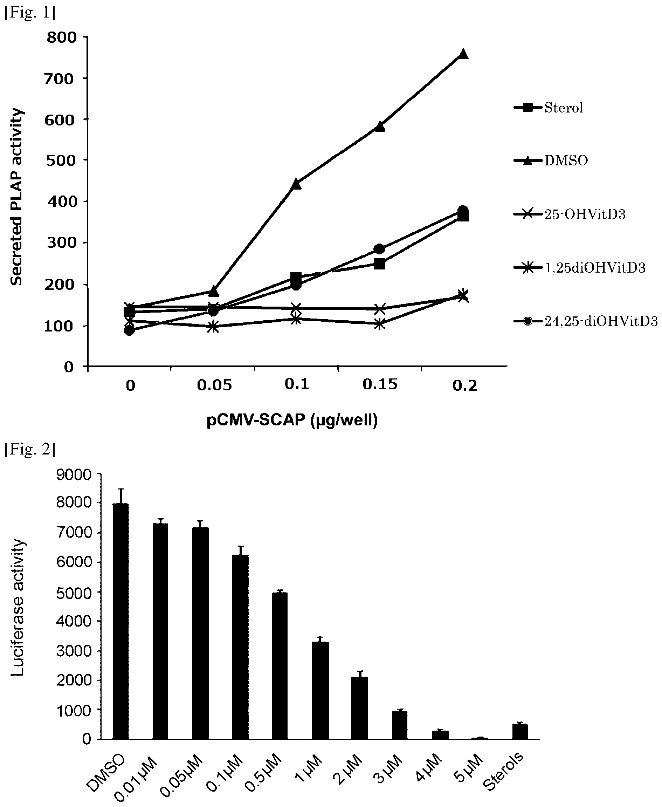

   Fig.1 shows the evaluation of vitamin D₃ (VD) derivatives by PLAP-BP assay. The concentration of VD derivatives was set at 10 µM. Sterol was a mixture of 10 µg/mL cholesterol and 1.0 µg/mL 25-hydroxycholesterol (25-HC). When cells were treated with DMSO, the secretion of PLAP increased in proportion to the amount of transfected SREBP cleavage-activating protein (SCAP). Addition of sterol, a known SREBP inhibitor, suppressed the increase in secretion of PLAP. 25(OH)D and 1,25(OH)₂D inhibited the secretion of PLAP to a greater extent than sterol. Each value represents the average of two independent experiments. 25-OHVitD3 means 25-hydroxyvitamin D₃ [25(OH)D]; 1,25diOHVitD3 means 1α,25-dihydroxyvitamin D₃ [1,25(OH)₂D]; 24,25-diOHVitD3 means 24,25-dihydroxyvitamin D₃.
[Fig. 2]
   Fig. 2 shows effects of 25(OH)D on the activation of SREBP in the reporter assay. The transfected cells were treated with DMSO alone, sterols (10 µg/mL cholesterol and 1.0 µg/mL 25-HC), or varied concentrations of 25(OH)D in a medium containing lipid-free serum. Luciferase activity was measured after 20 h incubation, and the data were normalized to β-galactosidase activity. Values are the mean ± SD of three independent experiments.
[Fig. 3]

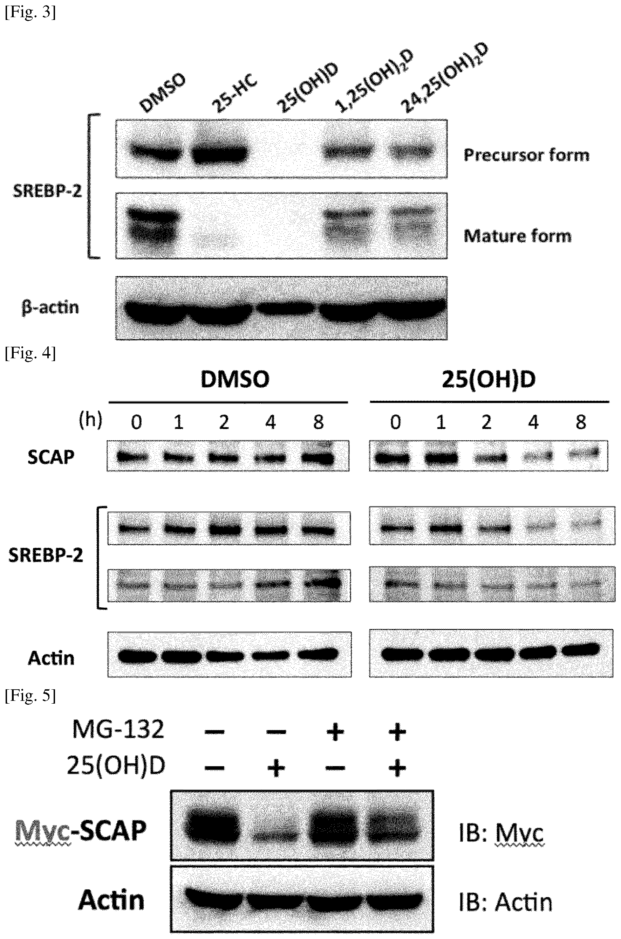

   Fig. 3 shows the effects of VD derivatives on SREBP-2. CHO-K1 cells were treated with DMSO alone or each molecule (5 µM) for 18 h. The levels of precursor and mature forms of SREBP-2 were analyzed by Western-blot. Western-blots of actin are shown in lower panel as a loading control.
[Fig. 4]
   Fig. 4 shows the effects of 25(OH)D on SCAP and SREBP-2 at different times after addition of 25(OH)D. CHO-K1 cells were treated with DMSO alone or 25(OH)D (5 µM). After incubation for the indicated time, the levels of SCAP and precursor and mature forms of SREBP-2 were analyzed by Western-blot. Western-blots of actin are shown in lower panel as a loading control.
[Fig. 5]
   Fig. 5 shows the effects of 25(OH)D in the presence or absence of MG-132 on Myc-SCAP. CHO-K1 cells were treated with DMSO alone or 25(OH)D (5 µM) in the presence or absence of MG-132 (10 µM) for 5 h. The levels of Myc-SCAP were analyzed by Western-blot. Western-blots of actin are shown in lower panel as a loading control.
[Fig. 6]

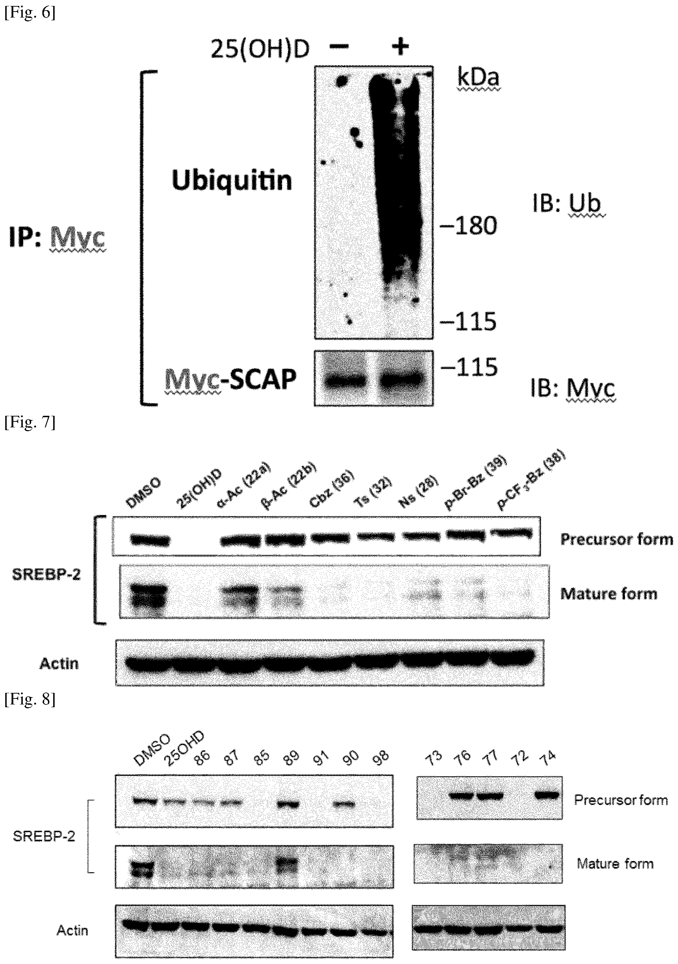

   Fig. 6 shows the effects of 25(OH)D on ubiquitination of SCAP. CHO-K1 cells were treated with DMSO alone (-) or 25(OH)D (5 µM, +). After 2 h incubation, each dish received MG-132 (10 µM). Following incubation for 2 h, cells were harvested and subjected to immunoprecipitation with monoclonal anti-*c*-Myc-conjugated agarose beads. The ubiquitination of SCAP was analyzed by Western-blot.
[Fig. 7]
   Fig. 7 shows the effects of Compounds **22a, 22b, 28, 32, 36, 38,** and **39** on SREBP-2. CHO-K1 cells were treated with DMSO alone or each molecule (5 µM) for 16 h. The levels of precursor and mature forms of SREBP-2 were analyzed by Western-blot. Western-blots of actin are shown in lower panel as a loading control. When treating the cells with the Example compounds, the levels of the mature form of SREBP were lower than that of DMSO.
[Fig. 8]
   Fig. 8 shows the effects of Compounds **72-74, 76, 77, 85-87, 89-91,** and **98** on SREBP-2. CHO-K1 cells were treated with DMSO alone or each molecule (5 µM) for 16 h. The levels of precursor and mature forms of SREBP-2 were analyzed by Western-blot. Western-blots of actin are shown in lower panel as a loading control. When treating the cells with the Example compounds, the levels of the mature form of SREBP were lower than that of DMSO.

### [Description of Embodiments]

The term "alkyl" used herein refers to a straight- or branched-chain hydrocarbon group preferably having 1 to 4 carbon atoms, and includes, for example, methyl, ethyl, normal-propyl, isopropyl, normal-butyl, isobutyl, *tert*-butyl, etc.

The term "alkoxy" used herein refers to a monovalent group wherein the above mentioned alkyl group attaches to oxygen atom, and may be a straight- or branched-chain group preferably having 1 to 4 carbon atoms. The alkoxy group includes, for example, methoxy, ethoxy, normal-propoxy, isopropoxy, normal-butoxy, isobutoxy, *tert*-butoxy, etc.

The term "alkylcarbonyl" used herein refers to a group wherein the above mentioned alkyl group attaches to carbonyl group, and is preferably C₁₋₄ alkylcarbonyl. The alkylcarbonyl group includes, for example, acetyl, ethylcarbonyl, normal-propylcarbonyl, isopropylcarbonyl, normal-butylcarbonyl, isobutylcarbonyl, *tert*-butylcarbonyl, etc.

The term "alkylsulfonyl" used herein refers to a group wherein the above mentioned alkyl group attaches to sulfonyl group, and is preferably C₁₋₄ alkylsulfonyl. The alkylsulfonyl group includes, for example, methylsulfonyl, ethylsulfonyl, normal-propylsulfonyl, isopropylsulfonyl, normal-butylsulfonyl, isobutylsulfonyl, *tert*-butylsulfonyl, etc.

The term "alkoxycarbonyl" used herein refers to a group wherein the above mentioned alkoxy group attaches to carbonyl group, and is preferably C₁₋₄ alkoxycarbonyl. The alkoxycarbonyl group includes, for example, methoxycarbonyl, ethoxycarbonyl, normal-propoxycarbonyl, isopropoxycarbonyl, normal-butoxycarbonyl, isobutoxycarbonyl, *tert-*butoxycarbonyl, etc.

The term "cycloalkyl" used herein refers to a saturated aliphatic monocyclic hydrocarbon ring preferably having 3 to 6 carbon atoms. The cycloalkyl group includes, for example, cyclopropyl, cyclobutyl, cyclopentyl, cyclohexyl, etc. A preferable cycloalkyl group is 3 to 6-membered cycloalkyl, and more preferable one is cyclopropyl.

The term "aryl" used herein refers to a monovalent group of monocyclic aromatic hydrocarbon ring or polycyclic aromatic hydrocarbon ring preferably having 6 to 10 carbon atoms. The aryl group includes, for example, phenyl, naphthyl, etc. A preferable aryl is C₆₋₁₀ aryl, and more preferable one is phenyl or naphthyl.

The term "arylcarbonyl" used herein refers to a group wherein the above mentioned aryl group attaches to carbonyl group, and is preferably C₆₋₁₀ arylcarbonyl. The arylcarbonyl group includes, for example, benzoyl, naphthylcarbonyl, etc. A preferable arylcarbonyl includes benzoyl, etc.

The term "arylsulfonyl" used herein refers to a group wherein the above mentioned aryl group attaches to sulfonyl group, and is preferably C₆₋₁₀ arylsulfonyl. The arylsulfonyl group includes, for example, phenylsulfonyl, naphthylsulfonyl, etc. A preferable arylsulfonyl includes phenylsulfonyl, etc.

The term "heterocyclyl" or "heterocyclic" used herein refers to a monovalent group of saturated or partially unsaturated 5 to 6-membered monocyclic group having at least one heteroatom, preferably one or two heteroatom(s), independently selected from nitrogen, oxygen or sulfur and carbon atoms. The heterocyclyl group includes, for example, pyrrolidinyl, oxazolinyl, pyrazolidinyl, piperidyl, piperazinyl, morpholinyl, etc. A preferable heterocyclyl group includes pyrrolidinyl, piperidyl, morpholinyl, etc.

The term "heteroaryl" used herein refers to a monovalent group of aromatic cyclic group having at least one heteroatom independently selected from nitrogen, oxygen or sulfur and carbon atoms, and is preferably 5 to 6-membered heteroaryl group. The heteroaryl group includes, for example, pyrrolyl, isoxazolyl, isothiazolyl, pyrazolyl, oxazolyl, thiazolyl, imidazolyl, pyridyl, pyrimidinyl, etc. A preferable heteroaryl group includes thiazolyl, pyridyl, etc.

The term "nitrogen-containing oxo-substituted saturated 5 to 6-membered heterocyclic ring which may be optionally fused with a C₆₋₁₀ aryl ring" used herein refers to the above mentioned heterocyclyl ring containing at least one nitrogen atom in the ring which is substituted with at least one oxo group, and includes, for example, γ-lactam, δ-lactam, phthalimidyl, etc.

The term "halogen" or "halo" used herein refers to fluorine atom, chlorine atom, bromine atom, iodine atom, etc.

The optionally substituted alkylcarbonyl refers to the above mentioned alkylcarbonyl which may be optionally substituted with the same or different at least one halogen. The substituent in the optionally substituted alkylcarbonyl includes the same or different 1 to 9, preferably 1 to 3, halogen atom(s) and in particular three fluorine atoms.

The optionally substituted arylcarbonyl refers to the above mentioned arylcarbonyl which may be optionally substituted with the same or different at least one group selected from the group consisting of halogen, halo-C₁₋₄ alkyl, -S-halo-C₁₋₄ alkyl, C₁₋₄ alkoxy, halo-C₁₋₄ alkoxy, nitro, cyano, C₁₋₄ alkoxycarbonyl, and C₆₋₁₀ aryl. Preferable substituents in the optionally substituted arylcarbonyl include the same or different 1 to 4 group(s) selected from the group consisting of chloro, fluoro, bromo, methyl, methoxy, trifluoromethyl, methoxycarbonyl, trifluoromethoxy, nitro, cyano, -S-CF₃, phenyl, etc.

The optionally substituted arylsulfonyl refers to the above mentioned arylsulfonyl which may be optionally substituted with the same or different at least one group selected from the group consisting of C₁₋₄ alkyl and nitro. A preferable substituent in the optionally substituted arylsulfonyl includes methyl, nitro, etc.

The optionally substituted 5 to 6-membered saturated heterocyclyl-alkyl refers to the above mentioned alkyl substituted with the above mentioned heterocyclyl which may be optionally substituted with the same or different at least one group selected from the group consisting of halogen and hydroxyl. A preferable substituent in the optionally substituted 5 to 6-membered saturated heterocyclyl-alkyl includes fluoro, hydroxy, etc.

In one aspect, the present invention is directed to the following Items or embodiments.

Item 1: A compound of the following general formula (I): 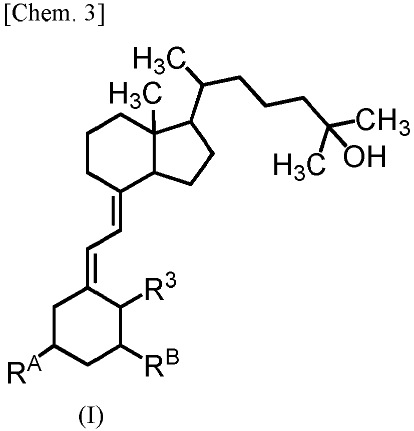 wherein one of R^{A} and R^{B} is hydroxyl and the other is NR¹R²;
   R¹ and R² are each independently selected from hydrogen; C₁₋₄ alkyl; C₁₋₄ alkylcarbonyl optionally substituted with at least one halogen which are the same or different; C₁₋₄ alkylsulfonyl; benzyloxycarbonyl; 3 to 6-membered cycloalkyl-C₁₋₄ alkyl; C₆₋₁₀ arylcarbonyl optionally substituted with at least one group independently selected from the group consisting of halogen, halo-C₁₋₄ alkyl, -S-halo-C₁₋₄ alkyl, C₁₋₄ alkoxy, halo-C₁₋₄ alkoxy, nitro, cyano, C₁₋₄ alkoxycarbonyl and C₆₋₁₀ aryl; C₆₋₁₀ arylsulfonyl optionally substituted with at least one group independently selected from the group consisting of C₁₋₄ alkyl, nitro, and di-(C₁₋₄ alkyl)amino; 5 to 6-membered saturated heterocyclyl-C₁₋₄ alkyl optionally substituted with at least one group independently selected from the group consisting of halogen and hydroxyl; 5 to 6-membered heteroaryl; and a group of the following formula: 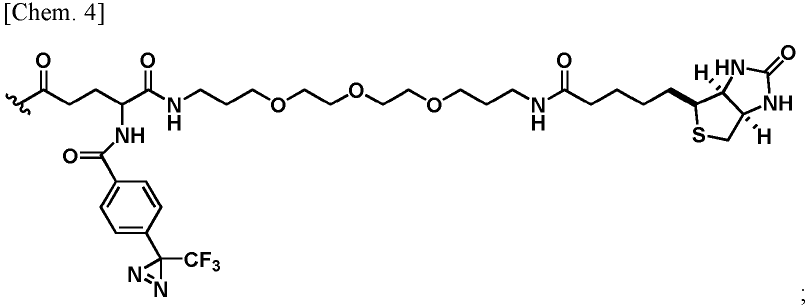 or
   R¹ and R² may optionally combine together with the nitrogen atom to which they attach to form a nitrogen-containing oxo-substituted saturated 5 to 6-membered heterocyclic ring which may be optionally fused with a C₆₋₁₀ aryl ring; and
   R³ is hydrogen or =CH₂; or a pharmaceutically acceptable salt thereof;
   provided that R¹ and R² are not concurrently hydrogen.
Item 2: The compound of Item 1, wherein R³ is =CH₂, or a pharmaceutically acceptable salt thereof.
Item 3: The compound of Item 1, wherein R³ is hydrogen, or a pharmaceutically acceptable salt thereof.
Item 4: The compound of any one of Items 1 to 3, having any one of the following formulae: 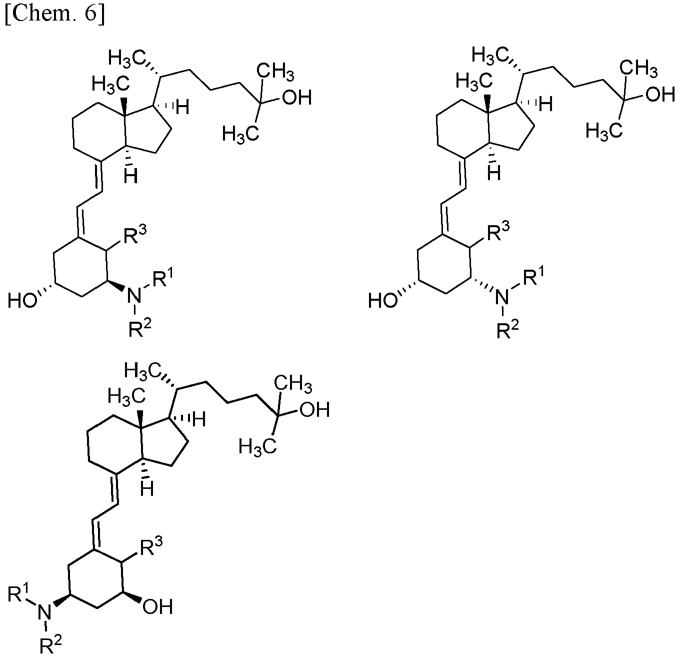 wherein R¹ and R² are as defined in Item 1, and R³ is as defined in any one of Items 1 to 3.
Item 5: The compound of any one of Items 1 to 4, wherein R² is hydrogen, or a pharmaceutically acceptable salt thereof.
Item 6: The compound of Item 1, having a structure: 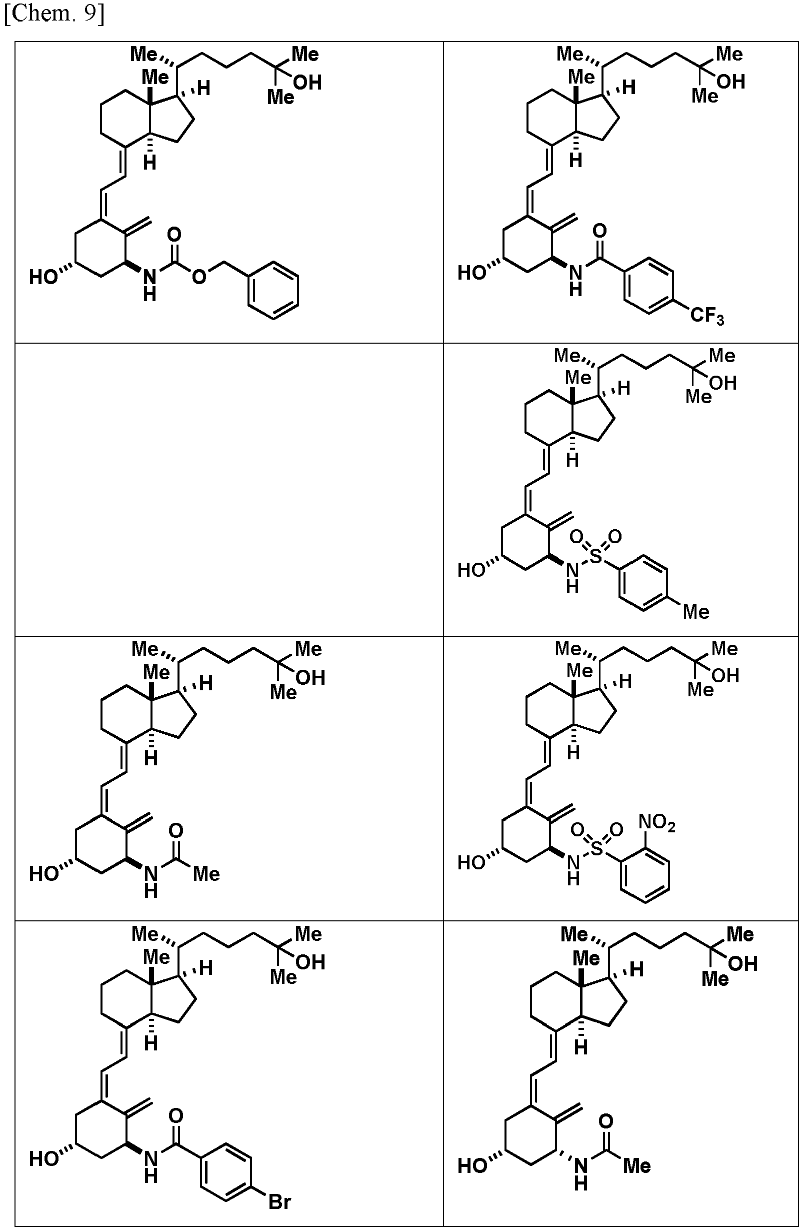 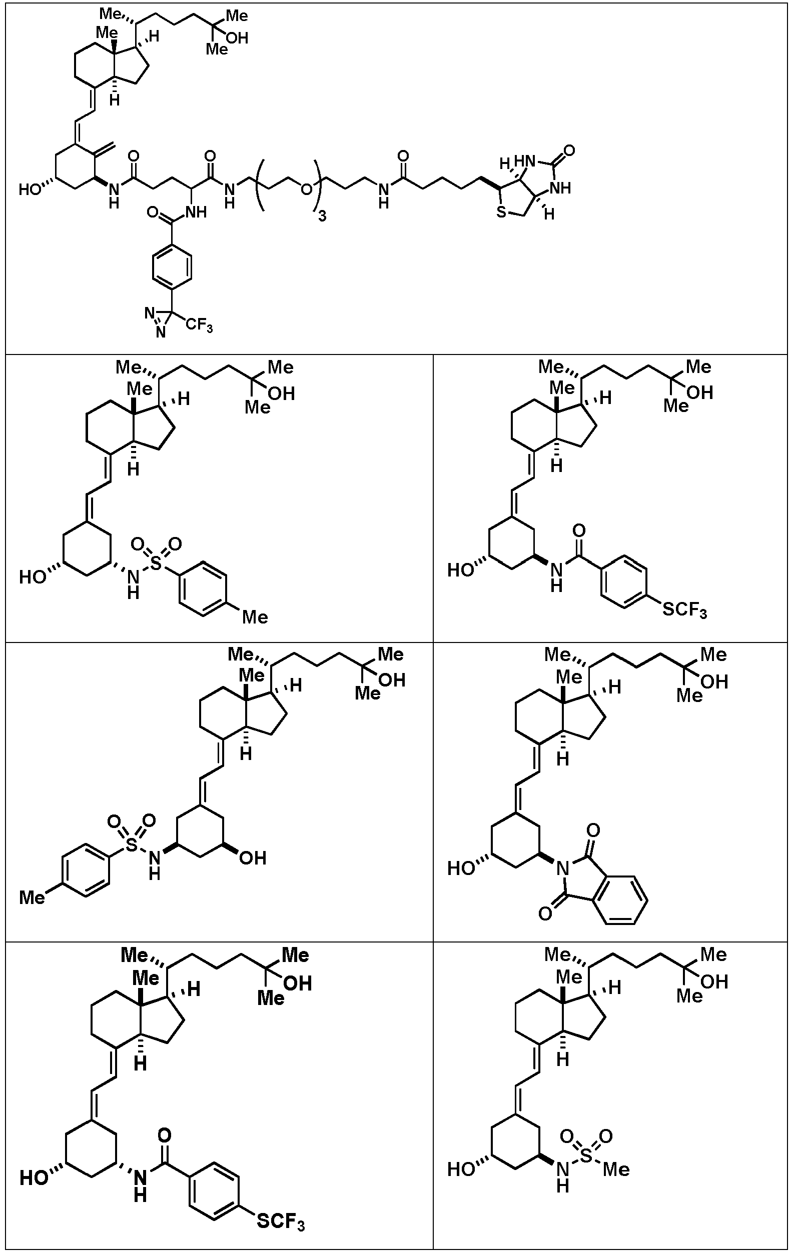 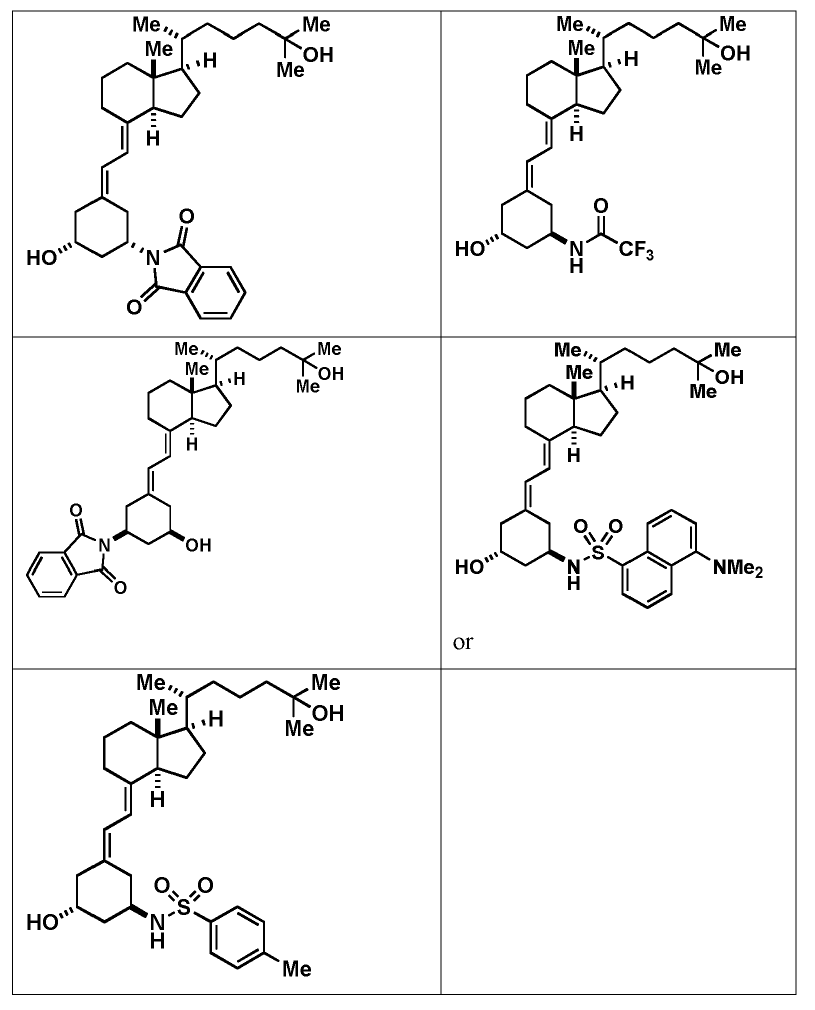
Item 7: A pharmaceutical composition, comprising as the active ingredient the compound of any one of Items 1 to 6 or a pharmaceutically acceptable salt thereof and a pharmaceutically acceptable carrier.
Item 8: A compound of any one of Items 1 to 6 or a pharmaceutically acceptable salt thereof, or a pharmaceutical composition of Item 7 for use in treating a metabolic disease, liver disease, obesity, diabetes, cardiovascular disease, or cancer in a subject.
Item 9: The compound or a pharmaceutically acceptable salt thereof, or the pharmaceutical composition for use of Item 8, wherein the disease is obesity, non-alcoholic steatohepatitis (NASH), fatty liver or cancer.
Item 10: The compound or a pharmaceutically acceptable salt thereof, or the pharmaceutical composition for use of Item 8 or 9, wherein the compound or the compound comprised in the pharmaceutical composition is selected from 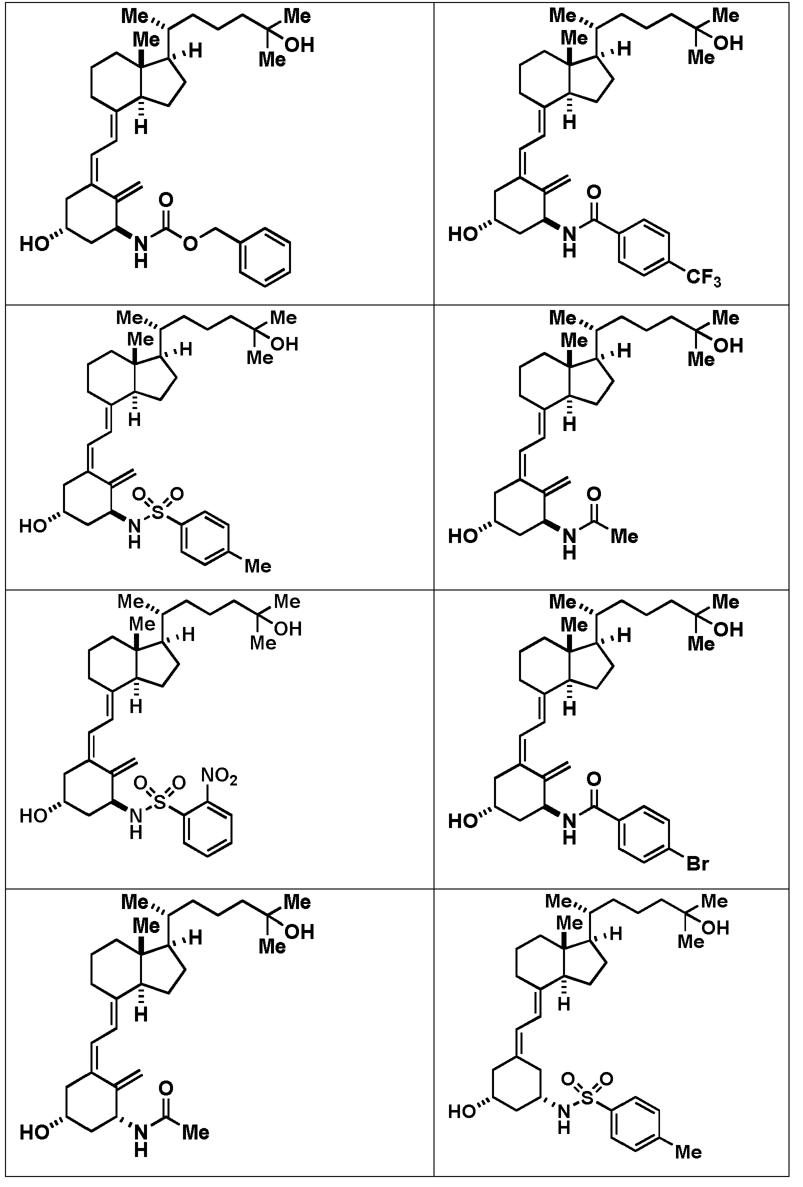 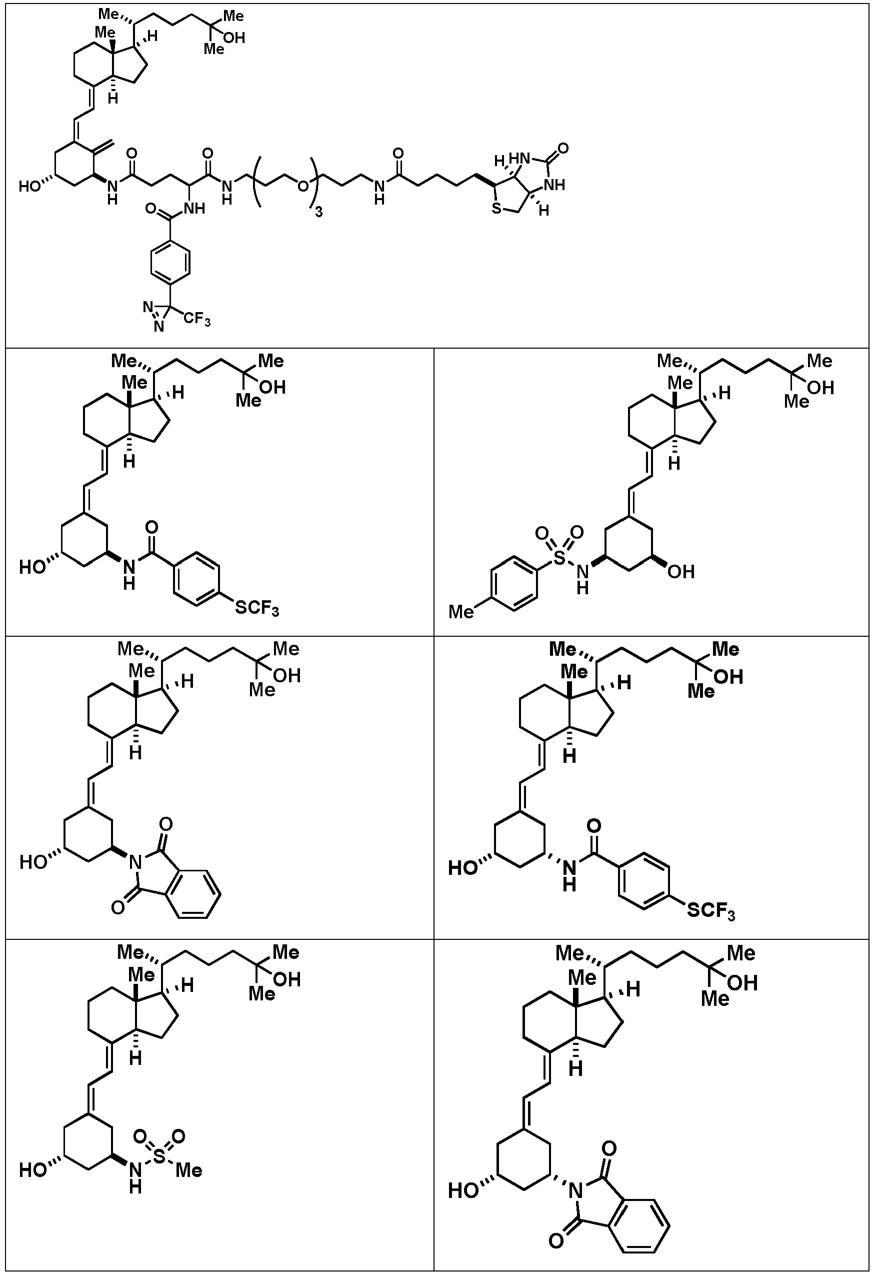 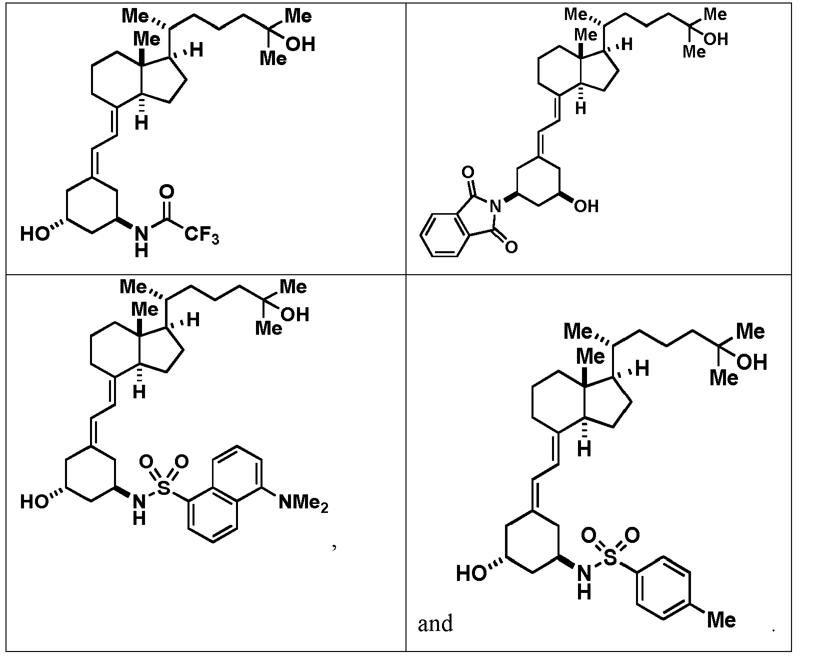 Item 11: A compound of any one of Items 1 to 6 or a pharmaceutically acceptable salt thereof for use in inhibiting an SREBP in a subject.
Item 12: The compound or a pharmaceutically acceptable salt thereof for use of Item 11, wherein the SREBP inhibition is for treating a metabolic disease, liver disease, obesity, diabetes, cardiovascular disease, or cancer in the subject.
Item 13: The compound or a pharmaceutically acceptable salt thereof for use of Item 12, wherein the SREBP inhibition is for treating obesity, non-alcoholic steatohepatitis (NASH), fatty liver, or cancer in the subject.

One of R^{A} and R^{B} is hydroxyl and the other is NR¹R².

In one embodiment, R¹ and R² each independently include hydrogen, tert-butoxycarbonyl, benzyloxycarbonyl, acetyl, p-methylphenylsulfonyl, o-nitrophenylsulfonyl, p-trifluoromethylbenzoyl, p-bromobenzoyl, ethylcarbonyl, propylcarbonyl, p-methoxybenzoyl, p-fluorobenzoyl, p-[(trifluoromethyl)thio]benzoyl, 2,3,4,5-tetrafluorobenzoyl, 2,4,5-trifluorobenzoyl, 3,4-dimethoxybenzoyl, 2,3,4-trifluorobenzoyl, 3,4-difluorobenzoyl, 2,4-difluorobenzoyl, 3-chloro-4-fluorobenzoyl, 2-chloro-4-fluorobenzoyl, p-nitrobenzoyl, 2-trifluoromethyl-4-fluorobenzoyl, 3-trifluoromethyl-4-fluorobenzoyl, p-trifluoromethoxybenzoyl, p-cyanobenzoyl, p-methoxycarbonylbenzoyl, p-phenylbenzoyl, 2-morpholinylethyl, 2-(4-fluoropiperidinyl)ethyl, 2-(4-hydroxypiperidinyl)ethyl, 2-pyridyl, 2-thiazolyl, cyclopropylmethyl, ethyl, butyl, methylsulfonyl, trifluoromethylcarbonyl, 5-dimethylamino-1-naphthylsulfonyl, and a group of the following probe structure: 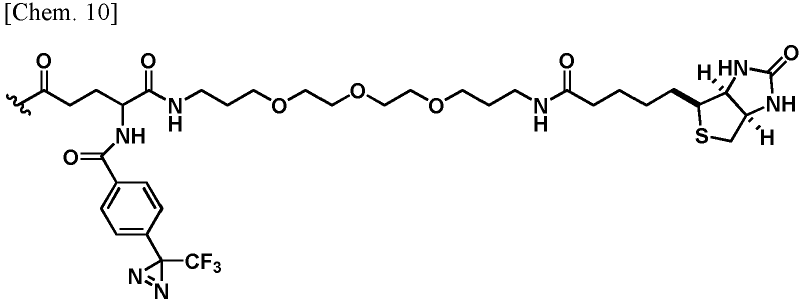 , etc.

In another embodiment, R¹ and R² may combine together with the nitrogen atom to which they attach to form for example γ-lactam, δ-lactam or phthalimidyl.

In still another embodiment, R³ is =CH₂.

In still another embodiment, R³ is hydrogen.

The pharmaceutically acceptable salt used herein refers to any salts which are known in the art and do not have excess toxicity. In particular, the pharmaceutically acceptable salt may include a salt with an inorganic acid, an organic acid, an inorganic base, or an organic base. Such an inorganic acid includes hydrofluoric acid, hydrochloric acid, hydrobromic acid, hydroiodic acid, nitric acid, sulfuric acid, and phosphoric acid. Such an organic acid includes acetic acid, trifluoroacetic acid, benzoic acid, p-toluenesulfonic acid, citric acid, oxalic acid, maleic acid, fumaric acid, lactic acid, malic acid, succinic acid, and tartaric acid. Such an inorganic base includes lithium, sodium potassium, magnesium, calcium, aluminum, and zinc. Such an organic base includes arginine and lysine. A preferable pharmaceutically acceptable salt is a salt with an inorganic acid, and in particular hydrochloride.

The pharmaceutically acceptable carrier used herein includes various conventional organic or inorganic carrier substances, for example, substances in solid preparations such as excipients, disintegrators, binders, glidants and lubricants, commonly used in the art, and substances in liquid preparations such as solvents, solubilizing agents, suspending agents, isotonizing agents, buffers and soothing agents, commonly used in the art. Additives commonly used in the art such as preservatives, antioxidants, colorants, and edulcorants may be added to a pharmaceutical composition in the present invention, if needed.

The compound of Formula (I) may be orally or parenterally administered in a therapeutically effective amount to mammals such as mice, rats, hamsters, guinea pigs, rabbits, cats, dogs, pigs, cattle, horses, sheep, monkeys, and human. While the therapeutically effective amount of the compound of Formula (I) may vary depending on subjects, diseases, dosage forms, routes of administration, and the like, the therapeutically effective amount of the compound of Formula (I) generally ranges for example from about 0.01 mg through about 0.1 mg to about 1 g through about 10 g per day, which may be administered once or several times in a divided amount.

For the avoidance of doubt, it is confirmed that in the general description above, the proposal of general preferences and options in respect of different features of the compounds, methods, use, and compositions constitutes in the usual way the proposal of general combinations of those general preferences and options for the different features, insofar as they are combinable and compatible and are put forward in the same context.

A method for preparing the compound of Formula (I) or a pharmaceutically acceptable salt thereof is illustrated as below, but is not limited thereto. For example, the schemes as below show illustrative preparation methods for exemplary compounds in the present invention. Compounds obtained in each step may be isolated or purified by known methods including distillation, recrystallization, column chromatography, etc., if needed, and may be also used in the next step without isolation or purification.

The following abbreviations may be used herein for example:
Ac: acetyl
Bz: benzoyl
Ts: toluenesulfonyl
TMS: trimethylsilyl
TES: triethylsilyl
Tf: trifluoromethylsulfonyl
Boc: tert-butoxycarbonyl
Ns: *o*-nitrobenzenesulfonyl
Cbz: benzyloxycarbonyl
TBS: *tert*-butyldimethylsilyl
HMDS: bis(trimethylsilyl)amide
PPTS: pyridinium p-toluenesulfonate
NPhTh: phthalimide
TBAF: tetrabutylammonium fluoride
McCl: chloromethylsulfonyl chloride
TPAP: tetrapropylammonium perruthenate
DIBAL: diisobutyl aluminum hydride
TFAA: trifluoroacetic anhydride
DMAP: dimethylaminopyridine
HOBt: 1-hydroxybenzotriazole
TFA: trifluoroacetic acid
NHS: *N*-hydroxysuccinimide
DCC: *N*,*N*'-dicyclohexylcarbodiimide
EDC: 1-ethyl-3-(3-dimethylaminopropyl)carbodiimide
DMT-MM: 4-(4,6-dimethoxy-1,3,5-triazin-2-yl)-4-methylmorpholinium chloride
DIPEA: *N*,*N-*diisopropylethylamine
TEA: triethylamine
THF: tetrahydrofuran
DMSO: dimethylsulfoxide
DMF: dimethylformamide
NMM: *N*-methylmorpholine
NMO: *N*-methylmorpholine *N*-oxide
DIAD: diisopropyl azodicarboxylate
DAST: *N,N*-diethylaminosulfur trifluoride

### Preparation method via coupling reaction

The compound of Formula (I) wherein R³ is =CH₂ may be prepared according to the following procedure: 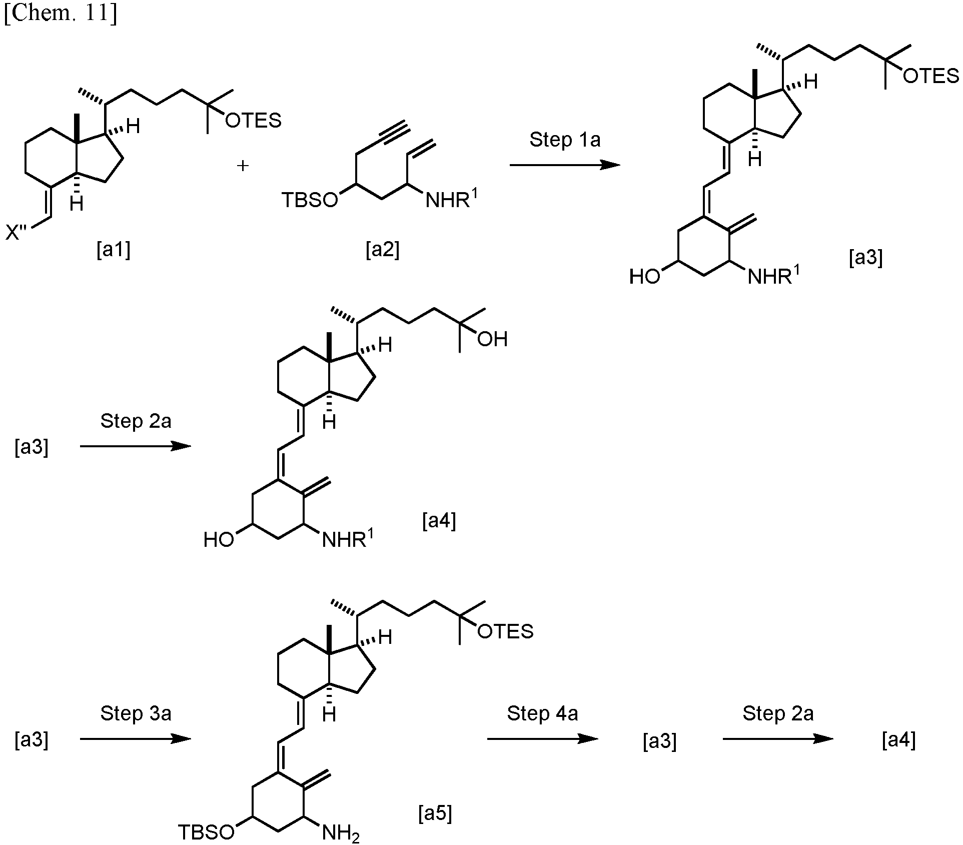 In the scheme, X" is halogen, and the other symbols have the same meanings as defined in Item 1.

### Step 1a

A compound of Formula [a1] may be coupled with a compound of Formula [a2] in the presence of a palladium catalyst such as tetrakis(triphenylphosphine)palladium (0) (*i.e.,* Pd(PPh₃)₄) and a base such as triethylamine in a solvent such as toluene to give a compound of Formula [a3]. The reaction temperature may range from room temperature to about 100 °C, preferably about 90 °C.

Compound [a1] and Compound [a2] may be prepared according to any one of the methods of preparing intermediate compounds below.

### Step 2a

A protecting group such as *tert*-butyldimethylsilyl and triethylsilyl groups in a compound of Formula [a3] may be deprotected by treatment with hydrogen fluoride with a base such as 3HF • Et₃N and HF • pyridine in a solvent such as tetrahydrofuran to give a compound of Formula [a4]. The reaction temperature may be any temperature that the reaction can proceed, preferably room temperature.

### Step 3a

A compound of Formula [a3] wherein R¹ and R² are not concurrently hydrogen (*e.g*. R¹ is arylsulfonyl and R² is hydrogen) may be optionally subjected to Mitsunobu reaction using an organic phosphine compound such as triphenylphosphine and an azocarboxylic acid ester such as diisopropyl azodicarboxylate in a solvent such as tetrahydrofuran. The resulting compound may be sequentially treated or a compound of Formula [a3] may be treated with a thiol such as 1-dodecanethiol in the presence of a base such as sodium hydride in a solvent such as ether including diethylether to give a compound of Formula [a5]. The reaction temperature may range from 0 °C to room temperature, preferably room temperature or a gradually changed temperature starting from 0 °C and raising to room temperature.

### Step 4a

A compound of Formula [a5] may be treated with R¹X' wherein X' is halogen or hydroxyl in the presence of a base such as triethylamine in a solvent such as dichloromethane to give a compound of Formula [a3]. The reaction temperature may be any temperature that the reaction can proceed, preferably 0°C. The resulting Compound [a3] may be then subjected to the deprotection according to Step 2a to give a compound of Formula [a4].

### Preparation method via Julia coupling reaction

The compound of Formula (I) wherein R³ is hydrogen or the comparative compound of formula [b6] may be prepared according to the following procedure: 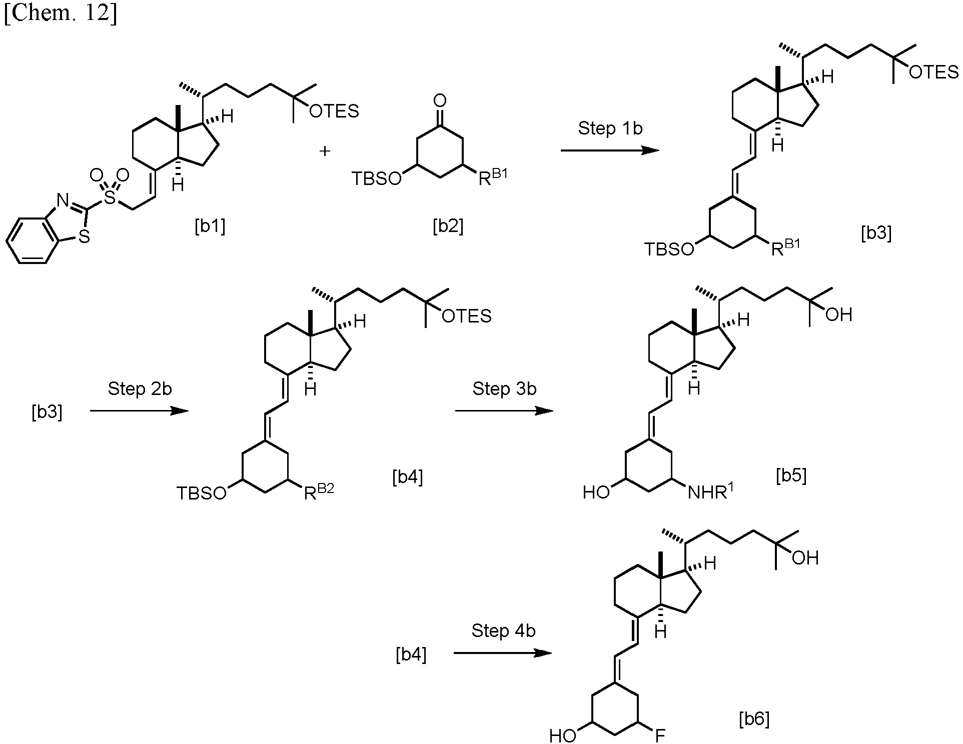 In the scheme, R^{B1} is phthalimidyl or benzyloxy, R^{B2} is amino or hydroxyl, and the other symbols have the same meanings as defined in Item 1.

### Step 1b

A compound of Formula [b1] may be treated with a compound of Formula [b2] in the presence of a base such as lithium bis(trimethylsilyl)amide (*i*.*e*., LiHMDS) in a solvent such as tetrahydrofuran to give a compound of Formula [b3]. The reaction temperature may range from -78 °C to room temperature, preferably a gradually changed temperature starting from -78 °C and warming to room temperature.

Compound [b1] and Compound [b2] may be prepared according to any one of the methods of preparing intermediate compounds below.

### Step 2b

A compound of Formula [b3] may be treated with a base such as hydrazine hydrate and potassium carbonate in a solvent such as methanol and ethanol to give a compound of Formula [b4]. The reaction temperature may range from room temperature to about 60 °C.

### Step 3b

A compound of Formula [b4] may be treated with R¹X' wherein X' is halogen or hydroxyl in the presence of a base such as triethylamine in a solvent such as dichloromethane, followed by the deprotection according to Step 2a to give a compound of Formula [b5]. The temperature of the reaction with R¹X' may be any temperature that the reaction can proceed, preferably 0 °C.

### Step 4b

A compound of Formula [b4] may be alternatively treated with a fluorination agent such as N,N-diethylaminosulfur trifluoride (*i.e*., DAST) in a solvent such as dichloromethane, followed by the deprotection according to Step 2a to give a compound of Formula [b6]. The temperature of the fluorination reaction may be any temperature that the reaction can proceed, preferably -78 °C.

Preparation methods for intermediate Compounds 14a and 14b 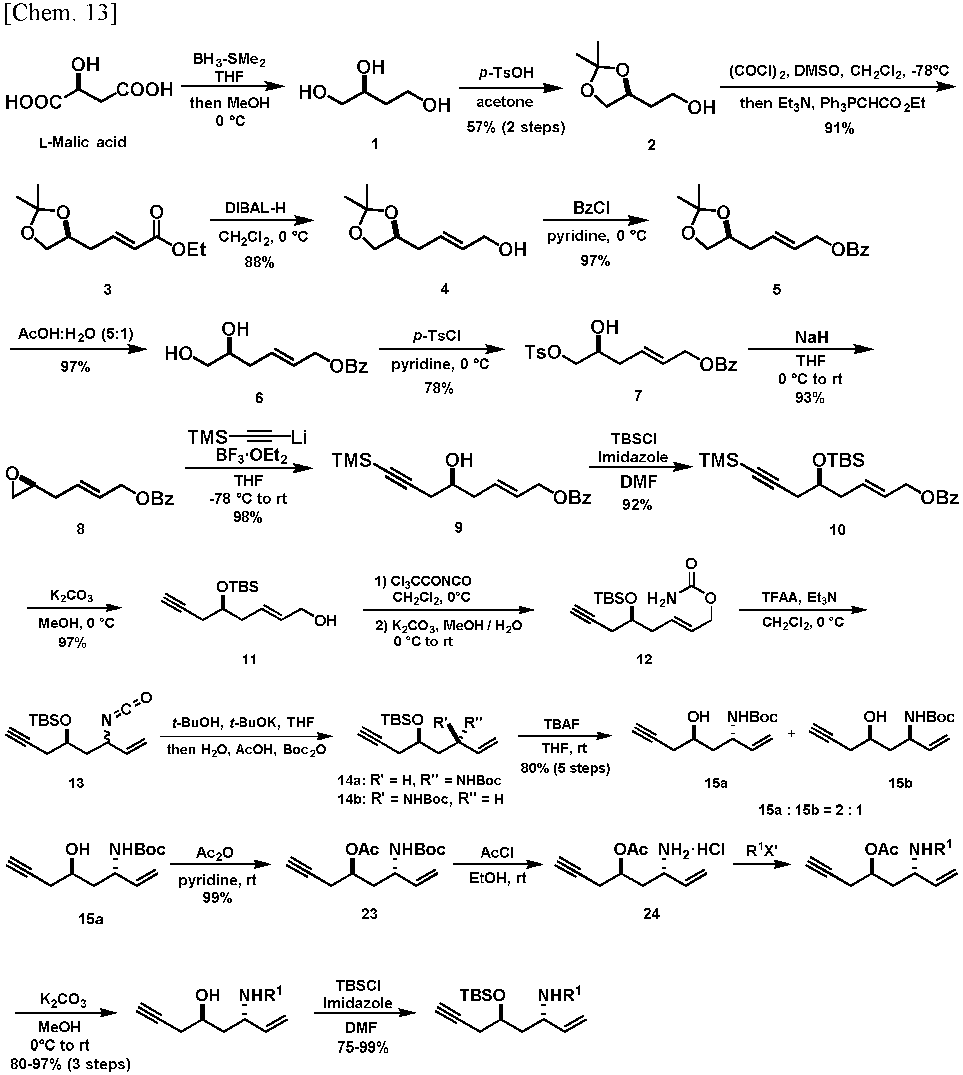 In the scheme, R¹ has the same meaning as defined in Item 1 and X' is halogen or hydroxyl.

Preparation methods for intermediate Compound 16 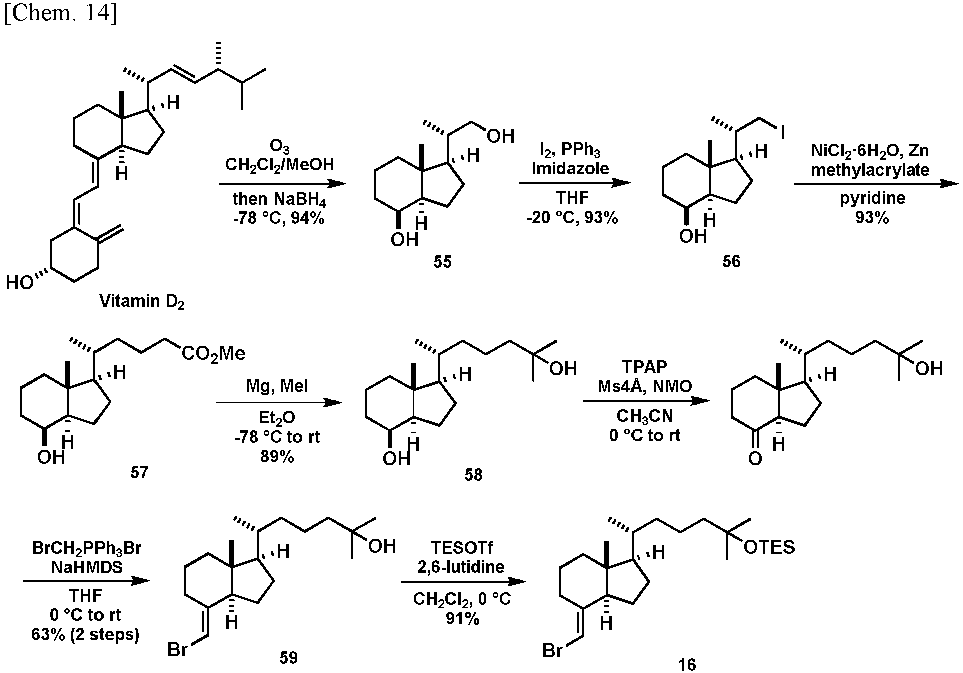 In the scheme, Compound 57 may be prepared from Compound 56 according to a common procedure in the art such as the method described in Antonio Mourino et al. Chem. Eur. J. 2010, 16, 1432-1435.

In particular, methods of preparing the compounds of Formula (I) wherein R³ is =CH₂ in the present invention are illustrated in the following schemes using the above prepared intermediate compounds or derivatives thereof which may be prepared in a similar way to the above schemes.

Preparation methods for Comparative Compounds 18a, 18b, 19a and 19b

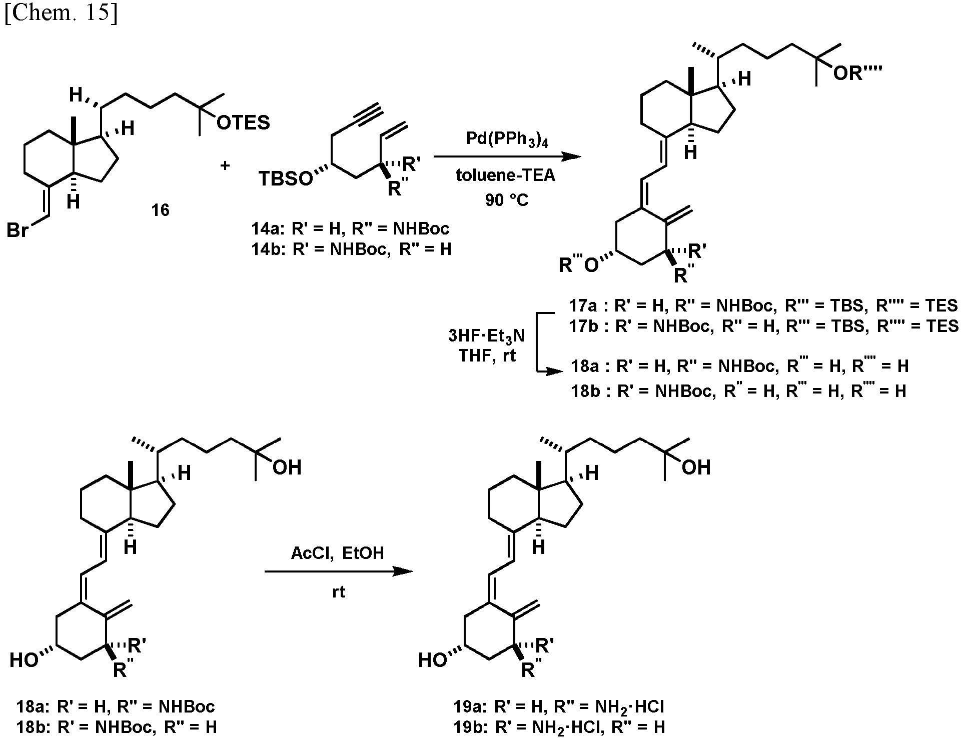

Derivatives of Comparative Compounds 18a and 18b may be also prepared in a similar procedure to the above. 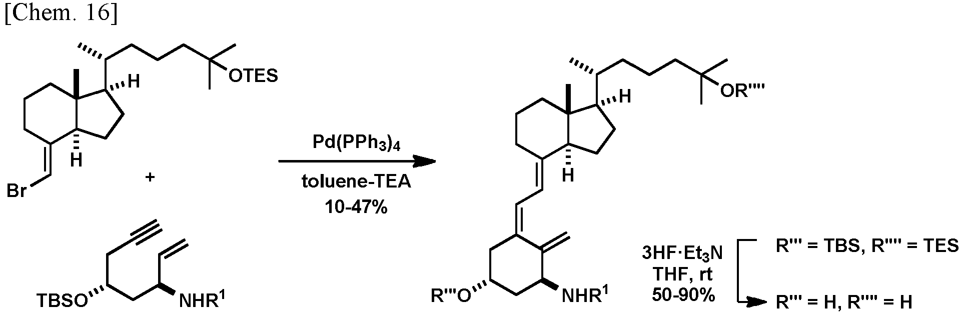 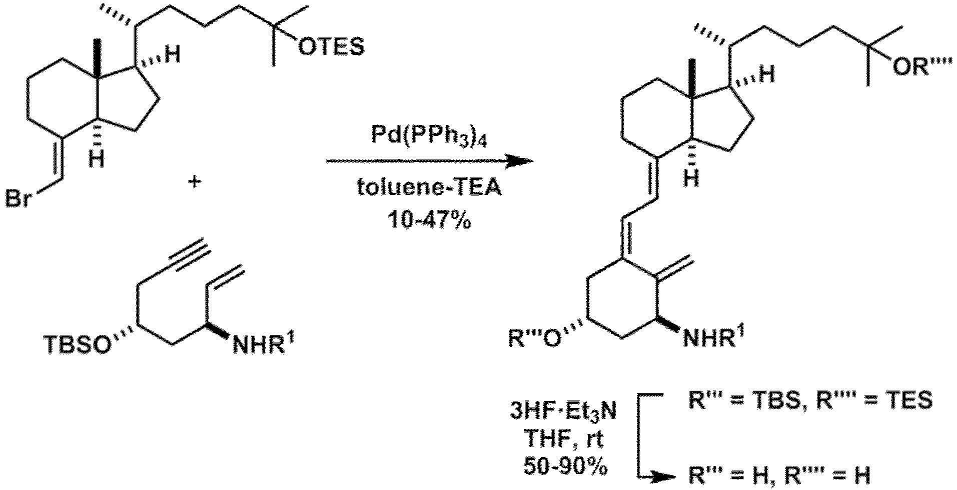 In the scheme, R¹ has the same meaning as defined in Item 1.

Preparation methods for Compounds 28, 32, 36, and 39 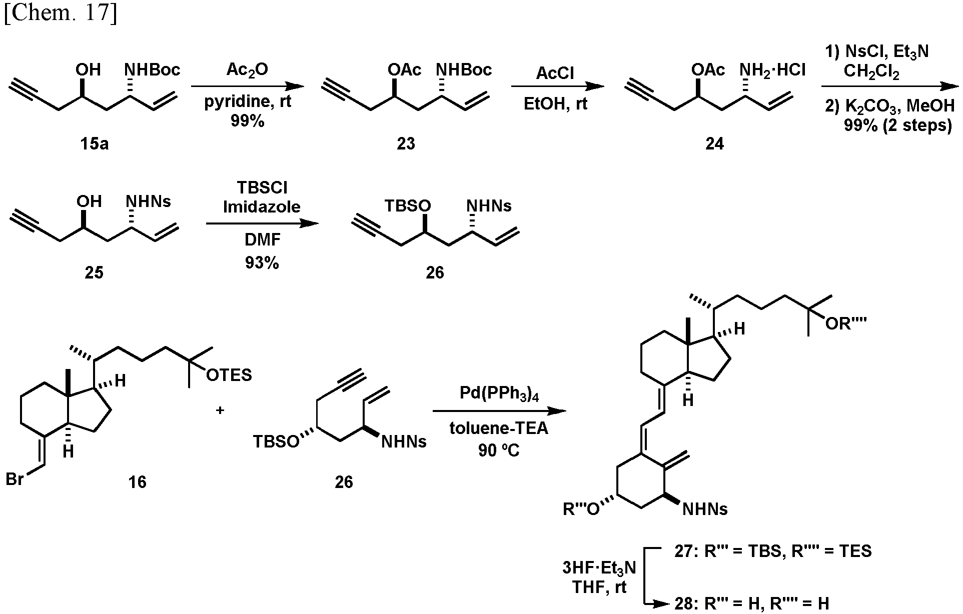 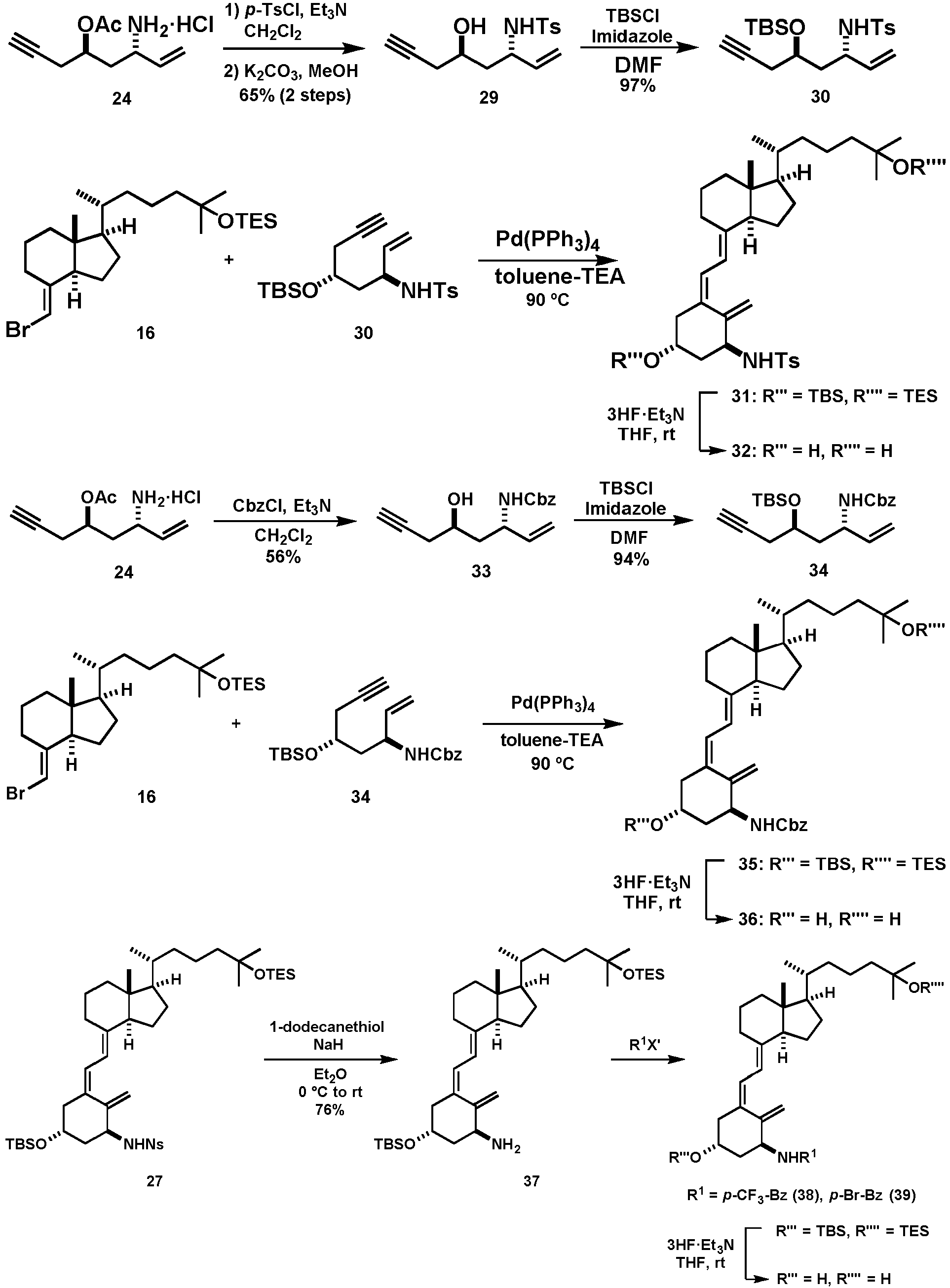 In the scheme, R¹ has the same meaning as defined in Item 1 and X' is halogen or hydroxyl.

Alternatively, the compound of Formula (I) wherein R¹ is a group of the following formula: 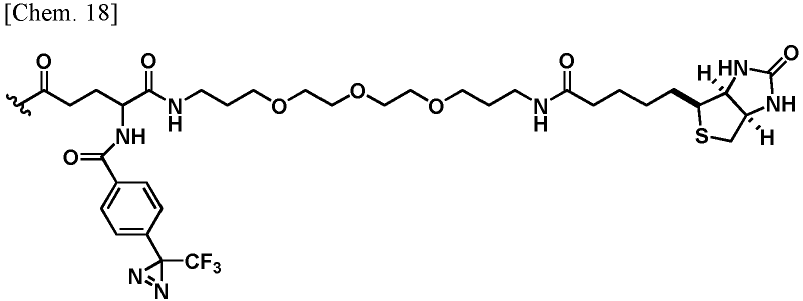 in the present invention may be prepared according to the following scheme.

Preparation methods for Compound 106

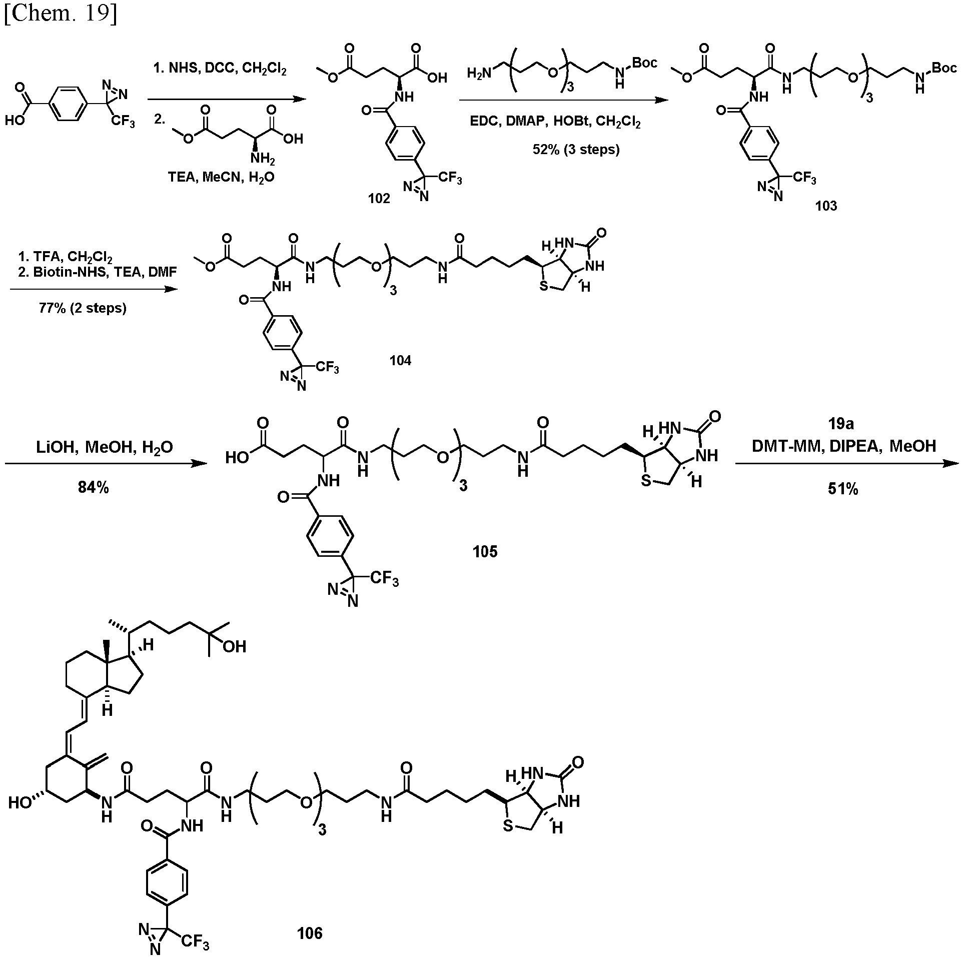

In addition, methods of preparing the compounds of Formula (I) wherein R³ is hydrogen in the present invention are illustrated in the following schemes.

Preparation methods for intermediate Compound 66 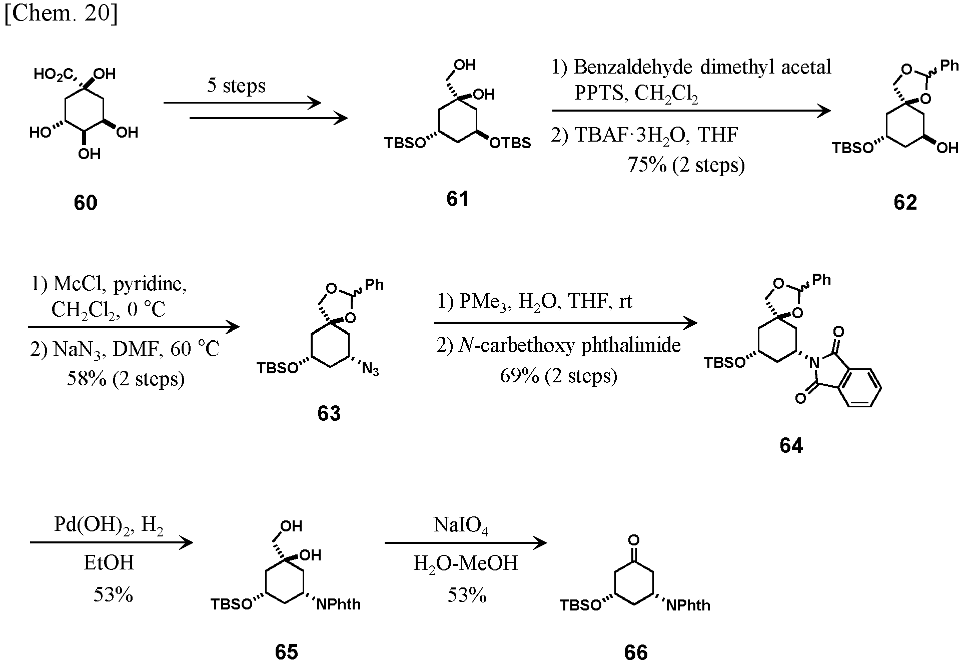 In the scheme, Compound 61 may be prepared from Compound 60 according to a common procedure in the art such as the method described in John H. White et al. Proc. Natl. Acad. Soc. 2008, 105, 8250-8255.

Preparation methods for intermediate Compound 67

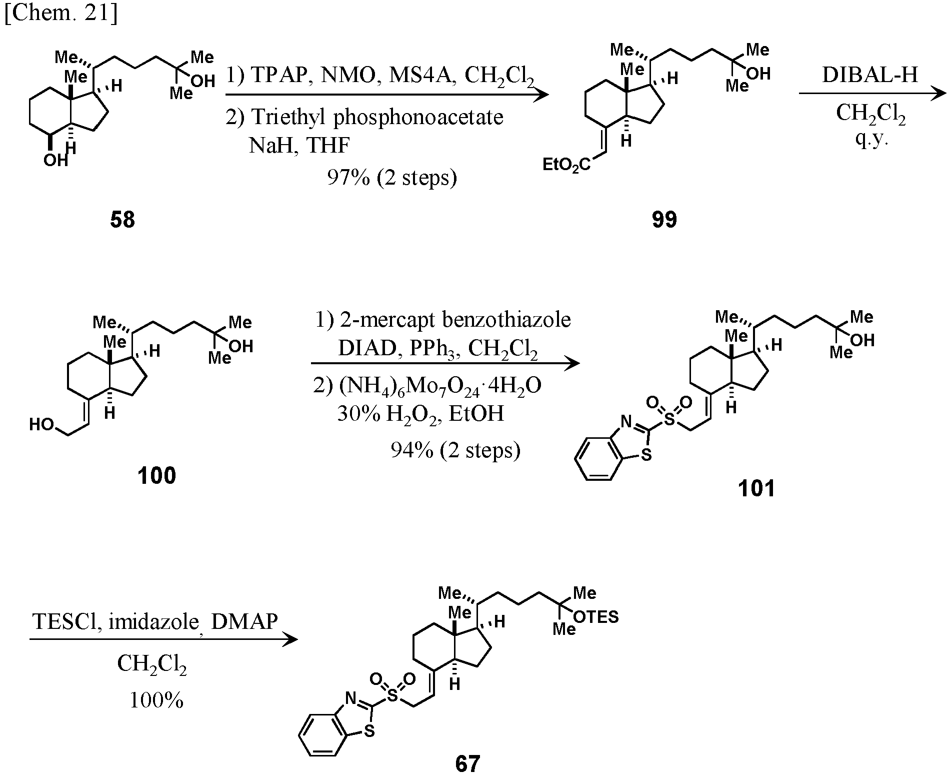

Preparation methods for intermediate Compounds 81 and 93

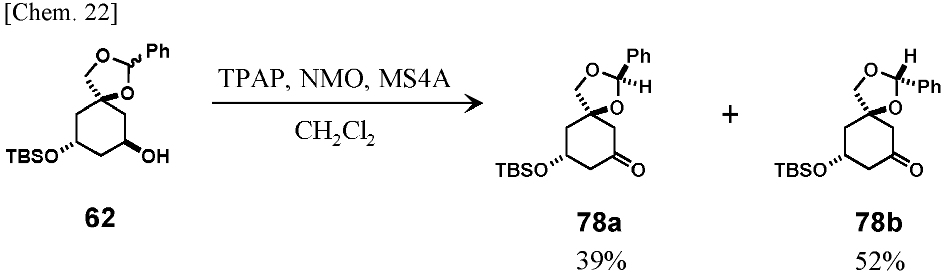

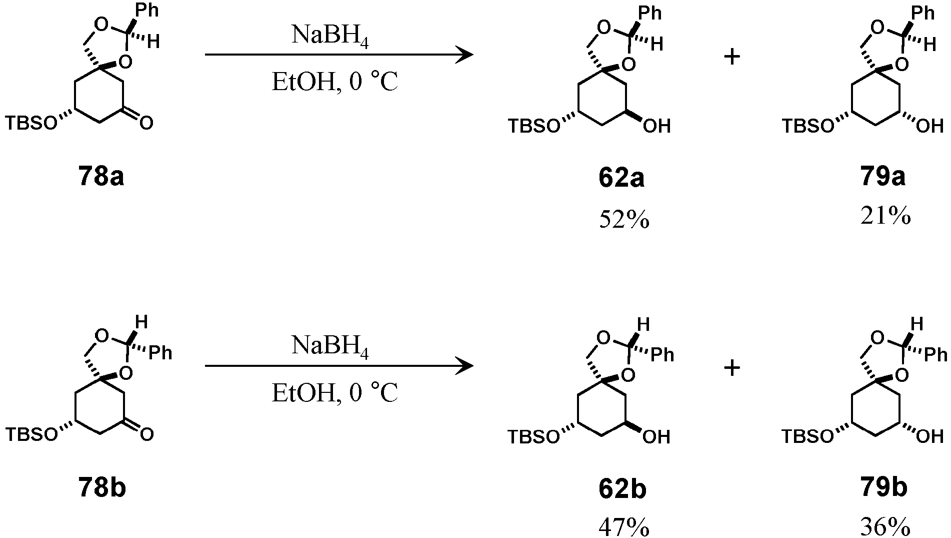

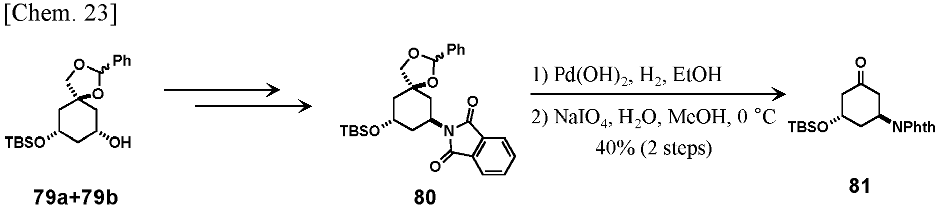

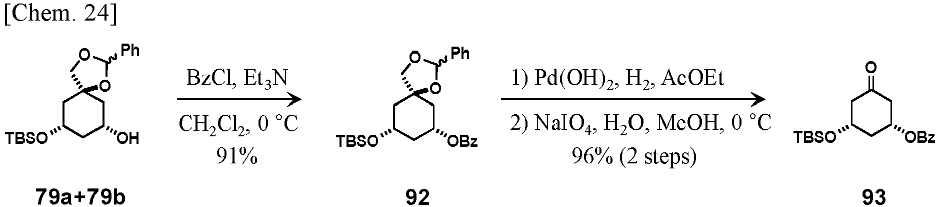

Preparation methods for Compounds 72 to 77 and derivatives thereof 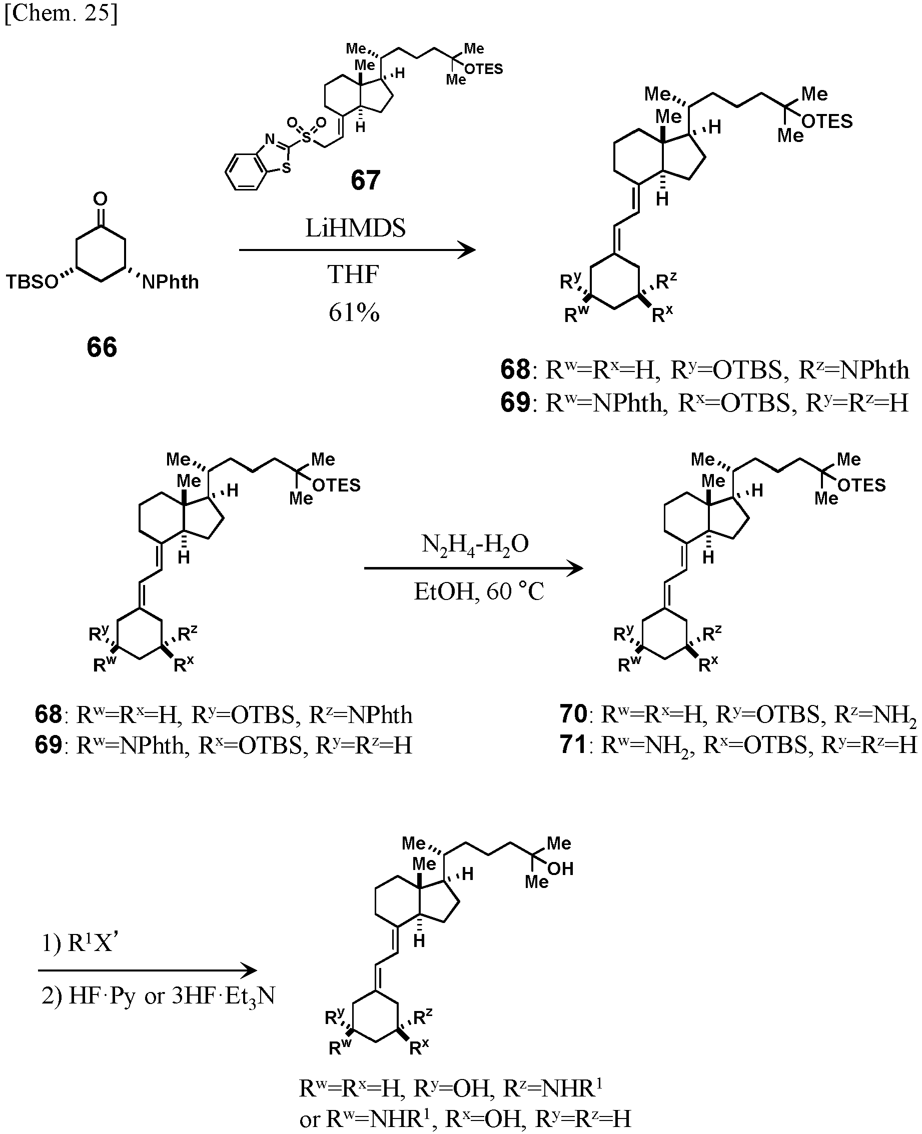 In the scheme, R¹ has the same meaning as defined in Item 1 and X' is halogen or hydroxyl.

Preparation methods for Compounds 82, 83 and Comparative Compound 84 and derivatives thereof 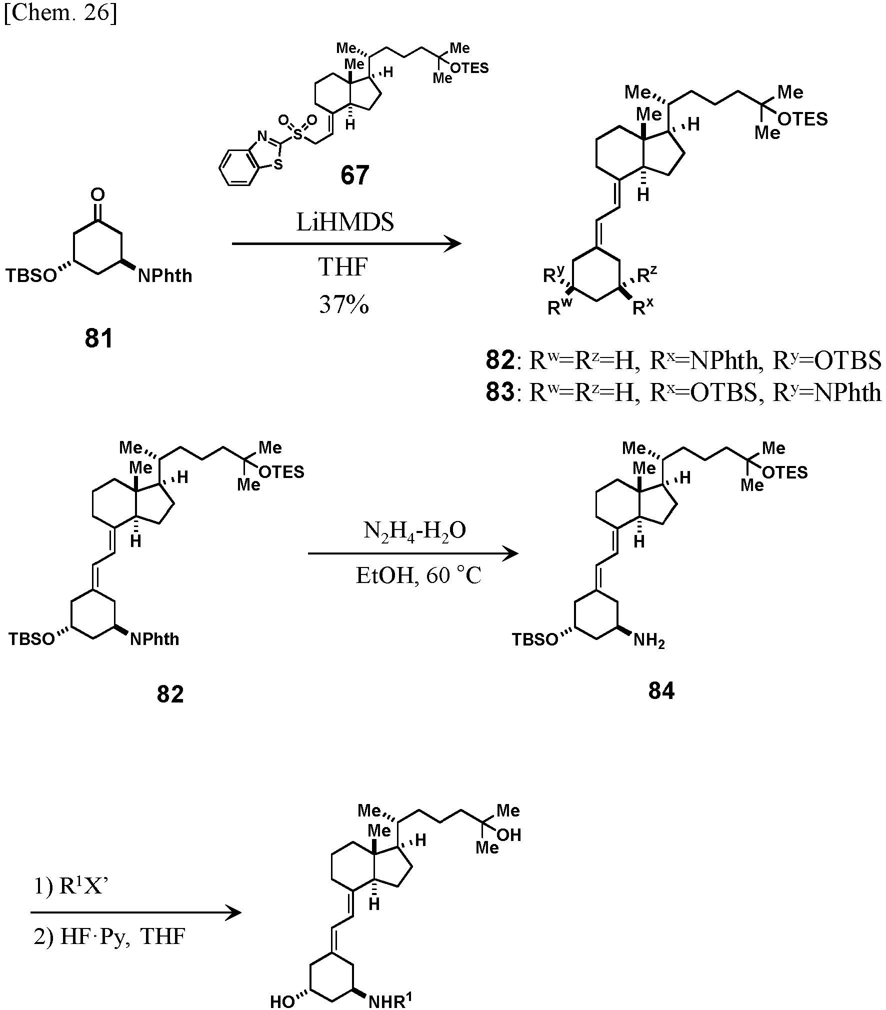 In the scheme, R¹ has the same meaning as defined in Item 1 and X' is halogen or hydroxyl.

Preparation methods for Comparative Compound 98

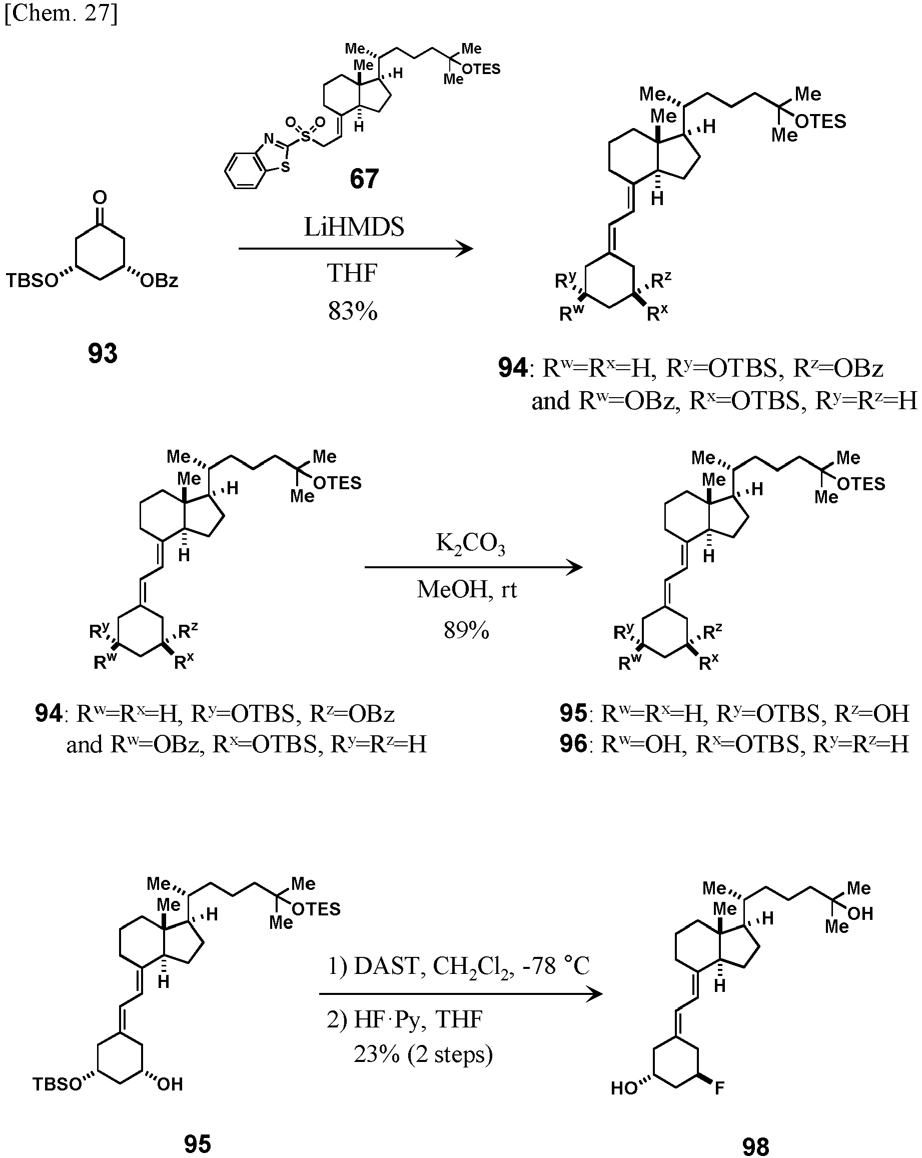

### [Examples]

Unless otherwise stated, preparations were performed under an argon atmosphere using freshly dried solvents. All preparations were monitored by thin-layer chromatography using Merck silica gel 60 F₂₅₄ pre-coated plates (0.25 mm) and were visualized by UV and p-anisaldehyde staining. Flash column chromatography was performed under pressurization using silica gel (particle size 40-100 µm) purchased from Cica or NH silica gel (NH-DM1020) purchased from FUJI SILYSIA CHEMICAL LTD. ¹H NMR spectra were recorded on JNM-ECX 400 or JNM-AL 300. The spectra are referenced internally according to residual solvent signals of CDCl₃ (¹H NMR; δ = 7.26 ppm) or CD₃OD (¹H NMR; δ = 3.34 ppm). Data for ¹H NMR spectra are reported as follows: chemical shift (δ ppm) (multiplicity, coupling constant (Hz), integration). Multiplicity and qualifier abbreviations are as follows: s = singlet, d = doublet, t = triplet, q = quartet, m = multiplet, br = broad. Mass spectra were recorded on JEOL JMS-T100X spectrometer with ESI-MS mode using methanol as solvent or JEOL JMS-MS700V spectrometer with FAB-MS mode using DMSO as solvent.

### Reference Example 1

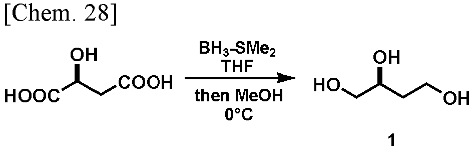

To a solution of BH₃-SMe₂ (100 mL, 1.05 mol) in THF (200 mL) was added a solution of L-malic acid (50 g, 0.37 mol; purchased from Tokyo Chemical Industry Co., Ltd.) in THF (400 mL) dropwise at 0 °C, then the reaction mixture was warmed to room temperature. After stirring for 16 h, the reaction mixture was cooled to 0 °C before adding MeOH (400 mL). After additive 30 min at room temperature, the mixture was evaporated. Moreover, the residue was evaporated with MeOH (200 mL) six times and acetone (200 mL) two times.

### Reference Example 2

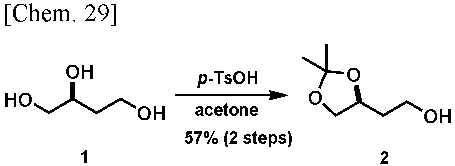

To a crude triol **1** in acetone (600 mL) was added p-TsOH • H₂O (4.0 g). After stirring for 21 h, Et₃N (2 mL) was added. After additive 30 min, the mixture was evaporated. The residue was chromatographed on silica gel (hexane/ethyl acetate; 3:1 to 1:1) to give acetal **2** (31.0 g, 57%, 2 steps) as a colorless oil.

### Reference Example 3

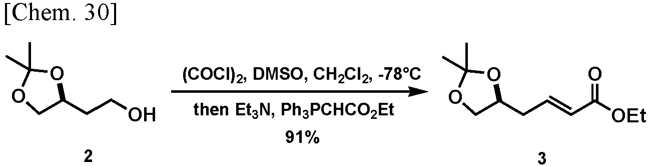

A solution of oxalyl chloride (2.1 mL, 24.1 mmol) in CH₂Cl₂ (110 mL) was cooled to - 78 °C and added dry DMSO (4.3 mL, 60.24 mmol) dropwise. After stirring for 30 min at same temperature, a solution of acetal **2** (1.8 g, 12.1 mmol) in CH₂Cl₂ (10 mL) was added dropwise, and the reaction mixture was stirred for 1 h. To a suspension was added Et₃N (16.8 mL, 120 mmol) dropwise, then the reaction mixture was warmed to room temperature. After additive 1 h, ethyl(triphenylphosphoranylidene)acetate (8.4 g, 24.1 mmol) was added and stirred for 1 day. The reaction was quenched by H₂O, and the aqueous layer was extracted with CH₂Cl₂ three times. The combined mixture was dried over MgSO₄ and concentrated *in vacuo.* The residue was chromatographed on silica gel (hexane/ethyl acetate; 15:1 to 5:1) to give ethyl ester 3 (2.38 g, 91%) as yellow oil.

### Reference Example 4

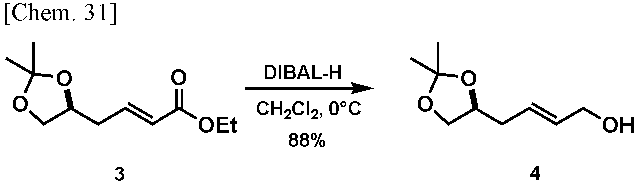

A solution of ethyl ester **3** (2.55 g, 11.9 mmol) in CH₂Cl₂ (120 mL) was added diisobutyl aluminum hydride in hexane (27.4 mL, 27.4 mmol, 1.0 M). After stirring for 2h, the reaction was quenched by careful addition of MeOH (19 mL). The mixture was added sat. Rochelle salt aq. (33 mL) and stirred for 30 min. The organic layer was washed with sat. Rochelle salt aq. three times, and the aqueous layer was extracted with CHCl₃ three times. The combined mixture was dried over MgSO₄ and concentrated *in vacuo.* The residue was chromatographed on silica gel (hexane/ethyl acetate; 6:1 to 2:1) to give alcohol **4** (1.80 g, 88%) as a colorless oil.

### Reference Example 5

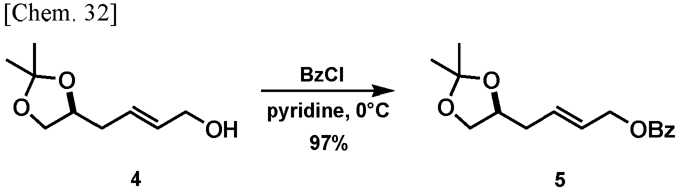

To a solution of allyl alcohol **4** (636 mg, 3.69 mmol) in pyridine (3.7 mL) was added benzoyl chloride (0.64 mL, 5.54 mmol) dropwise at 0 °C, then the reaction mixture was warmed to room temperature. After stirring for 2 h, the reaction mixture was added H₂O. The aqueous layer was extracted with ethyl acetate three times and organic layer was washed with brine. The combined mixture was dried over MgSO₄ and concentrated *in vacuo.* The residue was chromatographed on silica gel (hexane/ethyl acetate; 30:1) to give benzoate ester (991 mg, 97%) as colorless oil.
**¹H NMR** (300 MHz, CDCl₃) δ 8.05-7.40 (m, 5H), 5.90-5.74 (m, 2H), 4.78 (d, *J* = 4.80 Hz, 1H), 4.21-4.12 (m, 1H), 4.03 (dd, *J* = 7.89, 6.18 Hz, 1H), 1.98 (dd, *J* = 7.89, 7.20 Hz, 1H), 2.48-2.28 (m, 2H), 1.41 (s, 3H), 1.35 (s, 3H).
**¹³C NMR** (300 MHz, CDCl₃) δ 166.26, 132.89,z 130.64, 129.54, 128.28, 126.98, 109.00, 74.95, 68.75, 65.18, 36.45, 26.81, 25.56.
**HR-MS ESI** Calcd for C₁₆H₂₀Na₁O₄ [M+Na]⁺ : 299.12593, Found: 299.12835.
**[α]²⁵ _{D}** = +5.40 (c = 2.1 in CHCl₃)

### Reference Example 6

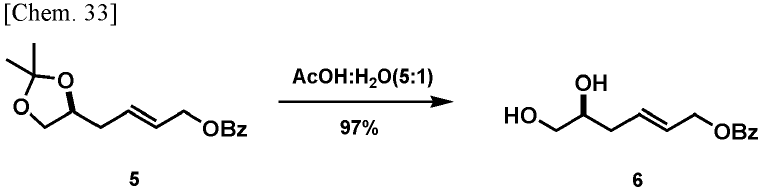

To a benzoate ester **5** (991 mg, 3.59 mmol) was added acetic acid (15.4 mL) and H₂O (2.6 mL). After stirring at room temperature for 12 h, the reaction mixture was evaporated. The residue was chromatographed on silica gel (hexane/ethyl acetate; 1:1) to give diol 6 (825 mg, 97%) as a colorless oil.
**¹H NMR** (300 MHz, CDCl₃) δ 8.04-7.39 (m, 5H), 5.91-5.72 (m, 2H), 4.77 (d, *J =* 5.49 Hz, 1H), 4.17 (dddd, *J =* 6.53, 6.53, 6.53, 3.09 Hz, 1H), 3.64 (dd, *J =* 11.31, 3.09 Hz, 1H), 3.46 (dd, *J =* 11.31, 7.23 Hz, 1H), 2.25 (dd, *J =* 6.54 Hz, 2H).
**¹³C NMR** (300 MHz, CDCl₃) δ 166.49, 133.00, 131.11, 130.03, 129.55, 128.32, 127.18, 71.28, 66.13, 65.27, 36.24.
**HR-MS ESI** Calcd for C₁₃H₁₆Na₁O₄ [M+Na]⁺ : 259.09463, Found: 259.09503.
**[α]²⁵ _{D}** = -6.18 (c = 2.0 in CHCl₃)

### Reference Example 7

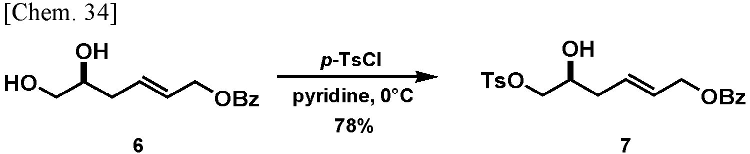

A solution of diol 6 (3.81 g, 16.1 mmol) in pyridine (32 mL) was added*p*-toluenesulfonyl chloride (3.69 g, 19.3 mmol) at 0 °C, then the reaction mixture was slowly warmed to room temperature. After stirring for 4h, the reaction was quenched by H₂O. The aqueous layer was extracted with ethyl acetate three times. The combined organic extracts were washed with brine, dried over MgSO₄, and concentrated *in vacuo.* The residue was chromatographed on silica gel (hexane/ethyl acetate; 5:1 to 1:1) to give tosylate **7** (4.93 g, 78%) as a colorless oil.
**¹H NMR** (300 MHz, CDCl₃) δ 8.04-7.31 (m, 9H), 5.85-5.69 (m, 2H), 4.74 (d, *J =* 4.80 Hz, 2H), 4.04 (dd, *J =* 12.69, 6.51 Hz, 1H), 3.96-3.91 (m, 2H), 2.42 (s, 3H), 2.42-2.26 (m, 2H).
**¹³C NMR** (300 MHz, CDCl₃) δ 166.30, 145.09, 132.98, 132.43, 130.01, 129.91, 129.70, 129.55, 128.32, 128.02, 127.90, 72.92, 68.60, 64.97, 35.82, 21.60.
**HR-MS ESI** Calcd for C₂₀H₂₂Na₁O₆ S₁ [M+Na]⁺ : 413.10348, Found: 413.10279.
**[α]²⁵ _{D}** = +5.36 (c = 2.0 in CHCl₃)

### Reference Example 8

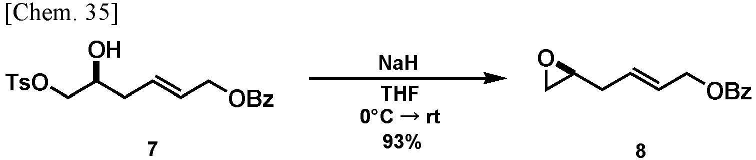

A solution of tosylate 7 (4.93 g, 12.6 mmol) in THF (120 mL) was added NaH (1.01 g, 25.2 mmol, 60%) at 0 °C, then the reaction mixture was warmed to room temperature. After stirring for 8 h, the reaction was quenched by sat. aq. NH₄Cl. The aqueous layer was extracted with ethyl acetate three times. The combined organic extracts were washed with brine, dried over MgSO₄, and concentrated in *vacuo.* The residue was chromatographed on silica gel (hexane/ethyl acetate; 1:0 to 6:1) to give epoxide **8** (2.55 g, 93%) as colorless oil.
**¹H NMR** (300 MHz, CDCl₃) δ 8.06-7.40 (m, 5H), 5.92-5.77 (m, 2H), 4.79 (d, *J =* 4.80 Hz, 2H), 3.03-2.98 (m, 1H), 2.76 (dd, *J =* 4.83 Hz, 1H), 2.51 (dd, *J =* 4.83, 2.76 Hz, 1H), 2.36-2.33 (m, 2H).
**¹³C NMR** (300 MHz, CDCl₃) δ 166.24, 132.89, 130.10, 129.82, 129.54, 128.27, 126.97, 65.09, 50.99, 46.51, 35.00.
**HR-MS ESI** Calcd for C₁₃H₁₄Na₁O₃ [M+Na]⁺ : 241.08406, Found: 241.08341.
**[α]²⁵ _{D}** = -2.69 (c = 2.1 in CHCl₃)

### Reference Example 9

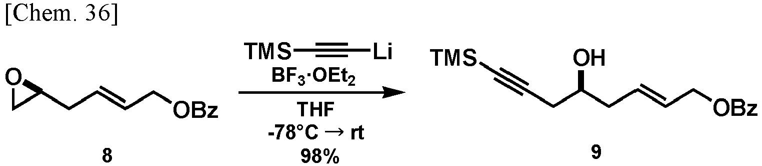

A solution of trimethylsilylacetylene (3.60 mL, 25.7 mmol) in THF (18 mL) was added *n*-BuLi in hexane (8.1 mL, 21.05 mmol, 2.6 M) dropwise at -78 °C and stirred for 30 min. A solution of epoxide **8** (2.55 g, 11.7 mmol) in THF (5 mL) and BF₃-OEt₂ (1.8 mL, 14 mmol) was added dropwise at the same temperature, then the reaction mixture was warmed to room temperature over 2 h. The reaction was quenched with sat. NH₄Cl aq., the aqueous layer was extracted with ethyl acetate three times. The combined organic extracts were washed with brine, dried over MgSO₄, and concentrated *in vacuo.* The residue was chromatographed on silica gel (hexane/ethyl acetate; 30:1 to 10:1) to give alcohol **9** (3.61 g, 98%) as a colorless oil.
**¹H NMR** (300 MHz, CDCl₃) δ 8.05-7.39 (m, 5H), 5.93-5.74 (m, 2H), 4.78 (d, *J =* 5.85 Hz, 2H), 3.82-3.78 (m, 1H), 2.51-2.26 (m, 4H), 0.14 (s, 9H).
**¹³C NMR** (300 MHz, CDCl₃) δ 166.28, 132.87, 131.08, 130.06, 129.52, 128.25, 127.37, 102.74, 87.67, 69.04, 65.19, 38.93, 28.12, -0.03.
**HR-MS ESI** Calcd for C₁₈H₂₄Na₁O₃Si₁ [M+Na]⁺ : 339.13924, Found: 339.14268.
**[α]²⁵ _{D}** = -2.89 (c = 3.5 in CHCl₃)

### Reference Example 10

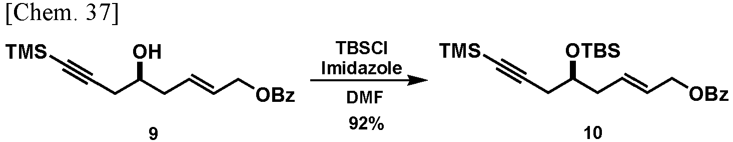

Alcohol **9** (3.61 g) was added imidazole (4.67 g, 68.48 mmol) and *tert*-butyldimethylsilyl chloride (6.88 g, 45.65 mmol) at room temperature, then DMF was added until the reagents were dissolved. After stirring for 4 h, the reaction mixture was diluted with diethyl ether and quenched with sat. NaHCO₃ aq. The aqueous layer was extracted with diethyl ether three times. The combined organic extracts were washed with H₂O and brine, dried over MgSO₄, and concentrated *in vacuo.* The residue was chromatographed on silica gel (hexane/ethyl acetate; 100:1 to 20:1) to give silyl ether **10** (4.52 g, 92%) as a colorless oil.
**¹H NMR** (300 MHz, CDCl₃) δ 8.07-7.41 (m, 5H), 5.92-5.70 (m, 2H), 4.78 (d, *J =* 5.85 Hz, 2H), 3.91-3.83 (m, 1H), 2.49-2.24 (m, 4H), 0.88 (s, 9H), 0.15 (s, 9H), 0.08 (s, 3H), 0.06 (s, 3H).
**¹³C NMR** (300 MHz, CDCl₃) δ 166.46, 132.98, 131.88, 130.38, 129.71, 128.41, 126.90, 104.36, 86.51, 70.78, 65.53, 39.91, 28.60, 25.90, 18.16, 0.17, -4.36, -4.51.
**HR-MS ESI** Calcd for C₁₈H₂₄Na₁O₃Si₁ [M+Na]⁺ : 453.22572 , Found: 453.22357.
**[α]²⁵ _{D}** = -5.34 (c = 2.4 in CHCl₃)

### Reference Example 11

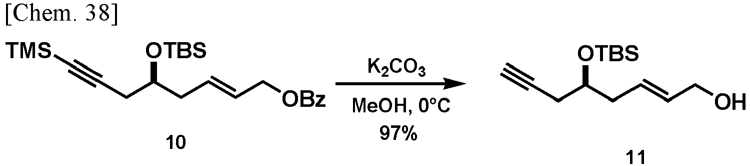

A solution of allyl alcohol **10** (8.28 g, 19.22 mmol) in MeOH (64 mL) was added potassium carbonate (7.97g, 57.67 mmol) at 0 °C, then the reaction mixture was cooled to room temperature. After stirring for 18 h, the reaction was quenched by H₂O. The aqueous layer was extracted with ethyl acetate three times. The combined organic extracts were washed with brine, dried over MgSO₄, and concentrated *in vacuo.* The residue was chromatographed on silica gel (hexane/ethyl acetate; 10:1 to 5:1) to give allyl alcohol **11** (4.74 g, 97%) as colorless oil.
¹H NMR (300 MHz, CDCl₃) δ 5.72-5.69 (m, 2H), 4.1 (d, *J =* 4.1 Hz, 2H), 3.89-3.81 (m, 1H), 2.43-2.24 (m, 2H), 2.31 (dd, *J =* 6.18, 2.73 Hz, 2H), 1.98 (t, *J =* 2.76 Hz, 1H), 0.88 (s, 9H), 0.07 (s, 3H), 0.05 (s, 3H).

### Reference Example 12

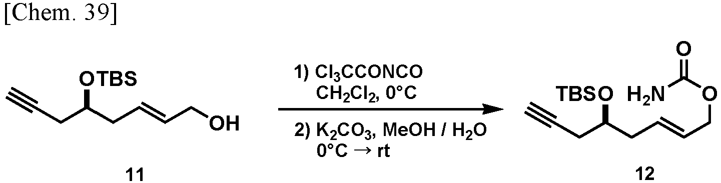

A solution of allyl alcohol **11** (1.46 g, 5.73 mmol) in CH₂Cl₂ (57 mL) was added trichloroacetyl isocyanate (1.4 mL, 11.46 mmol) at 0 °C. The reaction was monitored by thin layer chromatography on silica gel plates (eluent: 4:1 ethyl acetate to hexane). After 15 min, the starting material spot completely converted to a new spot, then the reaction mixture was concentrated *in vacuo.* A solution of the residue in MeOH (36 mL) was added H₂O (8.2 mL) and potassium carbonate (3.2 g, 22.93 mmol) at 0 °C, then the reaction mixture was warmed to room temperature. After stirring for 2 h, the reaction mixture was added H₂O, the aqueous layer was extracted with CH₂Cl₂ three times, dried over MgSO₄, and concentrated *in vacuo.* The residue was filtered through a pad of silica gel to give carbamate **12.**

### Reference Example 13

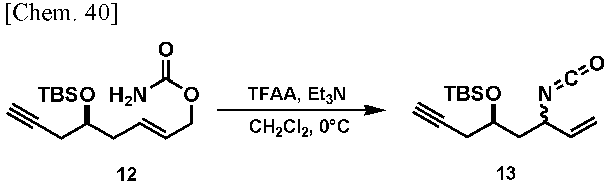

To a solution of carbamate **12** (5.73 mmol) and Et₃N (3.2 mL, 22.93 mmol) in CH₂Cl₂ (230 mL) was added trifluoroacetic anhydride (1.2 mL, 8.60 mmol) dropwise at 0 °C. After stirring for 1 h, the organic layer was washed with H₂O and brine. The combined organic extracts were dried over Na₂SO₄, and concentrated *in vacuo* to crude isocyanate **13.**

### Reference Example 14

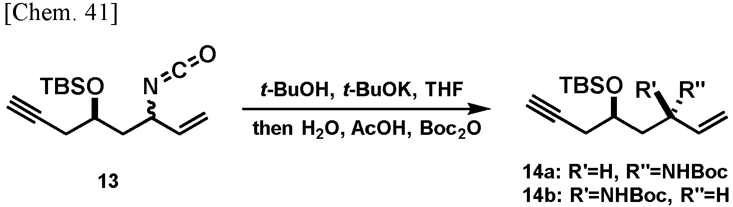

A suspension of *t*-BuOK (0.64 g, 5.73 mmol) and t-BuOH (1.1 mL, 11.46 mmol) in THF (30 mL) was cooled to 0 °C. A crude isocyanate **13** (5.73 mmol) in THF (8 mL) was slowly added dropwise. The reaction was monitored by thin layer chromatography on silica gel plates (eluent: 4:1 ethyl acetate to hexane). After stirring for 25 min, the starting material spot completely converted to new spots. The reaction mixture was added acetic acid (0.33 mL, 5.73 mmol) and H₂O (19 mL), then Boc₂O was added until disappeared amine. After stirring for 13 h, the amine completely converted to product, then the reaction mixture was diluted with ethyl acetate, the aqueous layer was extracted with ethyl acetate three times. The combined organic extracts were washed with brine, dried over MgSO₄, and concentrated *in vacuo.* The residue was chromatographed on silica gel (hexane/ethyl acetate; 100:1 to 20:1) to give Boc amine **14** as a diastereomeric mixture wherein the ratio of **14a** : **14b** was 2 : 1 determined by ¹H NMR.

### Reference Example 15

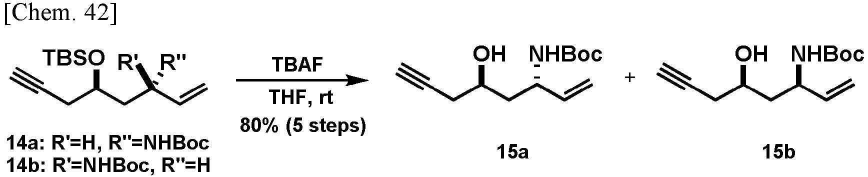

To a solution of diastereomeric mixture **14** (5.73 mmol) in THF (11 mL) was added tetrabutylammonium fluoride in THF (5.7 mL, 1 M). After stirring for 2.5 h, the reaction mixture was evaporated. The residue was chromatographed on silica gel (hexane/ethyl acetate; 50:1 to 1:1) to give alcohol **15a** (0.72 g, 52%) and alcohol **15b** (0.39 g, 28%) as a single diastereomer.
**15a:** ¹H NMR (300 MHz, CDCl₃) δ 5.90-5.78 (m, 1H), 5.24-5.12 (m, 2H), 4.76 (d, *J =* 8.9 Hz, 1H), 4.42 (br, 1H), 3.82 (br, 1H), 2.51-2.33 (m, 2H), 2.04 (t, *J =* 2.4 Hz, 1H), 1.79-1.60 (m, 2H), 1.45 (s, 9H).
**15b:** ¹H NMR (300 MHz, CDCl₃) δ 5.84-5.72 (m, 1H), 5.24-5.11 (m, 2H), 4.72 (br, 1H), 4.25 (br, 1H), 3.93-3.85 (m, 1H), 2.51-2.35 (m, 2H), 2.06 (t, *J* = 2.4 Hz, 1H), 1.78-1.65 (m, 2H), 1.44 (s, 9H).

### Reference Example 16

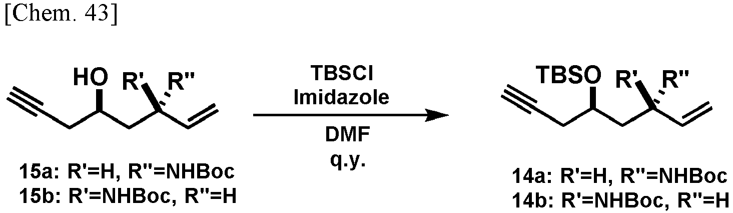

To the alcohol **15a** (35 mg, 0.146 mmol) was added imidazole (60 mg, 0.875 mmol) and *tert*-butyldimethylsilyl chloride (88 mg, 0.583 mmol) at room temperature, then DMF was added until the reagents were dissolved. After stirring for 2 h, the reaction mixture was diluted with diethyl ether and quenched with sat. NaHCO₃ aq. The aqueous layer was extracted with diethyl ether three times. The combined organic extracts were washed with H₂O and brine, dried over MgSO₄, and concentrated *in vacuo.* The residue was chromatographed on silica gel (hexane/ethyl acetate; 30:1) to give **14a** (52 mg, q.y.) as colorless oil.
¹H NMR (300 MHz, CDCl₃) δ 5.83-5.72 (m, 1H), 5.18-5.07 (m, 2H), 4.26 (br, 1H), 4.01-3.93 (m, 1H), 2.43-2.27 (m, 2H), 2.01 (t, *J =* 2.4 Hz, 1H), 1.79-1.72 (br, 2H), 1.42 (s, 9H), 0.90 (s, 9H), 0.10 (s, 3H), 0.09 (s, 3H).

By following the same procedure described for **14a, 14b** (35 mg, q.y.) was obtained from **15b** (23 mg, 0.094 mmol) as colorless oil.
¹H NMR (300 MHz, CDCl₃) δ 5.83-5.72 (m, 1H), 5.21-5.08 (m, 2H), 4.75 (br, 1H), 4.22-4.13 (m, 1H), 3.94-3.86 (m, 1H), 2.40-2.37 (m, 2H), 2.00 (t, *J =* 2.8 Hz, 1H), 1.86-1.59 (m, 2H), 1.43 (s, 9H), 0.91 (d, *J =* 3.5 Hz, 9H), 0.09 (s, 3H), 0.08 (d, *J =* 1.7 Hz, 3H).

### Reference Example 17

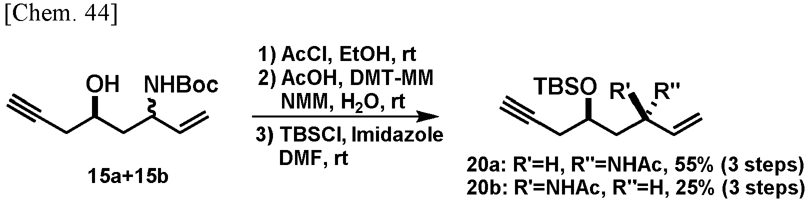

Acetyl chloride (1.1 mL, 15.09 mmol) was slowly added to EtOH (5.0 mL) dropwise at 0 °C. After stirring for 30 min, this solution was added to alcohol **15** (0.12 g, 0.50 mmol). After additional stirring for 2 h, the reaction mixture was evaporated. The residue was washed with Et₂O, affording amine as a yellow solid. To a solution of above amine (0.50 mmol) in H₂O (5 mL) was added acetic acid (0.086 mL, 1.5 mmol), DMT-MM (0.60 g, 2.01 mmol), *N-*methylmorpholine (0.33 mL, 3.01 mmol) and stirred for 2h. The aqueous layer was extracted with ethyl acetate three times. The combined organic extracts were washed with brine, dried over MgSO₄, and concentrated *in vacuo.* The residue was chromatographed on silica gel (hexane/ethyl acetate; 1:2 to 0:1) to give acetamide **α** and **β** as a single diastereomer. To α-acetamide (about 0.33 mmol) was added imidazole (92 mg, 1.34 mmol) and tert-butyldimethylsilyl chloride (102 mg, 0.67 mmol) at room temperature, and then DMF was added until the reagents were dissolved. After stirring for 1 h, the reaction mixture was diluted with diethyl ether and quenched with sat. aq. NaHCO₃. The aqueous layer was extracted with diethyl ether three times. The combined organic extracts were washed with H₂O and brine, dried over MgSO₄, and concentrated *in vacuo.* The residue was chromatographed on silica gel (hexane/ethyl acetate; 4:1 to 2:1) to give **20a** (82.0 mg, 55%, 3 steps) as a colorless oil.

By following the same procedure described for **20a, 20b** (37.2 mg, 25%, 3 steps) was obtained from β-acetamide (about 0.17 mmol) as a colorless oil.

### Reference Example 18

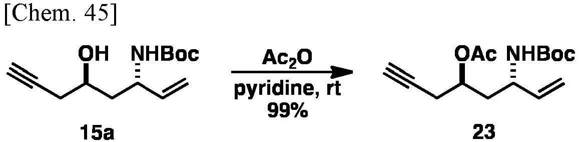

A solution of alcohol **15a** (0.62 g, 2.61 mmol) in pyridine (2.5 mL) was added acetic anhydride (2.5 mL) at room temperature. After stirring for 5.5 h, the reaction mixture was evaporated. The residue was chromatographed on silica gel (hexane/ethyl acetate; 10:1 to 5:1) to give acetate ester **23** (0.72 g, 99%).

### Reference Example 19

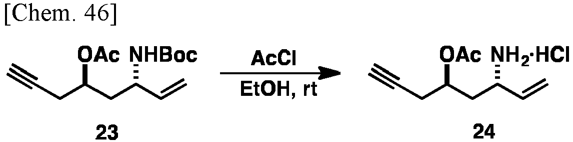

Acetyl chloride (2.8 mL, 38.58 mmol) was slowly added to EtOH (13.0 mL) dropwise at 0 °C. After stirring for 30 min, this solution was added to acetate ester **23** (0.72 g, 2.57 mmol). After additional stirring for 3 h, the reaction mixture was evaporated. The residue was washed with Et₂O, affording amine **24** as a yellow solid.

### Reference Example 20

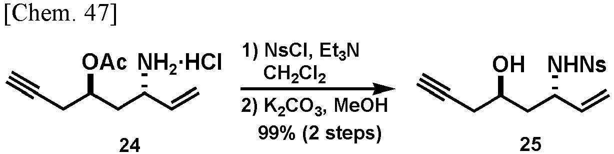

To a solution of crude amine **24** (2.57 mmol) in CH₂Cl₂ (13 mL) was added Et₃N (0.9 mL, 6.17 mmol), *o*-nitrobenzenesulfonyl chloride (0.68 g, 3.09 mmol) at 0 °C, then the reaction mixture was warmed to room temperature. After stirring for 6.5 h, the reaction was quenched by H₂O. The aqueous layer was extracted with ethyl acetate three times. The combined organic extracts were washed with brine, dried over MgSO₄, and concentrated *in vacuo.* The residue was filtered through a pad of silica gel to give acetate ester as colorless oil. To a solution of acetate ester (2.57 mmol) in MeOH (9 mL) was potassium carbonate (0.53 g, 3.86 mmol) at 0 °C, then the reaction mixture was warmed to room temperature. After stirring for 15 h, H₂O was added, and the aqueous layer was extracted with ethyl acetate three times. The combined organic extracts were washed with brine, dried over MgSO₄, and concentrated *in vacuo.* The residue was chromatographed on silica gel (hexane/ethyl acetate; 4:1 to 1:1) to give nosylate **25** (0.82 g, 99%) as a colorless oil.

### Reference Example 21

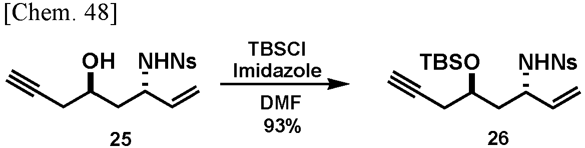

To the nosylate **25** (0.82 g, 2.53 mmol) was added imidazole (0.69 g, 10.14 mmol) and *tert*-butyldimethylsilyl chloride (0.77 g, 5.07 mmol) at room temperature, then DMF was added until the reagents were dissolved. After stirring for 3 h, the reaction mixture was diluted with diethyl ether and quenched with sat. NaHCO₃ aq. The aqueous layer was extracted with diethyl ether three times. The combined organic extracts were washed with H₂O and brine, dried over MgSO₄, and concentrated *in vacuo.* The residue was chromatographed on silica gel (hexane/ethyl acetate; 15:1 to 5:1) to give **26** (1.03 g, 93%) as a colorless oil.
**¹H NMR** (300 MHz, CDCl₃) δ 8.08-7.68 (m, 4H), 5.73 (d, *J =* 6.18 Hz, 1H), 5.55 (ddd, *J* = 13.08, 7.56, 5.52 Hz, 1H), 5.05 (dd, *J =* 12.72, 0.69 Hz, 1H), 4.90 (dd, *J =* 7.56, 0.69 Hz, 1H), 4.24-4.15 (m, 1H), 4.09-4.03 (m, 1H), 2.40 (ddd, *J =* 12.36, 3.09, 2.40 Hz, 1H), 2.32 (ddd, *J =* 12.36, 5.85, 2.07 Hz, 1H), 2.00 (t, *J =* 2.07 Hz, 1H), 1.89 (ddd, *J =* 10.65, 6.87, 2.07 Hz, 1H), 1.76 (ddd, *J=* 10.65, 5.85, 2.73 Hz, 1H), 0.92 (s, 9H), 0.17 (s,3H), 0.13 (s, 3H).

### Reference Example 22

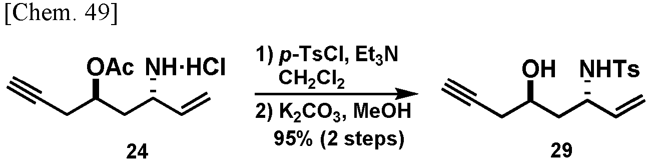

To a solution of crude amine **24** (21.9 mg, 0.105 mmol) in CH₂Cl₂ (0.8 mL) was added Et₃N (0.035 mL, 0.25 mmol), *p*-toluenesulfonyl chloride (24.2 mg, 0.126 mmol) at 0 °C, then the reaction mixture was warmed to room temperature. After stirring for 14 h, the reaction was quenched by H₂O. The aqueous layer was extracted with ethyl acetate three times. The combined organic extracts were washed with brine, dried over MgSO₄, and concentrated *in vacuo.* The residue was filtered through a pad of silica gel to give acetate ester (23.9 mg, not pure) as colorless oil. To a solution of acetate ester (0.071 mmol) in MeOH (0.7 mL) was potassium carbonate (15 mg, 0.11 mmol) at 0 °C, then the reaction mixture was warmed to room temperature. After stirring for 3 h, H₂O was added, and the aqueous layer was extracted with ethyl acetate three times. The combined organic extracts were washed with brine, dried over MgSO₄, and concentrated *in vacuo.* The residue was chromatographed on silica gel (hexane/ethyl acetate; 4:1 to 1:1) to give tosylate **29** (19.9 mg, 95%) as a colorless oil.

### Reference Example 23

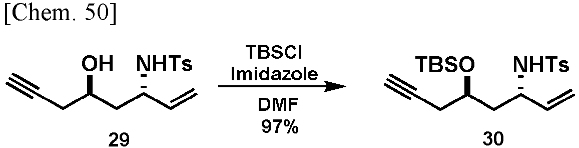

To the tosylate **29** (19.9 mg, 0.068 mmol) was added imidazole (19 mg, 0.271 mmol) and *tert*-butyldimethylsilyl chloride (21 mg, 0.135 mmol) at room temperature, then DMF was added until the reagents were dissolved. After stirring for 3 h, the reaction mixture was diluted with diethyl ether and quenched with sat. NaHCO₃ aq. The aqueous layer was extracted with diethyl ether three times. The combined organic extracts were washed with H₂O and brine, dried over MgSO₄, and concentrated *in vacuo.* The residue was chromatographed on silica gel (hexane/ethyl acetate; 6:1 to 3:1) to give **30** (26.8 mg, 97%) as a colorless oil.
**¹H NMR** (300 MHz, CDCl₃) δ 7.73-7.27 (m, 4H), 5.70 (d, *J =* 5.16 Hz, 1H), 5.63 (ddd, *J* = 17.19, 10.29, 6.54 Hz, 1H), 5.12 (dd, *J =* 17.19, 1.05 Hz, 1H), 5.01 (dd, *J =* 10.29, 1.05 Hz, 1H), 4.04-3.90 (m, 2H), 2.42 (s, 3H), 2.25 (ddd, *J =* 16.83, 4.80, 2.73 Hz, 1H), 2.10 (ddd, *J =* 16.83, 8.22, 2.73 Hz, 1H), 1.95 (t, *J =* 2.73 Hz, 1H), 1.85- 1.67 (m, 2H), 0.92 (s, 9H), 0.12 (s,3H), 0.10 (s, 3H).

### Reference Example 24

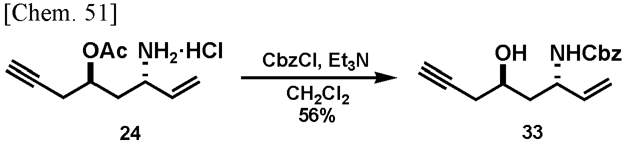

To a solution of crude amine **24** (26.7 mg, 0.122 mmol) in CH₂Cl₂ (1.0 mL) was added Et₃N (0.041 mL, 0.293 mmol), benzyl chloroformate (0.021 mL, 0.147 mmol) at 0 °C, then the reaction mixture was warmed to room temperature. After stirring for 14 h, the reaction was quenched by H₂O. The aqueous layer was extracted with ethyl acetate three times. The combined organic extracts were washed with brine, dried over MgSO₄, and concentrated *in vacuo.* The residue was chromatographed on silica gel (hexane/ethyl acetate; 4:1 to 1:1) to give NHCbz **33** (18.6 mg, 56%) as a colorless oil.

### Reference Example 25

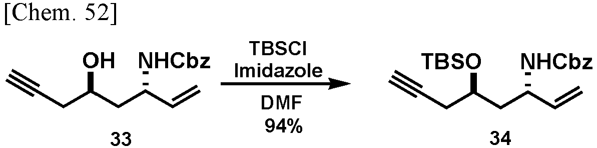

To a solution of NHCbz **33** (16.0 mg, 0.059 mmol) in CH₂Cl₂ (0.6 mL) was added 2,6-lutidine (0.041 mL, 0.351 mmol) and *tert*-butyldimethylsilyl trifluoromethanesulfonate (0.046 mL, 0.176 mmol) at room temperature. After stirring for 2 h, the reaction was quenched by sat. NaHCO₃ aq. The aqueous layer was extracted with CH₂Cl₂ three times. The combined organic extracts were dried over MgSO₄, and concentrated *in vacuo.* The residue was chromatographed on silica gel (hexane/ethyl acetate; 10:1 to 3:1) to give **34** (21.4 mg, 94%) as a colorless oil.

### Reference Example 26

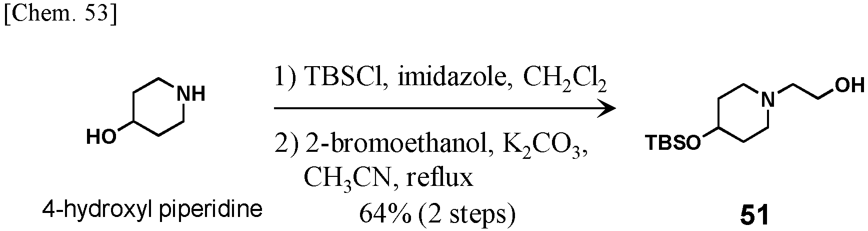

Imidazole (12.0 mg, 0.0461 mmol), TBSCI (4 µL, 0.0690 mmol) were successively added to a solution of 4-hydroxy piperidine (306.7 mg, 3.032 mmol) in dichloromethane (10.0 mL) at room temperature and stirred for 1 d. The reaction mixture was washed with H₂O, sat. NaHCO₃ aq. and brine, dried over MgSO₄, and concentrated *in vacuo.* The residue was evapolated with toluene for removing residual reagents to give crude product (692.3 mg, not pure). To a solution of above crude product (692.3 mg) in acetonitrile (1.5 mL) was added 2-bromoethanol (0.45 mL, 6.367 mmol) and potassium carbonate (0.67 g, 4.851 mmol) at room temperature and then the mixture was heated under reflux for 4 h. The reaction mixture was filtered through a pad of celite and the resulting filtrate was concentrated. Purification by flash chromatography on silica gel (NH silica gel, hexane/ethyl acetate; 10:0 to 1:1) to give **51** (500.9 mg, 64%).

### Reference Example 27

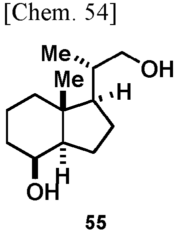

To a solution of vitamin D₂ (8.0 g, 20.2 mmol; purchased from Tokyo Chemical Industry Co., Ltd.) in dichloromethane (270 mL) was added methanol (112 mL) and the solution was cooled to - 78 °C. Ozone was passed through the solution at - 78 °C for 3 h. After flushing nitrogen to the solution to remove the residual ozone, the resulting mixture was treated with NaBH₄ (4.56 g, 120 mmol) and stirred for 30 min at - 78 °C, and then allowed to warm to room temperature. The reaction was quenched by the addition of 0.5 M aq. HCl, and extracted with dichloromethane. The organic phase was washed with water and dried over MgSO₄, and concentrated *in vacuo.* The residue was chromatographed on silica gel (hexane/ethyl acetate; 6:1 to 2:1) to give diol **55** (4.46 g, >99%).
¹H NMR (CDCl₃) δ 4.09 (d, *J =* 2.6 Hz, 1H), 3.64 (dd, *J =* 3.3, 10.6 Hz, 1H), 3.39 (dd, *J =* 6.6, 10.6 Hz, 1H), 1.99 (dd, *J =* 2.6, 13.2 Hz, 1H), 1.88-1.76 (m, 3H), 1.63-1.29 (m. 9H), 1.20-1.12 (m. 2H), 1.03 (d, *J* = 6.6 Hz, 3H), 0.95 (s, 3H)

### Reference Example 28

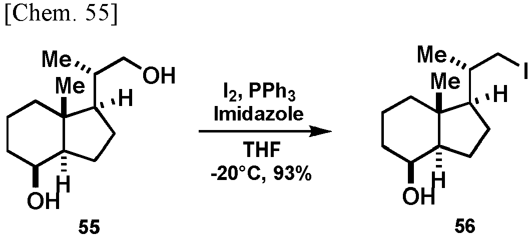

To a solution of diol **55** (1.00 g, 4.71 mmol) in THF (20 mL) was successively added imidazole (960 mg, 14.14 mmol), triphenylphosphine (1.20 g, 5.65 mmol), iodide (1.32 g, 5.18 mmol) at - 20°C and stirring for 15 min. The reaction mixture was warmed to room temperature. After additional stirring for 2 h, the mixture was cooled to 0 °C before adding sat. NaHCO₃ aq. The aqueous layer was extracted with ethyl acetate three times. The combined organic extracts were washed with sat. Na₂S₂O₃ aq. and H₂O, dried over MgSO₄, and concentrated *in vacuo.* The residue was chromatographed on silica gel (hexane/ethyl acetate; 10:1) to give iodide **56** (1.41 g, 93%).

### Reference Example 29

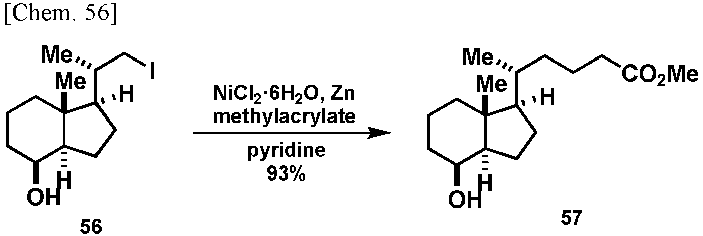

This step was carried out according to the method described in Antonio Mourino et al. Chem. Eur. J. 2010, 16, 1432-1435. A suspension of activated zinc (8.32 g, 127.26 mmol) in pyridine (90 mL) were successively added methyl acrylate (13.3 mL, 148 mmol) and NiCl₂·6H₂O (3.78 g, 15.91 mmol) at room temperature. The mixture was stirred at 60 °C for 1 h and then cooled to 0 °C. A solution of iodide **56** (3.42 g, 10.61 mmol) in pyridine (10 mL) was added dropwise. The reaction mixture was stirred for 2 h, and then diluted with ethyl acetate. The mixture was filtered through a pad of celite. The filtrate was washed with 1 M HCl aq. two times, and the aqueous layer was extracted with ethyl acetate three times. The combined organic extracts were washed with H₂O and brine, dried over MgSO₄, and concentrated *in vacuo.* The pyridine hydrochloride was removed by filtering through a plug of cotton, and the filtrate was evaporated. The residue was chromatographed on silica gel (hexane/ethyl acetate; 8:1 to 4:1) to give methyl ester **57** (2.77 g, 93%).

### Reference Example 30

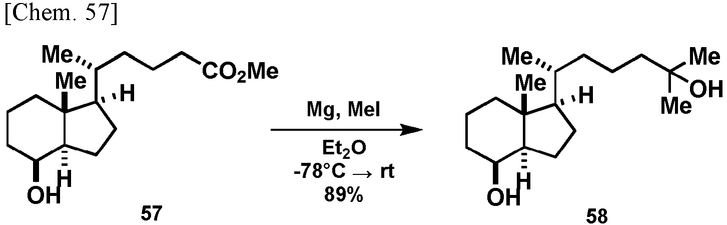

Activated magnesium (2.91 g, 119.6 mmol) in dry diethyl ether (46 mL) was added methyl iodide (5.7 mL, 92 mmol) dropwise at 0 °C. To a solution of methyl ester **57** (5.17 g, 18.30 mmol) in dry diethyl ether (90 mL) was added above Grignard reagent (46 mL, 2 m) dropwise at -78 °C. The mixture was stirred for 15 min at same temperature and then warmed to room temperature. After stirring for 2.5 h, the reaction was quenched by careful addition of sat. NH₄Cl aq. The aqueous layer was extracted with ethyl acetate three times. The combined organic extracts were washed with brine, dried over MgSO₄, and concentrated *in vacuo.* The residue was chromatographed on silica gel (Hexane/ethyl acetate; 4:1 to 2:1) to give diol **58** (4.59 g, 89%).

### Reference Example 31

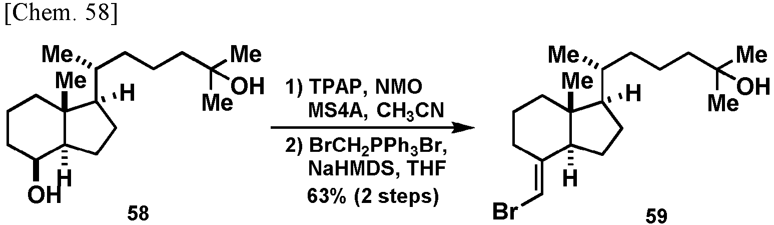

A solution of diol **58** (300 mg, 1.06 mmol) in dry acetonitrile (10 mL) was added molecular sieves 4 angstroms (530 mg) and *N*-methyl morpholine *N-*oxide (250 mg, 2.12 mmol) and stirred for 30 min. Tetraisopropylammonium perruthenate (15 mg, 0.043 mmol) was added, then the reaction mixture was warmed to room temperature. After stirring for 2.5 h, the mixture was filtered through a pad of silica gel, and the filtrate was evaporated to give crude ketone. A suspension of (bromomethyl)triphenylphosphonium bromide (2.32 g, 5.31 mmol) in toluene (50 mL) was sonicated for 30 min at room temperature and evaporated with toluene (50 mL) three times at 50 °C. To the suspension was added dry THF (7 mL) and cooled to 0 °C. Sodium bis(trimethylsilyl)amide in THF (2.8 mL, 5.20 mmol, 1.9 M) was added dropwise. After stirring for 30 min, a solution of above crude ketone (1.06 mmol) in THF (1.2 mL) was added dropwise. After additive 3 h, the reaction was quenched by the addition of a few drops of sat. NH₄Cl aq. The mixture was filtered, and the filtrate was evaporated. The residue was chromatographed on silica gel (hexane/ethyl acetate; 20:1 to 10:1) to give bromo olefin **59** (239 mg, 63%, 2 steps).

### Reference Example 32

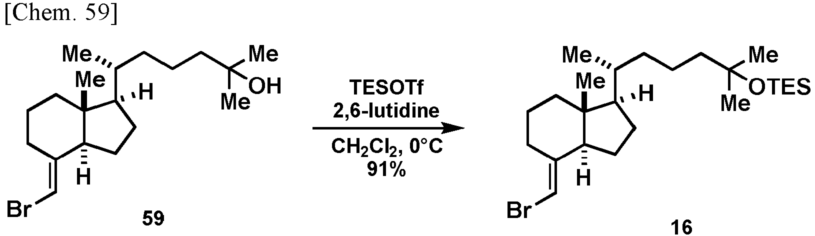

A solution of bromo olefin **59** (0.51 g, 1.45 mmol) in CH₂Cl₂ (7.2 mL) was added 2,6-lutidine (0.34 mL, 2.89 mmol) and dropwised triethylsilyl trifluoromethanesulfonate (0.42 mL, 1.88 mmol). After stirring for 2.5 h, the reaction was quenched by sat. aq. NaHCO₃. The aqueous layer was extracted with CH₂Cl₂ three times. The combined organic extracts were dried over MgSO₄, and concentrated *in vacuo.* The residue was chromatographed on silica gel (hexane/dichloromethane; 10:1) to give diol **16** (0.62 g, 91%).
**¹H NMR** (300 MHz, CDCl₃) δ 5.64 (s, 1H), 2.89-2.84 (m, 1H), 2.04-1.15 (m, 18H), 1.18 (s, 6H), 0.97-0.62 (m, 12H), 0.56 (s, 3H), 0.56 (q, *J* = 7.89 Hz, 2H).

### Reference Example 33

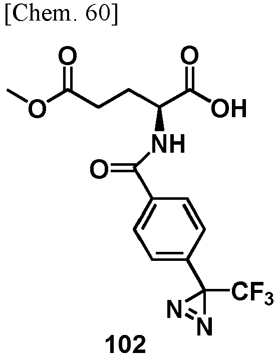

To a solution of 4-[3-(trifluoromethyl)-3*H*-diazirin-3-yl]benzoic acid (0.500 g, 2.17 mmol; purchased from Tokyo Chemical Industry Co., Ltd.) and *N*-hydroxysuccinimide (0.250 g, 2.17 mmol) in dichloromethane (15 mL) was added *N*,*N*'-dicyclohexylcarbodiimide (0.450 g, 2.17 mmol) at 0 °C and the mixture was stirred at room temperature overnight. The precipitation was filtered off and the filtrate was concentrated under reduced pressure to give a crude product. To a solution of 5-methyl L-glutamate (0.380 g, 2.36 mmol) in acetonitrile/H₂O (10:3, 13 mL) was added the crude product and triethylamine (0.660 g, 6.52 mmol) and the mixture was stirred at room temperature overnight. The mixture was evaporated and the residue was dissolved in ethyl acetate, washed with aq. HCl (1 M), water, brine, and dried (MgSO₄). The solvent was evaporated to afford crude product **102** (0.890 g), which was used in next step without further purification.

### Reference Example 34

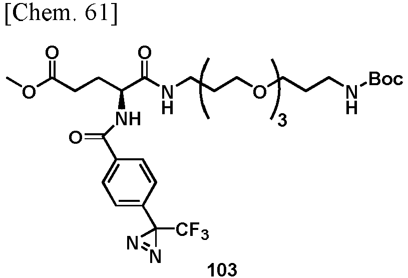

To a mixture of crude **102** (0.890 g) and *N*-Boc-4,7,10-trioxa-1,13-tridecanediamine (0.690 g, 2.15mmol) in dichloromethane (10 mL) was added 1-(3-dimethylaminopropyl)-3-ethylcarbodiimide hydrochloride (0.50 g, 2.6 mmol), 4-dimethylaminopyridine (0.32 g, 2.85 mmol) and 1-hydroxybenzotriazole monohydrate (0.35 g, 2.59 mmol) at 0 °C, and the mixture was stirred at room temperature overnight. The mixture was washed with sat. aq. NaHCO₃, water, and brine. The organic layer was dried over anhydrous magnesium sulfate and concentrated *in vacuo.* The residue was purified by column chromatography (ethyl acetate:hexane; 2:1) to yield **103** (0.760 g, 1.13 mmol, 52% for 3 steps).

### Reference Example 35

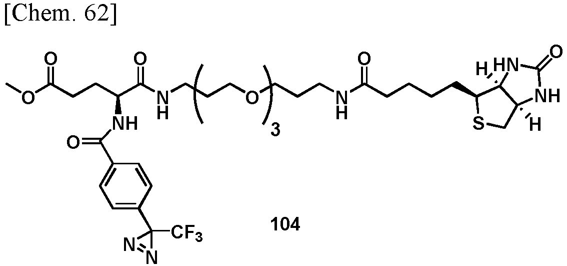

To a solution of **103** (0.760 g, 1.13 mmol) in dichloromethane (10 mL) was added trifluoroacetic acid (2 mL) at 0 °C and the mixture was stirred at room temperature for 2 h. The solvent was evaporated and the residue was dissolved in chloroform, washed with sat. aq. Na₂CO₃, water and brine. The organic layer was dried over anhydrous magnesium sulfate and concentrated *in vacuo* to afford a crude product (0.60 g). To a solution of the crude product (0.60 g) in *N*,*N*'-dimethylformamide (5 mL) was added NHS-Biotin (0.36 g, 1.04 mmol) and triethylamine (0.32 g, 3.12 mmol), and the mixture was stirred at room temperature overnight. The solvent was evaporated and the residue was purified by column chromatography (methanol: chloroform; 1:10) to yield **104** (0.70 g, 0.87 mmol, 77% for steps).

### Reference Example 36

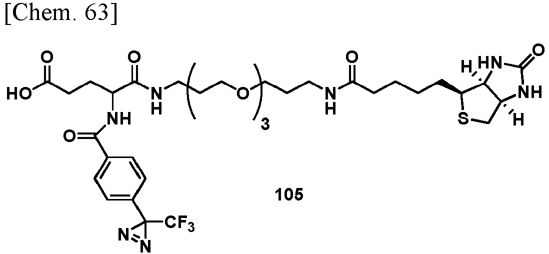

To a mixture of **104** (0.56 g, 0.70 mmol) in tetrahydrofuran (3 mL) and water (1 mL) was added lithium hydroxide monohydrate (59 mg, 1.4 mmol) at 0°C and the mixture was stirred at room temperature for 2h. The mixture was acidified with aq. HCl (1 M) and extracted with chloroform three times. The combined extracts were dried over anhydrous magnesium sulfate and concentrated *in vacuo.* The residue was purified by column chromatography (methanol:chloroform; 1:3) to yield **105** (0.46 g, 0.59 mmol, 84%).

### Reference Example 37

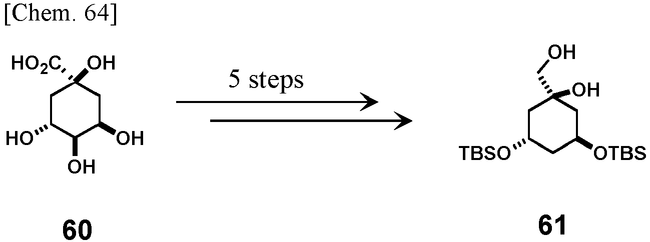

This step was carried out according to the method described in John H. White et al. Proc. Natl. Acad. Soc. 2008, 105, 8250-8255. 1,2-Diol **61** (3.69 g, 9.442 mmol) was obtained in 39% yield from D-(-)-Quinic acid **60** (4.72 g, 24.562 mmol).

### Reference Example 38

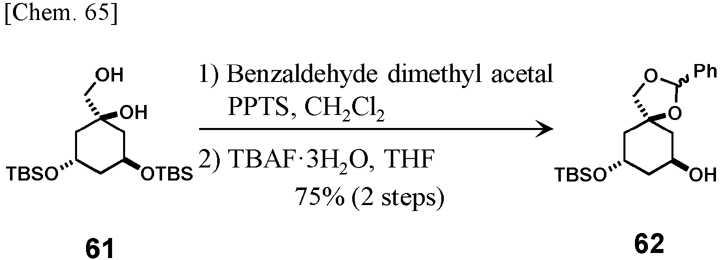

A solution of diol **61** (7.69 g, 19.680 mmol) in CH₂Cl₂ (190 mL) was added benzaldehyde dimethyl acetal and pyridinium p-toluenesulfonate at room temperature. After stirring for 7 h, the reaction was quenched by sat. NaHCO₃ aq. The aqueous layer was extracted with CH₂Cl₂ three times. The combined organic extracts were dried over MgSO₄, and concentrated *in vacuo.* The residue was chromatographed on silica gel (Hexane/dichloromethane; 50:1) to give benzylidene acetal (12.76 g) including residual reagent (benzaldehyde dimethyl acetal). To a solution of above benzylidene acetal (12.76g) in THF (480 mL) was added a solution of TBAF·3H₂O (65 mL, 1.0 M in THF) at 0 °C. After stirring for 5 h, the reaction mixture was quenched with sat. NH₄Cl aq. The aqueous layer was extracted with ethyl acetate three times. The combined organic extracts were washed with brine, dried over MgSO₄, and concentrated *in vacuo.* The residue was chromatographed on silica gel (Hexane/ethyl acetate; 50:1 to 2:1) to give alcohol 62 (5.38 g, 75%).

### Reference Example 39

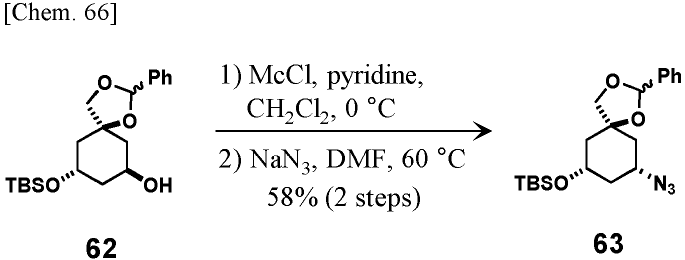

A solution of alcohol **62** (0.57 g, 1.556 mmol) in CH₂Cl₂ (5.0 mL) was added pyridine (0.50 mL, 6.222 mmol) and chloromethylsulfonyl chloride (0.28 mL, 3.111 mmol) at 0 °C. After stirring for 2 h, the reaction was quenched with sat. NH₄Cl aq. The aqueous layer was extracted with CH₂Cl₂ three times. The combined organic extracts were dried over MgSO₄, and concentrated *in vacuo* to give crude monochlate. To a solution of above crude monochlate in DMF (7.8 mL) was added sodium azide (0.51 mg, 7.778 mmol) at room temperature, then the mixture was warmed to 60 °C and stirred for 4.5 h. The reaction mixture was quenched with H₂O. The aqueous layer was extracted with ethyl ether three times. The combined organic extracts were washed with brine, dried over MgSO₄, and concentrated *in vacuo.* The residue was chromatographed on silica gel (Hexane/ethyl acetate; 50:1) to give azide **63** (0.35 g, 58% from **62).**

### Reference Example 40

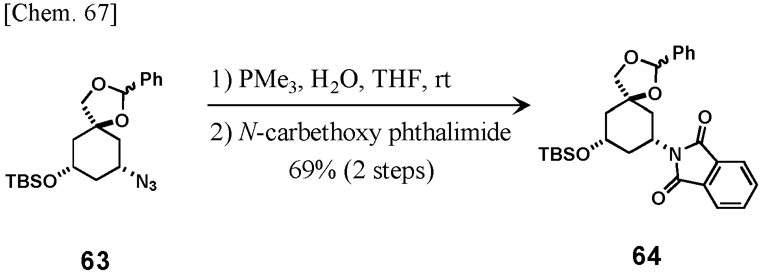

A solution of trimethylphosphine (1.40 mL, 1.355 mmol, 1.0 M in toluene) and distilled water (0.23 mL) was added to a solution of azide **63** (0.35 g, 0.903 mmol) in THF (9.0 mL) at room temperature. After stirring for 21 h, the mixture was concentrated at 50 °C to give crude amine. To a solution of above crude amine in THF (9.0 mL) was added N-carbethoxy phthalimide (0.50 mg, 2.258 mmol) at room temperature. After stirring for 22 h, to the mixture was added sat. Na₂CO₃ aq. (1 mL) and stirred vigorously. After additive 10 min, the aqueous layer was extracted with ethyl ether three times. The combined organic extracts were washed with brine, dried over MgSO₄, and concentrated *in vacuo.* The residue was chromatographed on silica gel (Hexane/ethyl acetate; 10:1 to 6:1) to give azide **64** (0.31 g, 69% from **63).**

### Reference Example 41

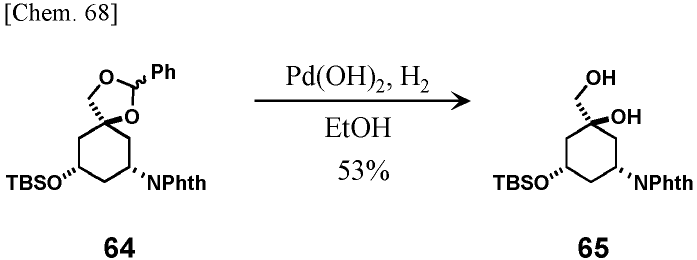

A solution of phthalimide **64** (334.0 mg, 1.355 mmol) in ethanol (6.8 mL) was added palladium hydroxide (34 mg, 10 wt%, 20% on Carbon wetted with ca. 50% Water from TCI Co., Ltd.) at room temperature. The reaction vessel was purged and placed under hydrogen and stirred vigorously for 15 h. The reaction mixture was filterd through a pad of celite and filtrate was concentrated. The residue was chromatographed on silica gel (Hexane/ethyl acetate; 10:1 to 1:1) to give diol **65** (177.8 mg, 53%) and starting material (92.6 mg, 34% recovery).

### Reference Example 42

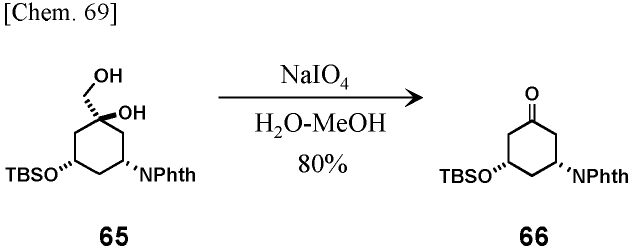

To a solution of **65** (155.2 mg, 0.383 mmol) in methanol (16.7 mL) was added dropwise a solution of sodium periodate (368 mg, 1.722 mmol) in distilled water (2.4 mL) at 0 °C. After stirring for 17 h, the reaction mixture was diluted with water and the aqueous layer was extracted with dichloromethane three times. The combined organic extracts were washed with brine, dried over MgSO₄, and concentrated *in vacuo.* The residue was chromatographed on silica gel (Hexane/ethyl acetate; 15:1 to 1:1) to give ketone **66** (114.7 mg, 80%) and starting material **65** (18.2 mg, 12% recovery).
**¹H NMR** (300 MHz, CDCl₃) δ 7.88-7.82 (m, 2H), 7.77-7.71 (m, 2H), 4.43 (dddd, *J=* 13.07, 12.73, 4.47, 4.13 Hz, 1H), 3.94 (dddd, *J* = 11.01, 10.66, 6.19, 4.47 Hz, 1H), 3.35 (dd, *J* = 14.10, 13.07 Hz, 1H), 2.75-2.63 (m, 2H), 2.58-2.43 (m, 2H), 2.19-2.10 (m, 1H), 0.87 (s, 9H), 0.07 (s, 3H), 0.06 (s, 3H).

### Reference Example 43

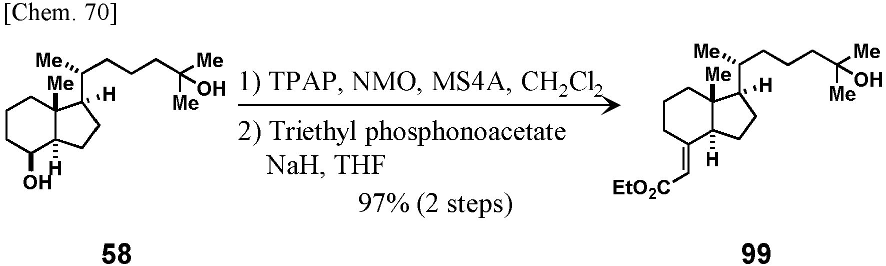

A solution of diol **58** (0.91 g, 3.211 mmol) in dry dichloromethane (11 mL) was added molecular sieves 4A (1.61 g) and *N-*methyl morpholine A-oxide (0.75 g, 6.422 mmol) and stirred for 30 min. Tetraisopropylammonium perruthenate (45 mg, 0.128 mmol) was added, then the reaction mixture was warmed to room temperature. After stirring for 2 h, the mixture was filtered through a pad of silica gel, and the filtrate was evaporated to give crude ketone. A suspension of sodium hydride (0.77 g, 19.266 mmol, 60% stabilized by oil) in THF (20 mL) was added triethyl phosphonoacetate (5.8 mL, 28.900 mmol) at 0 °C and warmed to room temperature. To the solution was added a solution of above ketone (3.211 mmol) in THF (12 mL). After stirring for 3 days, the reaction was quenched with H₂O and aqueous layer was extracted with ethyl acetate three times. The combined organic extracts were washed with brine, dried over MgSO₄, and concentrated *in vacuo.* The residue was chromatographed on silica gel (Hexane/ethyl acetate; 15:1 to 10:1) to give α,β-unsaturated ester **99** (1.09 g, 97%) as a colorless oil.
**¹H NMR** (300 MHz, CDCl₃) δ 5.45 (s, 1H), 4.14 (q, *J* = 7.22 Hz, 2H), 3.87-3.82 (m, 1H), 2.13-1.20(m, 18H), 1.28 (t, *J=* 7.22 Hz, 3H), 1.21 (s, 6H), 0.94 (d, *J=* 6.19 Hz, 3H), 0.57 (s, 3H).

### Reference Example 44

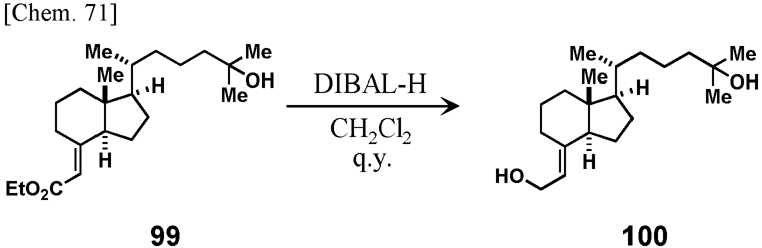

A solution of α,β-unsaturated ester **99** (0.80 g, 2.278 mmol) in CH₂Cl₂ (23 mL) was added diisobutyl aluminum hydride in hexane (6.8 mL, 6.835 mmol, 1.0 M). After stirring for 2 h, the reaction was quenched by careful addition of MeOH (5 mL). The mixture was added sat. Rochelle salt aq. (8 mL) and stirred for 30 min. The organic layer was washed with sat. Rochelle salt aq. three times, and the aqueous layer was extracted with CHCl₃ three times. The combined mixture was dried over MgSO₄ and concentrated *in vacuo.* The residue was chromatographed on silica gel (Hexane/ethyl acetate; 5:1 to 1:1) to give allyl alcohol **100** (0.77 g, q.y.) as a colorless oil.
**¹H NMR** (300 MHz, CDCl₃) δ 5.21 (dd, *J=* 7.22, 6.88 Hz, 1H), 4.20 (dd, *J=* 6.19, 5.85 Hz, 2H), 2.64-2.59 (m, 1H), 2.01-1.20(m, 18H), 1.21 (s, 6H), 0.93 (d, *J* = 6.19 Hz, 3H), 0.54 (s, 3H).

### Reference Example 45

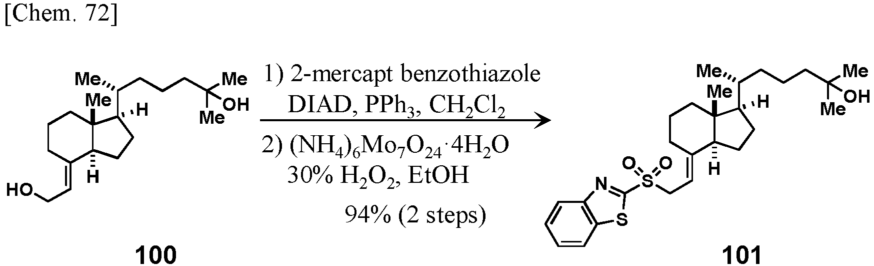

Triphenylphosphine (104.0 mg, 0.395 mmol), 2-mercapt benzothiazole (66 mg, 0.395 mmol) were added to a solution of allyl alcohol **100** (75.5 mg, 0.247 mmol) in dichloromethane (0.80 mL) at 0 °C, then added dropwise diisopropyl azodicarboxylate (77 µL, 0.395 mmol). After stirring for 1 h, the reaction mixture was quenched with H₂O. The aqueous layer was extracted with dichloromethane three times. The combined organic extracts were dried over MgSO₄, and concentrated *in vacuo.* The residue was chromatographed on silica gel (hexane/ethyl acetate; 10:1 to 5:1) to thioether (0.247 mmol) including residual reagent. To a solution of thioether (0.247 mmol) in ethanol (3.3 mL) was added hydrogen peroxide (1.3 mL, 30% solution in water) and hexaammonium heptamolybdate tetrahydrate (97 mg, 0.079 mmol) at room temperature. After stirring for 3 h, the reaction was quenched with 10% Na₂S₂O₃ aq. The aqueous layer was extracted with ethyl acetate three times. The combined organic extracts were washed with brine, dried over MgSO₄, and concentrated *in vacuo.* The residue was chromatographed on silica gel (hexane/ethyl acetate; 5:1 to 3:1) to give alcohol **101** (0.18 g, 94%).

### Reference Example 46

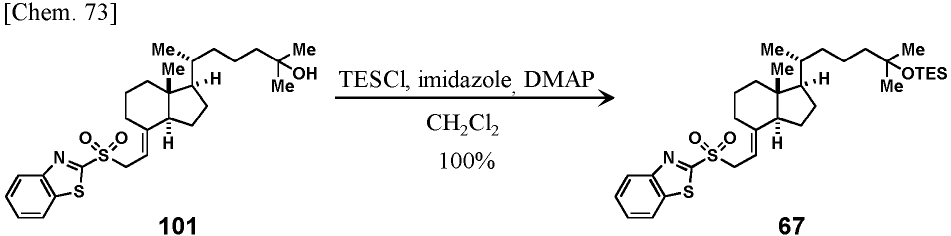

To a solution of alcohol **101** (1.04 g, 2.127 mmol) in *N*,*N-*dimethylformamide (7 mL) was added imidazole (0.43 g, 6.382 mmol) and *N*,*N*-dimethylamino-4-pyridine (52 mg, 0.425 mmol) at room temperature, then added dropwise chloro triethylsilane (0.89 mL, 5.318 mmol). After stirring for 6 h, the reaction mixture was quenched with H₂O. The aqueous layer was extracted with ethyl ether three times. The combined organic extracts were washed with H₂O and brine, dried over MgSO₄, and concentrated *in vacuo.* The residue was chromatographed on silica gel (hexane/ethyl acetate; 40:1 to 20:1) to give benzothiazolyl sulfone **67** (1.28 g, 100%).
**¹H NMR** (300 MHz, CDCl₃) δ 8.22-7.56 (m, 4H), 5.01 (dd, *J=* 7.79, 7.79 Hz, 1H), 4.43 (dd, *J* = 14.20, 8.70 Hz, 1H), 4.20 (dd, *J=* 14.20, 6.87 Hz, 1H), 2.56-2.53 (m, 1H), 1.90-1.81 (m,
3H), 1.62-1.15(m, 15H), 1.17 (s, 6H), 0.93 (t, *J=* 7.79 Hz, 9H), 0.84 (d, *J=* 6.41 Hz, 3H), 0.55 (q, *J=* 7.79 Hz, 6H), 0.25 (s, 3H).

### Reference Example 47

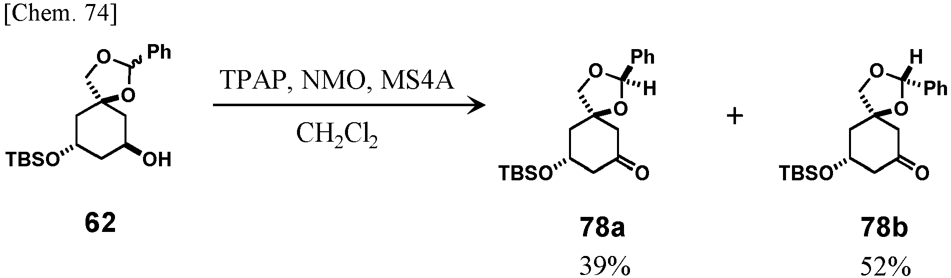

A solution of alcohol **62** (1.22 g, 3.353 mmol) in CH₂Cl₂ (190 mL) was added molecular sieves 4A and *N*-methylmorpholine *N*-oxide at room temperature. After stirring for 15 min, tetrapropylammonium perruthenate was added at 0 °C. After additive 1 h, the reaction mixture was filtered through a pad of silica gel and filtrate was concentrated. The residue was chromatographed on silica gel (Hexane/dichloromethane; 15:1 to 5:1) to give ketone **78a** (0.48 g, 39%) and **78b** (0.63 g, 52%).

### Reference Example 48

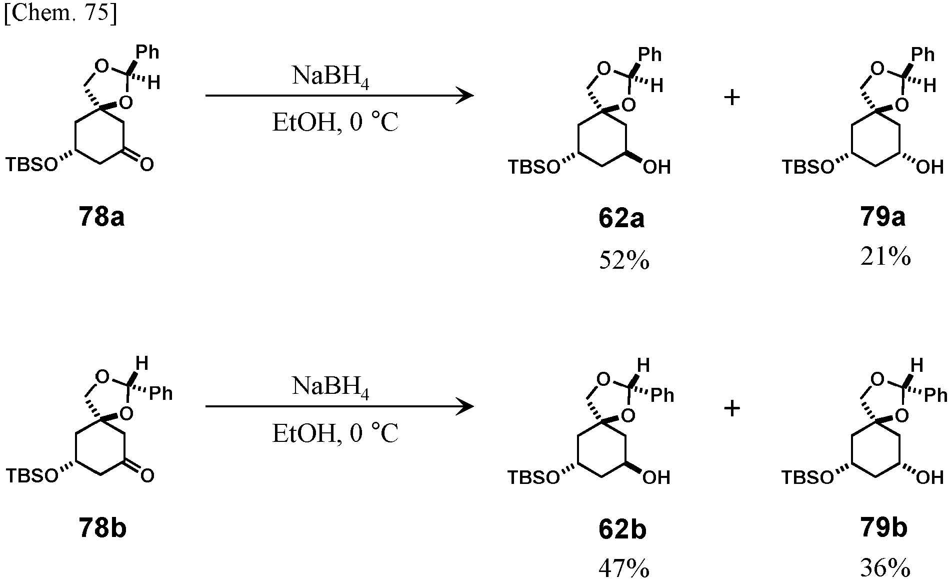

A solution of ketone **78a** (130.5 mg, 0.360 mmol) in ethanol (3.6 mL) was added sodium borohydride (27.2 mg, 0.720 mmol). After stirring for 3.5 h, the reaction mixture was quenched with H₂O and brine, then aqueous layer was extracted with ethyl acetate three times. The combined organic extracts were washed with brine, dried over MgSO₄, and concentrated *in vacuo.* The residue was chromatographed on silica gel (hexane/ethyl acetate; 15:1 to 5:1) to give cis-alcohol **79a** (16.5 mg, 21%) and trans-alcohol **62a** (41.8 mg, 52%) as a colorless oil.

By following the same procedure described for **79a,** cis-alcohol **79b** (47.8 mg, 36%) and trans-alcohol **62b** (61.5 mg, 47%) was obtained from **78b** (79.5 mg, 0.219 mmol) as a colorless oil.

### Reference Example 49

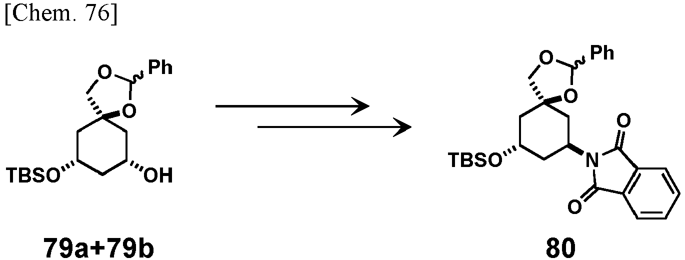

By following the same procedure described for **64,** phthalimide **80** (0.70 g, 62%) was obtained from **79a** and **79b** (0.84 g, 2.291 mmol) as an amorphous solid like foam.

### Reference Example 50

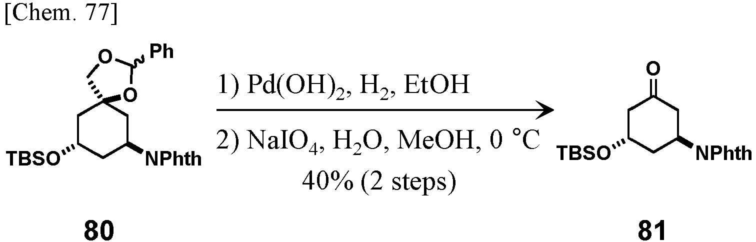

A solution of phthalimide **80** (46.4 mg, 0.094 mmol) in ethanol (1.0 mL) was added palladium hydroxide (5 mg, 10 wt%, 20% on Carbon wetted with ca. 50% Water from TCI Co., Ltd.) at room temperature. The reaction vessel was purged and placed under hydrogen and stirred vigorously for 5.5 h. The reaction mixture was filterd through a pad of celite and filtrate was concentrated. The residue was chromatographed on silica gel (Hexane/ethyl acetate; 10:1 to 1:1) to give diol (19.2 mg, 50%) and starting material (12.7 mg, 27% recovery). To a solution of above diol (19.2 mg, 0.047 mmol) in methanol (2.1 mL) was added dropwise a solution of sodium periodate (46 mg, 0.213 mmol) in distilled water (0.3 mL) at 0 °C. After stirring for 7 h, the reaction mixture was diluted with water and the aqueous layer was extracted with dichloromethane three times. The combined organic extracts were washed with brine, dried over MgSO₄, and concentrated *in vacuo.* The residue was chromatographed on silica gel (Hexane/ethyl acetate; 15:1 to 2:1) to give ketone **81** (13.9 mg, 79%) as a colorless solid.
**¹H NMR** (300 MHz, CDCl₃) δ 7.87-7.70 (m, 4H), 5.05 (dddd, *J* = 12.38, 12.04, 4.47, 4.47 Hz, 1H), 4.52-4.46 (m, 1H), 3.31 (dd, *J=* 13.76, 12.73 Hz, 1H), 2.75-2.47 (m, 3H), 2.02-1.94 (m, 1H), 0.90 (s, 9H), 0.08 (s, 6H).

### Reference Example 51

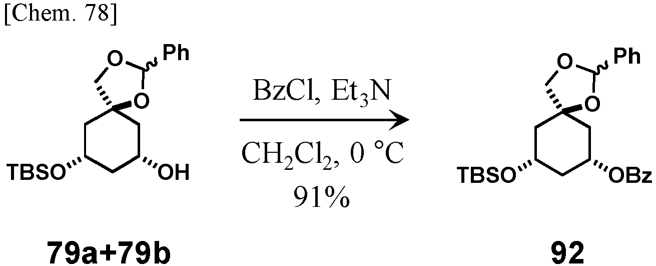

A solution of alcohol **79a** and **79b** (0.82 g, 2.2436 mmol) in CH₂Cl₂ (22.0 mL) was added triethylamine (0.94 mL, 6.735 mmol), *N,N-*dimethyl-4-aminopyridine (60.5 mg, 0.495 mmol) and benzoyl chloride (0.52 mL, 4.513 mmol) at 0 °C, then the mixture was warmed to room temperature and stirred for 2 h. The reaction was quenched with sat. NaHCO₃ aq. The aqueous layer was extracted with CH₂Cl₂ three times. The combined organic extracts were dried over MgSO₄, and concentrated *in vacuo.* The residue was chromatographed on silica gel (Hexane/ethyl acetate; 20:1) to give **92** (0.96 mg, 91%) as a colorless solid.

### Reference Example 52

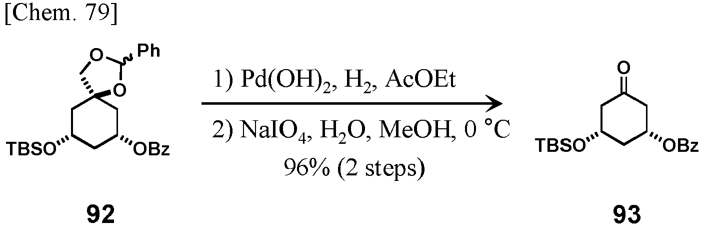

A solution of benzoate ester **92** (0.96 g, 2.047 mmol) in ethyl acetate (41 mL) was added palladium hydroxide (97.8 mg, 10 wt%, 20% on Carbon wetted with ca. 50% Water from TCI Co., Ltd.) at room temperature. The reaction vessel was purged and placed under hydrogen and stirred vigorously for 2 h. The reaction mixture was filterd through a pad of celite and filtrate was concentrated. The residue was chromatographed on silica gel (Hexane/ethyl acetate; 5:1 to 2:1) to give diol (0.77 g, 99%) as a colorless solid. To a solution of above diol (0.77 g, 2.017 mmol) in methanol (84 mL) was added dropwise a solution of sodium periodate (2.00 g, 9.335 mmol) in distilled water (12 mL) at 0 °C. After stirring for 1.5 h, the reaction mixture was diluted with water and the aqueous layer was extracted with dichloromethane three times. The combined organic extracts were washed with brine, dried over MgSO₄, and concentrated *in vacuo.* The residue was chromatographed on silica gel (Hexane/ethyl acetate; 10:1 to 5:1) to give ketone **93** (0.68 g, 97%) as a colorless solid.

### Example 1 (Comparative Example)

### NHBoc VD₃ 18a and 18b

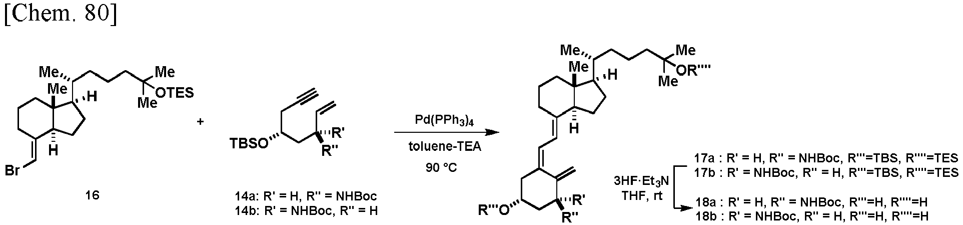

To a solution of **16** (109 mg, 0.231 mmol) and **14a** (36 mg, 0.101 mmol) in toluene (1.0 mL) and Et₃N (1.0 mL) was added Pd(PPh₃)₄ (10-20 mol%) at room temperature, then the reaction mixture was heated at 90 °C. After stirring for 3.5 h, the reaction mixture was filtered through a pad of silica gel column and the filtrates were evaporated. The residue was chromatographed on silica gel (hexane/ethyl acetate; 100:1) to give **17a** (19 mg, 0.025 mmol, not pure). To a solution of **17a** (19 mg, 0.025 mmol) in THF (1 mL) was added 3HF-Et₃N (0.050 mL, 0.299 mmol) at room temperature. The reaction was monitored by thin layer chromatography on silica gel plates (eluent: 1:1 ethyl acetate to hexane). The reaction was quenched by sat. NaHCO₃, the aqueous layer was extracted with ethyl acetate three times. The combined organic extracts were washed with brine, dried over MgSO₄, and concentrated *in vacuo.* The residue was chromatographed on silica gel (hexane/ethyl acetate; 5:2 to 2:1) to give 1α-NHBoc VD₃ **18a** (7.2 mg, 2 steps 14%).
¹H NMR (300 MHz, CDCl₃) δ 6.35 (d, *J* = 11.3 Hz, 1H), 5.99 (d, *J* = 11.3 Hz, 1H), 5.22 (s, 1H), 4.95 (s, 1H), 4.51-4.08 (m, 3H), 2.81 (d, *J=* 12.5 Hz, 1H), 2.56 (d, *J=* 12.5 Hz, 1H), 2.35-0.85 (m, 39H), 1.45 (s, 9H), 1.21 (s, 6H), 0.93 (d, *J=* 6.2 Hz, 3H), 0.53 (s, 3H);
HRMS (ESI) Calcd for C₃₂H₅₃N₁Na₁O₄ [M+Na]⁺: 538.3872, Found: 538.3882.

By following the same procedure described for **18a,** 1β-NHBoc VD₃ **18b** (3.5 mg, 13%) was obtained from **16** (59 mg, 0.124 mmol) and **14b** (19 mg, 0.054 mmol) as a colorless oil.

### Example 2 (Comparative Example)

### 1-amino-25(OH)D 19a and 19b

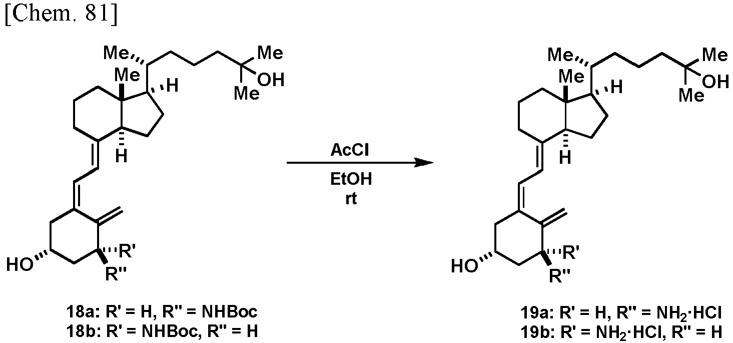

Acetyl chloride (0.080 mL, 1.13 mmol) was slowly added to EtOH (1.1 mL) dropwise at 0 °C. After stirring for 30 min, this solution was added to diol **18a** (15.9 mg, 0.0308 mmol). After additional stirring for 6 h, the reaction mixture was evaporated. The residue was washed with Et₂O, affording **19a** (13.01 mg, 93%) as a yellow solid.
¹H NMR (300 MHz, MeOH-*d*₄) δ 6.45 (d, *J* = 11.0 Hz, 1H), 6.04 (d, *J=* 11.0 Hz, 1H), 5.28 (s, 1H), 5.17 (s, 1H), 4.19-4.11 (m, 1H), 4.07-4.02 (m, 1H), 2.89 (d, *J* = 10.5 Hz, 1H), 2.57 (d,*J* = 10.5 Hz, 1H), 2.41-1.13 (m, 27H), 0.97 (d, *J* = 6.2 Hz, 3H), 0.58 (s, 3H);
HRMS (ESI) Calcd for C₂₇H₄₆N₁O₂ [M+H]⁺: 416.3529, Found: 416.3532.

By following the same procedure described for **19a, 19b** (2.82 mg, 92%) was obtained from **18b** (3.5 mg, 0.00678 mmol) as a yellow solid.
¹H NMR (300 MHz, MeOH-*d*₄) δ 6.50 (d, *J* = 11.0 Hz, 1H), 6.01 (d, *J=* 11.0 Hz, 1H), 5.38 (s, 1H), 5.19 (s, 1H), 4.11-4.01 (m, 1H), 3.92-3.86 (m, 1H), 2.89 (d, *J* = 10.5 Hz, 1H), 2.57 (d,*J* = 10.5 Hz, 1H), 2.42-1.14 (m, 27H), 0.97 (d, *J=* 6.2 Hz, 3H), 0.57 (s, 3H).

### Example 3

### NHAc VD₃ 22a and 22b

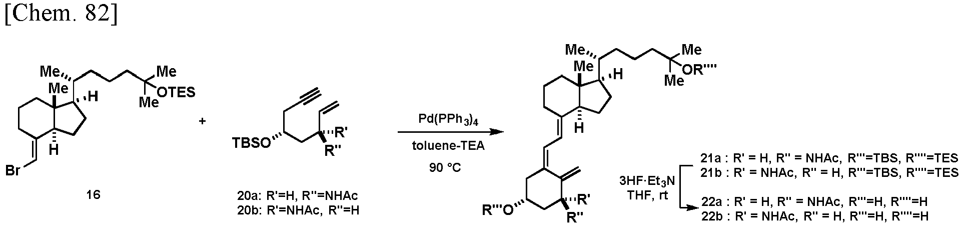

To a solution of **16** (106 mg, 0.225 mmol) and **20a** (40.6 mg, 0.137 mmol) in toluene (1.4 mL) and Et₃N (1.4 mL) was added Pd(PPh₃)₄ (10 - 20 mol%) at room temperature, then the reaction mixture was heated at 90 °C. After stirring for 5 h, the reaction mixture was filtered through a pad of silica gel column and the filtrates were evaporated. The residue was chromatographed on silica gel (hexane/ethyl acetate; 6:1 to 3:1) to give **21a** (9.7 mg, 10%). To a solution of **21a** (9.7 mg, 0.014 mmol) in THF (0.3 mL) was added 3HF-Et₃N (0.030 mL, 0.18 mmol) at room temperature. The reaction was monitored by thin layer chromatography on silica gel plates (eluent: 3:2:1 chloroform to ethyl acetate to methanol). The reaction mixture was quenched with sat. NaHCO₃ aq. The aqueous layer was extracted with ethyl acetate three times. The combined organic extracts were washed with brine, dried over MgSO₄, and concentrated *in vacuo.* The residue was chromatographed on silica gel (hexane/ethyl acetate; 1:10 to 0:1) to give 1α-NHAc VD₃ **22a** (5.87 mg, 90%).
¹H NMR (400 MHz, CDCl₃) δ 6.38 (d, *J* = 11.0 Hz, 1H), 5.97 (d, *J=* 11.0 Hz, 1H), 5.37 (d,*J* = 8.7 Hz, 1H), 5.20 (s, 1H), 4.96 (s, 1H), 4.77 (dd, *J* = 13.7, 6.0 Hz, 1H), 4.10-4.05 (m, 1H), 2.81 (d, *J* = 12.4 Hz, 1H), 2.57 (d, *J=* 12.4 Hz, 1H), 2.35-0.83 (m, 32H), 1.99 (s, 3H), 1.21 (s, 6H), 0.93 (d, *J=* 6.0 Hz, 3H), 0.52 (s, 3H);
HRMS (ESI) Calcd for C₂₉H₄₇N₁Na₁O₃ [M+Na]⁺: 480.3454, Found: 480.3458.

By following the same procedure described for **22a,** 1β-NHAc VD₃ **22b** (10.6 mg, 79%) was obtained from **16** (104 mg, 0.221 mmol) and **20b** (37.2 mg, 0.126 mmol) as colorless oil.
¹H NMR (400 MHz, CDCl₃) δ 6.46 (d, *J* = 9.2 Hz, 1H), 6.36 (d, *J* = 11.0 Hz, 1H), 5.95 (d, *J*= 11.0 Hz, 1H), 5.27 (s, 1H), 4.95 (s,1H), 4.70-4.65 (m, 1H), 4.18-4.13 (m, 1H), 2.81 (d, *J* = 12.0 Hz, 1H), 2.59 (d, *J* = 12.0 Hz, 1H), 2.36-0.81 (m, 32H), 1.94 (s, 3H), 1.21 (s, 6H), 0.92 (d, J = 6.4 Hz, 3H), 0.52 (s, 3H).

### Example 4

### 1α-NHNs VD₃ 28

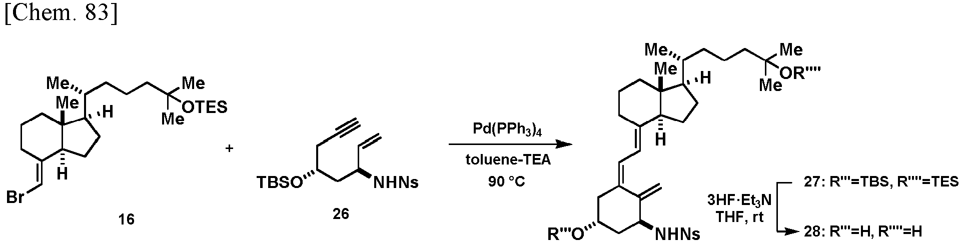

To a solution of **16** (64.0 mg, 0.136 mmol) and **26** (39.3 mg, 0.090 mmol) in toluene (0.9 mL) and Et₃N (0.9 mL) was added Pd(PPh₃)₄ (10-20 mol%) at room temperature, then the reaction mixture was heated at 90 °C. After stirring for 5 h, the reaction mixture was filtered through a pad of silica gel column and the filtrates were evaporated. The residue was chromatographed on silica gel (hexane/ethyl acetate; 10:1) to give **27** (12.4 mg, 47%). To a solution of **27** (12.4 mg, 0.015 mmol) in THF (0.3 mL) was added 3HF-Et₃N (0.31 mL, 1.92 mmol) at room temperature. The reaction was monitored by thin layer chromatography on silica gel plates (eluent: 1:1 ethyl acetate to hexane). The reaction was quenched by sat. aq. NaHCO₃. The aqueous layer was extracted with ethyl acetate three times. The combined organic extracts were washed with brine, dried over MgSO₄, and concentrated *in vacuo.* The residue was chromatographed on silica gel (hexane/ethyl acetate; 3:1 to 1:2) to give 1α-NHNs VD₃ **28** (6.51 mg, 90%).
¹H NMR (300 MHz, CDCl₃) δ 8.20-7.24 (m, 4H), 6.36 (d, *J* = 11.3 Hz, 1H), 5.74 (d, *J* = 11.3 Hz, 1H), 5.41 (d, *J* = 8.2 Hz, 1H), 5.04 (s, 1H), 4.82 (s, 1H), 4.37-4.09 (m, 2H), 2.78 (d, *J =* 11.9 Hz, 1H), 2.55 (d, *J* = 11.9 Hz, 1H), 2.31-0.80 (m, 30H), 1.23 (s, 6H), 0.93 (d, *J* = 6.5 Hz, 3H), 0.51 (s, 3H);
HRMS (ESI) Calcd for C₃₃H₄₈N₂Na₁O₆S₁ [M+Na]⁺: 623.3131, Found: 623.3161.

### Example 5

### 1α-NHTs VD₃ 32

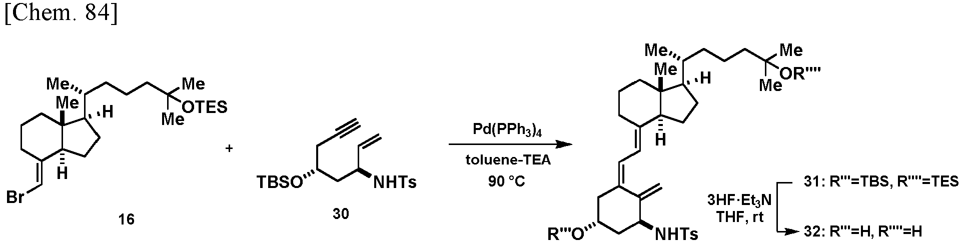

To a solution of **16** (47.0 mg, 0.099 mmol) and **30** (26.8 mg, 0.066 mmol) in toluene (0.65 mL) and Et₃N (0.65 mL) was added Pd(PPh₃)₄ (10-20 mol%) at room temperature, then the reaction mixture was heated at 90 °C. After stirring for 6 h, the reaction mixture was filtered through a pad of silica gel column and the filtrate was evaporated. The residue was chromatographed on silica gel (hexane/ethyl acetate; 10:1) to give **31** (5.1 mg, 10%). To a solution of **31** (5.1 mg, 0.00639 mmol) in THF (0.13 mL) was added 3HF-Et₃N (0.091 mL, 0.556 mmol) at room temperature. The reaction was monitored by thin layer chromatography on silica gel plates (eluent: 1:1 ethyl acetate to hexane). The reaction mixture was quenched with sat. NaHCO₃ aq. The aqueous layer was extracted with ethyl acetate three times. The combined organic extracts were washed with brine, dried over MgSO₄, and concentrated *in vacuo.* The residue was chromatographed on silica gel (hexane/ethyl acetate; 2:1 to 1:1) to give 1**α**-NHTs VD₃ **32** (2.20 mg, 60%).
¹H NMR (300 MHz, CDCl₃) δ 7.73 (d, *J* = 8.2 Hz, 2H), 7.29 (d, *J* = 8.2 Hz, 2H), 6.33 (d, *J* = 11.0 Hz, 1H), 5.80 (d, *J=* 11.0 Hz, 1H), 4.93 (s, 1H), 4.64 (s, 1H), 4.37 (d, *J =* 7.2 Hz, 1H), 4.16-4.01 (m, 2H), 2.79-2.73 (m, 2H), 2.43 (s, 3H), 2.24-0.80 (m, 30H), 1.22 (s, 6H), 0.94 (d, *J=* 6.2 Hz, 3H), 0.54 (s, 3H);
HRMS (FAB) Calcd for C₃₄H₅₁NNaO₄S [M+Na]⁺: 592.3437, Found: 592.3433.

### Example 6

### 1α-NHCbz VD₃ 36

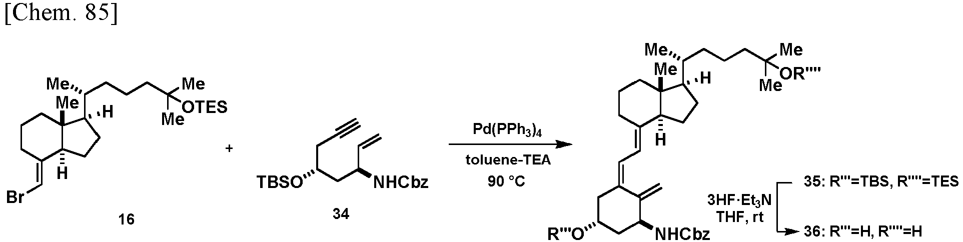

To a solution of **16** (45.7 mg, 0.097 mmol) and **34** (21.4 mg, 0.055 mmol) in toluene (0.55 mL) and Et₃N (0.55 mL) was added Pd(PPh₃)₄ (10-20 mol%) at room temperature, then the reaction mixture was heated at 90 °C. After stirring for 6 h, the reaction mixture was filtered through a pad of silica gel column and the filtrates were evaporated. The residue was chromatographed on silica gel (hexane/ethyl acetate; 75:1 to 40:1) to give **35** (5.6 mg, 13%). To a solution of **35** (5.6 mg, 0.0072 mmol) in THF (0.145 mL) was added 3HF-Et₃N (0.13 mL, 0.818 mmol) at room temperature. The reaction was monitored by thin layer chromatography on silica gel plates (eluent: 1:1 ethyl acetate to hexane). The reaction mixture was quenched with sat. NaHCO₃ aq. The aqueous layer was extracted with ethyl acetate three times. The combined organic extracts were washed with brine, dried over MgSO₄, and concentrated *in vacuo.* The residue was chromatographed on silica gel (Hexane/ethyl acetate; 2:1 to 1:1) to give 1**α**-NHCbz VD₃ **36** (1.16 mg, 29%).
¹H NMR (300 MHz, CDCl₃) δ 7.36 (s, 5H), 6.36 (d, *J =* 11.0 Hz, 1H), 5.97 (d, *J =* 11.0 Hz, 1H), 5.23 (s, 1H), 5.12-5.09 (m, 2H), 4.97 (s, 1H), 4.57-4.46 (m, 1H), 4.12-4.06 (m, 1H), 2.84-0.81 (m, 32H), 1.22 (s, 6H), 0.93 (d, *J=* 6.2 Hz, 3H), 0.51 (s, 3H).
HRMS (FAB) Calcd for C₃₅H₅₁NNaO₄ [M+Na]⁺: 572.3716, Found: 572.3717.

### Example 7 (Comparative Example)

### Amine 37

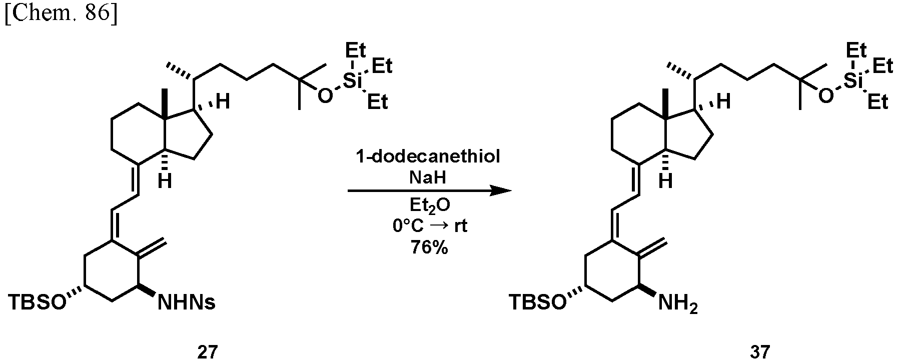

A solution of 1-dodecanethiol (0.0040 mL, 0.01411 mmol) in diethyl ether (0.10 mL) was added NaH (1.00 mg, 0.01881 mmol, 60% stabilized in mineral oil) at 0 °C and stirred for 30min. To the suspension was added nosylate **27** (7.8 mg, 0.00941 mmol) in diethyl ether (0.10 mL), and then the reaction mixture was warmed to room temperature. After additive 2 h, the reaction was quenched by H₂O. The aqueous layer was extracted with ethyl acetate three times. The combined organic extracts were washed with brine, dried over MgSO₄, and concentrated *in vacuo.* The residue was chromatographed on silica gel (hexane/ethyl acetate; 4:1 to 1:1) to give amine **37** (4.6 mg, 76%).
**¹H NMR** (300 MHz, CDCl₃) δ 6.27 (d, *J* = 11.34 Hz, 1H), 5.98 (d, *J=* 11.34 Hz, 1H), 5.17 (s, 1H), 4.91 (s, 1H), 4.16-4.08 (m, 1H), 3.76-3.69 (m, 1H), 2.85-2.77 (m, 1H), 2.49-0.80 (m, 25H), 1.18 (s, 6H), 0.94 (t, *J=* 7.89 Hz, 9H), 0.86 (s, 9H), 0.56 (q, *J* = 7.89 Hz, 6H), 0.54 (s, 3H), 0.59 (s, 6H).

### Example 8

### 1α-NH(p-CF₃Bz) VD₃ 38

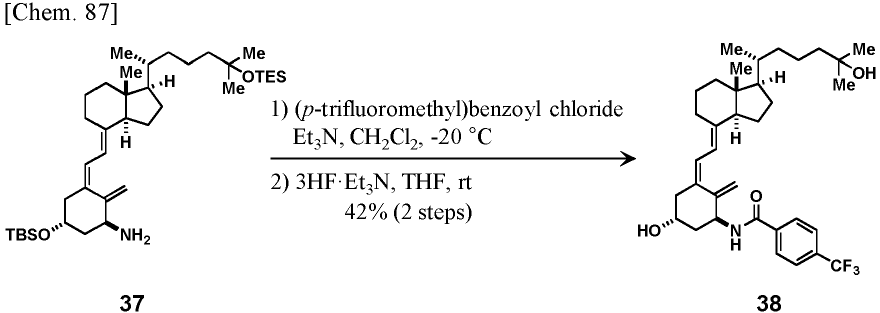

A solution of amine **37** (5.2 mg, 0.00807 mmol) in CH₂Cl₂ (0.20 mL) was added Et₃N (0.0028 mL, 0.02018 mmol), and carefully dropwised *p*-(trifluoromethyl)benzoyl chloride (0.0014 mL, 0.00969 mmol) at - 20 °C. After stirring for 1.5 h, the reaction was quenched by sat. NaHCO₃ aq. The aqueous layer was extracted with ethyl acetate three times. The combined organic extracts were washed with brine, dried over MgSO₄, and concentrated *in vacuo.* The residue was chromatographed on silica gel (hexane/ethyl acetate; 30:1 to 15:1) to give benzamide (6.0 mg, 91%, not pure). To a solution of the benzamide (6.0 mg, 7.35 µmol) in THF (0.20 mL) was added 3HF-Et₃N (0.14 mL, 0.878 mmol) at room temperature. The reaction was monitored by thin layer chromatography on silica gel plates (eluent: 2:1 ethyl acetate to hexane). The reaction mixture was quenched with sat. aq. NaHCO₃. The aqueous layer was extracted with ethyl acetate three times. The combined organic extracts were washed with brine, dried over MgSO₄, and concentrated *in vacuo.* The residue was chromatographed on silica gel (hexane/ethyl acetate; 2:1 to 1:a1) to give 1α-NH(*p*-CF₃Bz) VD₃ **38** (2.0 mg, 46%).
¹H NMR (400 MHz, CDCl₃) δ 7.83 (d, *J* = 8.2 Hz, 2H), 7.67 (d, *J* = 8.2 Hz, 2H), 6.45 (d, *J* = 11.4 Hz, 1H), 6.03 (d, *J* = 8.2 Hz, 1H), 5.99 (d, *J* = 11.4 Hz, 1H), 5.31 (s, 1H), 5.03 (s, 1H), 5.00-4.95 (m, 1H), 4.12-4.06 (m, 1H), 2.84 (d, *J =* 12.8 Hz, 1H), 2.65 (d, *J* = 12.8 Hz, 1H), 2.39-0.81 (m, 30H), 1.21 (s, 6H), 0.93 (d, *J =* 6.4 Hz, 3H), 0.46 (s, 3H);
HRMS (ESI) Calcd for C₃₅H₄₈F₃N₁Na₁O₃ [M+Na]⁺: 610.3484, Found: 610.3458.

### Example 9

### 1α-NH(p-BrBz) VD₃ 39

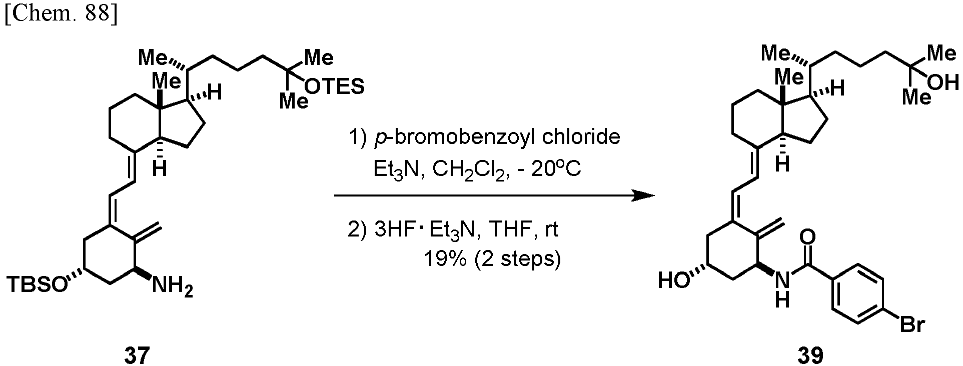

By following the similar procedure described for **38,** 1α-NH(*p*-CF₃Bz) VD₃ **39** (1.77 mg, 19%) purified by flash chromatography (Hexane/EtOAc; 1:3 to 1:10) was obtained from **37** (7.3 mg, 11.33 µmol) as a solid.
¹H NMR (400 MHz, CDCl₃) δ 7.59-7.52 (m, 4H), 6.45 (d, *J* = 11.0 Hz, 1H), 5.99 (d, *J* = 15.6 Hz, 1H), 5.96 (d, *J* = 11.0 Hz, 1H), 5.29 (s, 1H), 5.01 (s, 1H), 4.98-4.92 (m, 1H), 4.10-4.02 (m, 1H), 2.83 (d, *J=* 12.4 Hz, 1H), 2.64 (d, *J* = 12.4 Hz, 1H), 2.38-0.83 (m, 30H), 1.21 (s, 6H), 0.94 (d, *J=* 5.9 Hz, 3H), 0.49 (s, 3H);
HRMS (ESI) Calcd for C₃₄H₄₈Br₁N₁Na₁O₃ [M+Na]⁺: 622.2695, Found: 622.2683.

### Example 10

### 1α-NH(p-OMeBz) VD₃ 40

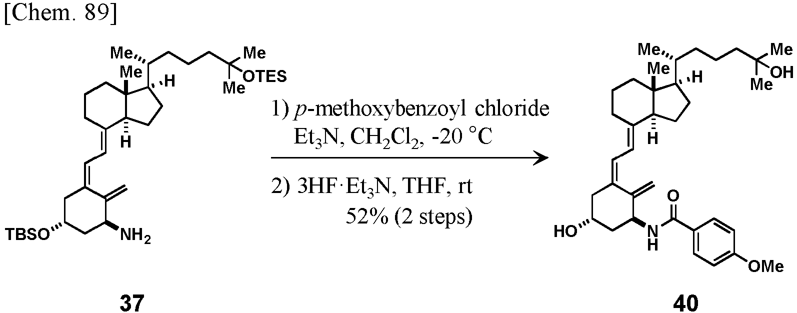

By following the similar procedure described for **38,** 1α-NH(*p*-OMeBz) VD₃ **40** (2.46 mg, 52%) purified by flash chromatography (hexane/ethyl acetate; 1:3 to 1:10) was obtained from **37** (5.6 mg, 8.69 µmol) as a solid.
**¹H NMR** (400 MHz, CDCl₃) δ 7.68 (d, *J=* 8.72 Hz, 2H), 6.89 (d, *J=* 8.72 Hz, 2H), 6.44 (d, *J* = 11.44 Hz, 1H), 6.01 (d, *J* = 11.44 Hz, 1H), 5.93 (d, *J* = 8.28 Hz, 1H), 5.30 (s, 1H), 5.00 (s, 1H), 4.97-4.92 (m, 1H), 4.13-4.04 (m, 1H), 3.85 (s, 3H), 2.86-0.83 (m, 22H), 2.35 (dd, *J=* 12.80, 7.80 Hz, 1H) 1.20 (s, 6H), 0.93 (d, *J=* 6.4 Hz, 3H), 0.51 (s, 3H);
**HR-MS ESI** Calcd for C₃₅H₅₁N₁Na₁O₄ [M+Na]⁺ : 572.37158, Found: 572.36923.

### Example 11

### 1α-NH(p-SCF₃Bz) VD₃ 41

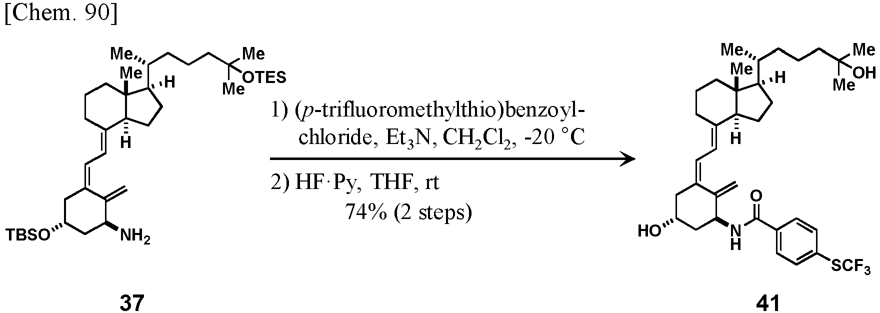

By following the similar procedure described for **38,** 1α-NH(*p*-SCF₃Bz) VD₃ **41** (118.88 mg, 74%) purified by flash chromatography (hexane/ethyl acetate; 3:1 to 1:1) was obtained from **37** (166.2 mg, 0.258 mmol) as a solid.
**¹H NMR** (300 MHz, CDCl₃) δ 7.75 (d, *J=* 8.25 Hz, 2H), 7.68 (d, *J=* 8.25 Hz, 2H), 6.45 (d, *J* = 10.98 Hz, 1H), 6.07 (d, *J=* 7.92 Hz, 1H), 5.99 (d, *J=* 10.98 Hz, 1H), 5.30 (s, 1H), 5.01 (s, 1H), 4.95 (dd, *J=* 11.67, 6.87 Hz, 1H), 4.12-4.04 (m, 1H), 2.86-1.15 (m, 23H), 1.21 (s, 6H), 0.93 (d, *J* = 5.82 Hz, 3H), 0.48 (s, 3H);
**HR-MS ESI** Calcd for C₃₅H₄₈F₃N₁Na₁O₃S₁ [M+Na]⁺ : 642.32047, Found: 642.31973.

### Example 12

### 1α-NH(2,3,4,5-tetrafluoroBz) VD₃ 42

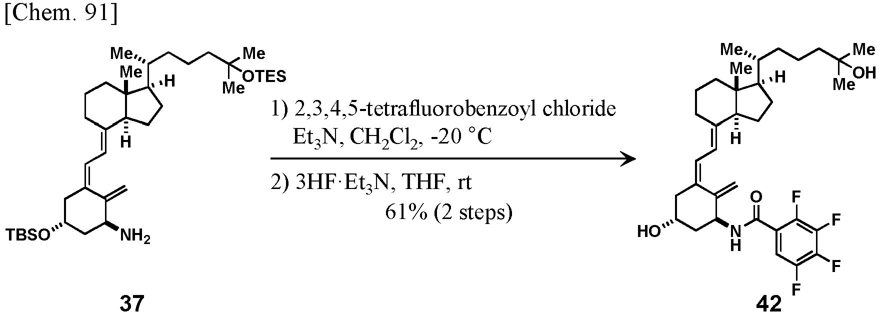

By following the similar procedure described for **38,** 1α-NH(2,3,4,5-tetrafluoroBz) VD₃ **42** (2.21 mg, 61%) purified by flash chromatography (NH silica gel, hexane/ethyl acetate; 1:1) was obtained from **37** (3.8 mg, 5.899 µmol) as a solid.
**¹H NMR** (400 MHz, CDCl₃) δ 7.80-7.72 (m, 1H), 6.52 (dd, *J=* 11.92, 7.32 Hz, 1H), 6.46 (d, *J* = 11.44 Hz, 1H), 5.97 (d, *J* = 11.44 Hz, 1H), 5.30 (s, 1H), 5.03 (s, 1H), 4.98-4.92 (m, 1H), 4.11-4.04 (m, 1H), 2.86-1.15 (m, 22H), 2.35 (dd, *J* = 13.28, 7.80 Hz, 1H), 1.21 (s, 6H), 0.93 (d, *J* = 5.52 Hz, 3H), 0.48 (s, 3H);
**HR-MS ESI** Calcd for C₃₄H₄₅F₄N₁Na₁O₃ [M+Na]⁺ : 614.32333, Found: 614.32730.

### Example 13

### 1α-NH(2,4,5-trifluoroBz) VD₃ 43

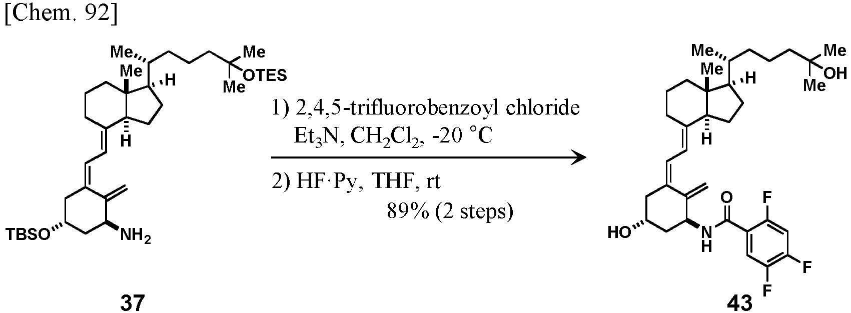

By following the similar procedure described for **38,** 1α-NH(2,4,5-trifluoroBz) VD₃ **43** (19.10 mg, 89%) purified by flash chromatography (NH silica gel, hexane/ethyl acetate; 1:1) was obtained from **37** (23.5 mg, 0.037 mmol) as a solid.
**¹H NMR** (400 MHz, CDCl₃) δ 8.00-7.93 (m, 1H), 6.98-6.92 (m, 1H), 6.62 (dd, *J=* 13.28, 8.24 Hz, 1H), 6.44 (d, *J* = 11.00 Hz, 1H), 5.97 (d, *J* = 11.00 Hz, 1H), 5.29 (s, 1H), 5.01 (s, 1H), 4.99-4.92 (m, 1H), 4.12-4.04 (m, 1H), 2.85-1.15 (m, 22H), 2.35 (dd, *J* = 12.84, 7.80 Hz, 1H), 1.21 (s, 6H), 0.93 (d, *J=* 6.44 Hz, 3H), 0.47 (s, 3H);
**HR-MS ESI** Calcd for C₃₄H₄₆F₃N₁Na₁O₃ [M+Na]⁺ : 596.33275, Found: 596.33404.

### Example 14

### 1α-NH(3,4-dimethoxyBz) VD₃ 44

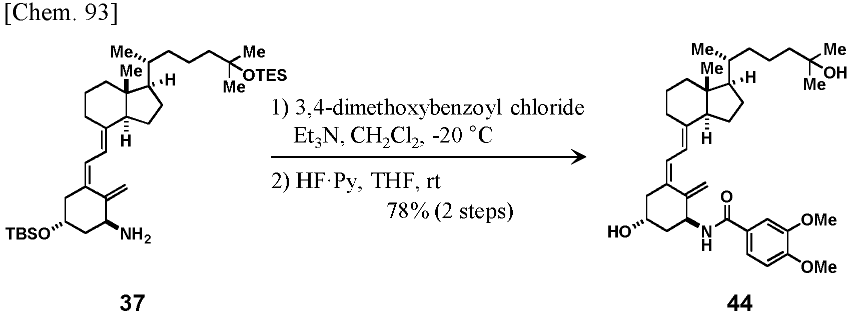

By following the similar procedure described for **38,** 1α-NH(3,4-dimethoxyBz) VD₃ **44** (1.76 mg, 78%) purified by flash chromatography (hexane/ethyl acetate; 1:3) was obtained from **37** (2.5 mg, 3.881 µmol) as a solid.
**¹H NMR** (300 MHz, CDCl₃) δ 7.46-7.44 (m, 1H), 7.17-7.13 (m, 1H), 6.80 (d, *J=* 8.58 Hz, 1H), 6.44 (d, *J=* 10.65 Hz, 1H), 6.01 (d, *J=* 10.65 Hz, 1H), 5.96 (d, *J=* 8.25 Hz, 1H), 5.30 (s, 1H), 5.01 (s, 1H), 5.00-4.92 (m, 1H), 4.16-4.05 (m, 1H), 3.94 (s, 3H), 3,91 (s,3H), 2.86-1.15 (m, 23H), 1.21 (s, 6H), 0.93 (d, *J=* 6.18 Hz, 3H), 0.50 (s, 3H);
**HR-MS** ESI Calcd for C₃₆H₅₃N₁Na₁O₅ [M+Na]⁺ : 602.38214, Found: 602.38563.

### Example 15

### 1α-NH(propionyl) VD₃ 45

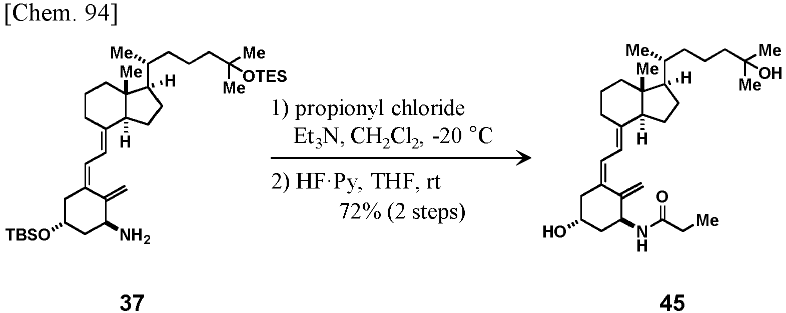

By following the similar procedure described for **38,** 1α-NH(propionyl) VD₃ **45** (1.31 mg, 72%) purified by flash chromatography (chloroform/ethyl acetate/methanol; 18:1:1) was obtained from **37** (2.5 mg, 3.881 µmol) as a solid.
**¹H NMR** (300 MHz, CDCl₃) δ 6.39 (d, *J=* 11.34 Hz, 1H), 5.96 (d, *J* = 11.34 Hz, 1H), 5.33 (d, *J* = 8.58 Hz, 1H), 5.20 (s, 1H), 4.96 (s, 1H), 4.85-4.74 (m, 1H), 4.10-4.01 (m, 1H), 2.85-1.15 (m, 23H), 2.21 (q, *J=* 7.56 Hz, 2H), 1.21 (s, 6H), 1.15 (t, *J=* 7.56 Hz, 3H), 0.93 (d, *J* = 6.18 Hz, 3H), 0.51 (s, 3H);
**HR-MS ESI** Calcd for C₃₀H₄₉N₁Na₁O₃ [M+Na]⁺ : 494.36101, Found: 494.36202.

### Example 16

### 1α-NH(butyryl) VD₃ 46

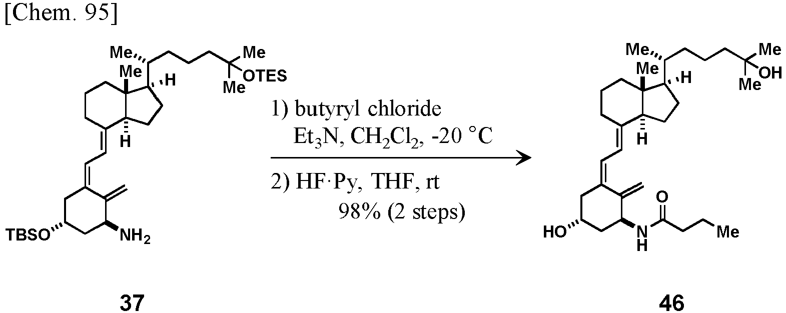

By following the similar procedure described for **38,** 1α-NH(butyryl) VD₃ **46** (1.84 mg, 98%) purified by flash chromatography (chloroform/methanol; 20:1) was obtained from **37** (2.5 mg, 3.881 µmol) as a solid.
**¹H NMR** (300 MHz, CDCl₃) δ 6.39 (d, *J=* 11.34 Hz, 1H), 5.96 (d, *J=* 11.34 Hz, 1H), 5.33 (d, *J* = 8.25 Hz, 1H), 5.21 (s, 1H), 4.95 (s, 1H), 4.84-4.75 (m, 1H), 4.10-4.00 (m, 1H), 2.84-1.15 (m, 23H), 2.16 (t, *J=* 7.56 Hz, 2H), 1.66 (q, *J=* 7.56 Hz, 2H), 1.21 (s, 6H), 0.95 (t, *J=* 7.56 Hz, 3H), 0.93 (d, *J=* 6.18 Hz, 3H), 0.52 (s, 3H);
**HR-MS ESI** Calcd for C₃₁H₅₁N₁Na₁O₃ [M+Na]⁺ : 508.37666, Found: 508.37541.

### Example 17

### 1α-NHEt VD₃ 47

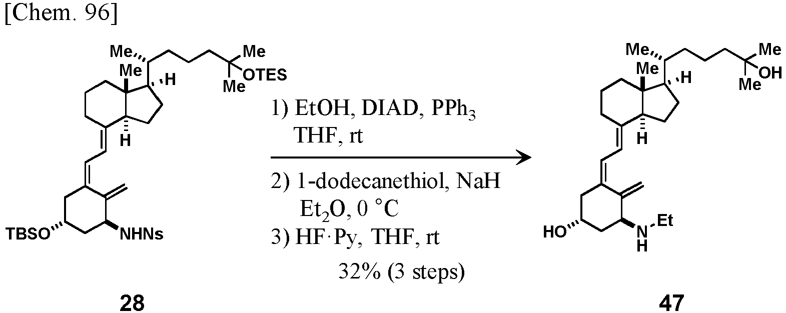

Triphenylphosphine (12.0 mg, 0.0461 mmol), ethanol (4 µL, 0.0690 mmol) and diisopropyl azodicarboxylate (9.0 µL, 0.0461 mmol) were added to a solution of nosylamide **28** (19.1 mg, 0.0230 mmol) in THF (0.43 mL) at room temperature and stirred for 12 h. The reaction mixture was quenched with H₂O. The aqueous layer was extracted with ethyl acetate three times. The combined organic extracts were washed with brine, dried over MgSO₄, and concentrated *in vacuo.* The residue was chromatographed on silica gel (hexane/ethyl acetate; 20:1) to give ethylated nosylamide (13.0 mg, 66%, not pure). To a solution of 1-dodecanethiol (7.2 µL, 0.0303 mmol) in diethyl ether (100 µL) was added sodium hydride (1.2 mg, 0.0288 mmol, 60% stabilized by oil) at 0 °C and stirred for 30 min. The suspension was added to a solution of above ethylated nosylamide (13.0 mg, 0.0152 mmol) in diethyl ether (200 µL). After additive 8 h, the reaction was quenched with H₂O. The aqueous layer was extracted with ethyl acetate three times. The combined organic extracts were washed with brine, dried over MgSO₄, and concentrated *in vacuo.* The residue was chromatographed on silica gel (hexane/ethyl acetate; 10:1 to 1:1) to give ethyl amine (5.8 mg, 57%). HF Py (83.5 mg, 0.842 mmol) was added to a solution of ethyl amine (5.8 mg, 8.628 µmol) in THF (0.50 mL) at room temperature. The reaction was monitored by thin layer chromatography on silica gel plates (eluent: 3:2:1 chloroform to ethyl acetate to methanol). The reaction mixture was quenched with sat. NaHCO₃ aq. The aqueous layer was extracted with ethyl acetate three times. The combined organic extracts were washed with brine, dried over MgSO₄, and concentrated *in vacuo.* The residue was chromatographed on silica gel (NH silica gel, chloroform/methanol; 100:1) to give 1α-NHEt VD₃ **47** (3.93 mg, 86%).
**¹H NMR** (300 MHz, CDCl₃) δ 6.38 (d, *J* = 11.31 Hz, 1H), 5.99 (d, *J* = 11.31 Hz, 1H), 5.12 (d, *J* = 2.04 Hz, 1H), 4.97 (d, *J* = 2.04 Hz, 1H), 4.13-4.04 (m, 1H), 3.33 (t, *J* = 4.47 Hz,, 1H), 2.81 (dd, *J* = 11.67, 3.78 Hz, 1H), 2.68 (dd, *J* = 11.34, 7.23 Hz, 1H), 2.65-1.15 (m, 22H), 2.52 (dd, *J* = 11.34, 7.23 Hz, 1H), 1.21 (s, 6H), 1.08 (t, *J=* 7.20 Hz, 3H), 0.93 (d, *J* = 6.18 Hz, 3H), 0.51 (s, 3H);
**HR-MS ESI** Calcd for C₂₉H₅₀N₁O₂ [M+H]⁺ : 444.38415, Found: 444.38318.

### Example 18

### 1α-NHBu VD₃ 48

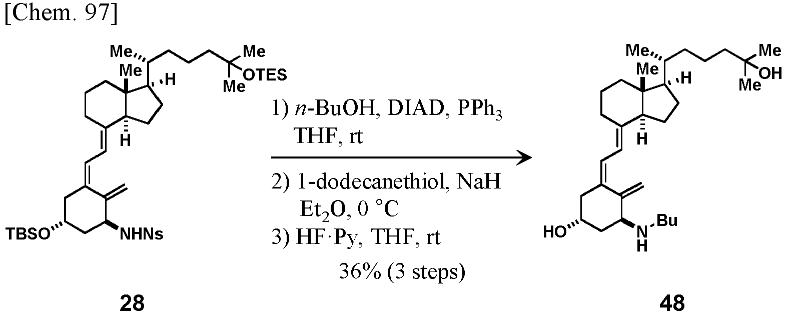

By following the similar procedure described for **47,** 1α-NHBu VD₃ **48** (4.70 mg, 36%) purified by flash chromatography (chloroform/methanol; 200:1) was obtained from **28** (20.4 mg, 0.0246 mmol) as a solid.
**¹H NMR** (300 MHz, CDCl₃) δ 6.38 (d, *J* = 11.31 Hz, 1H), 5.98 (d, *J* = 11.31 Hz, 1H), 5.13 (d, *J=* 2.40 Hz, 1H), 4.98 (d, *J=* 2.40 Hz, 1H), 4.13-4.02 (t, *J=* 4.11 Hz. 1H), 2.84-1.15 (m, 29H), 1.21 (s, 6H), 0.93 (d, *J=* 6.51 Hz, 3H), 0.89 (t, *J* = 7.20 Hz, 3H), 0.51 (s, 3H);
**HR-MS ESI** Calcd for C₃₁H₅₄N₁O₂ [M+H]⁺ : 472.41545, Found: 472.41701.

### Example 19

### 1α-NH(cyclopropylmethyl) VD₃ 49

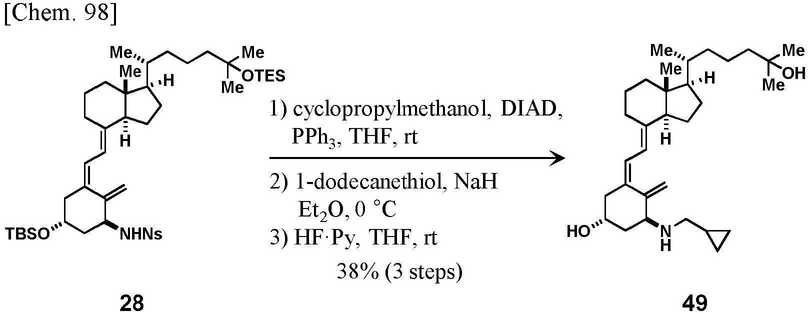

By following the similar procedure described for **47,** 1α-NH(cyclopropylmethyl) VD₃ **49** (8.95 mg, 38%) purified by flash chromatography (chloroform/ethyl acetate/methanol; 6:4:1 to 3:2:2) was obtained from **28** (41.2 mg, 0.0497 mmol) as a solid.
**¹H NMR** (300 MHz, CDCl₃) δ 6.41 (d, *J* = 11.34 Hz, 1H), 5.92 (d, *J* = 11.34 Hz, 1H), 5.13 (d, *J* = 2.07 Hz, 1H), 5.01 (d, *J* = 2.07 Hz, 1H), 4.20-4.09 (m, 1H), 3.39 (t, *J=* 4.11 Hz, 1H), 2.85-1.15 (m, 30H), 1.21 (s, 6H), 0.93 (d, *J* = 6.51 Hz, 3H), 0.52 (s, 3H);
**HR-MS ESI** Calcd for C₃₁H₅₂N₁O₂ [M+H]⁺ : 470.39980, Found: 470.39797.

### Example 20

### 1α-NH(2-morpholinoethyl) VD₃ 50

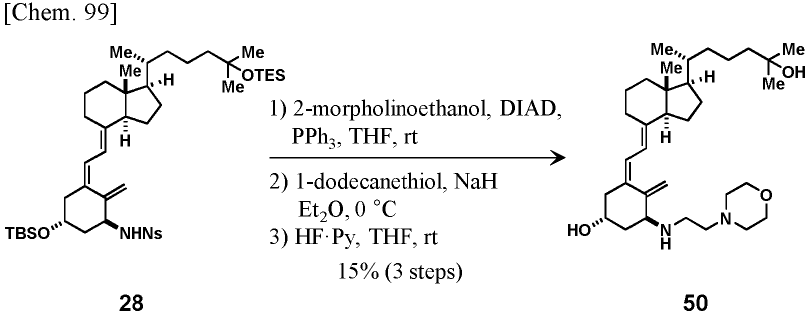

By following the similar procedure described for **47,** 1α-NH(2-morpholinoethyl) VD₃ **50** (2.56 mg, 15%) purified by flash chromatography (chloroform/ethyl acetate/methanol; 6:4:1 to chloroform/methanol; 1:1) was obtained from **28** (38.9 mg, 0.0469 mmol) as a solid.
**¹H NMR** (300 MHz, CDCl₃) δ 6.38 (d, *J* = 11.34 Hz, 1H), 5.98 (d, *J* = 11.34 Hz, 1H), 5.21 (s, 1H), 5.04 (s, 1H), 4.18-4.08 (m, 1H), 3.68 (t, *J=* 4.47 Hz, 4H), 3.48-3.42 (m, 1H), 2.83-1.15 (m, 31H), 1.21 (s, 6H), 0.93 (d, *J=* 6.18 Hz, 3H), 0.50 (s, 3H);
**HR-MS ESI** Calcd for C₃₃H₅₇N₂O₃ [M+H]⁺ : 529.43692, Found: 529.43542.

### Example 21

### 1α-NH(2-(4-hydroxy piperidyl)ethyl) VD₃ 52

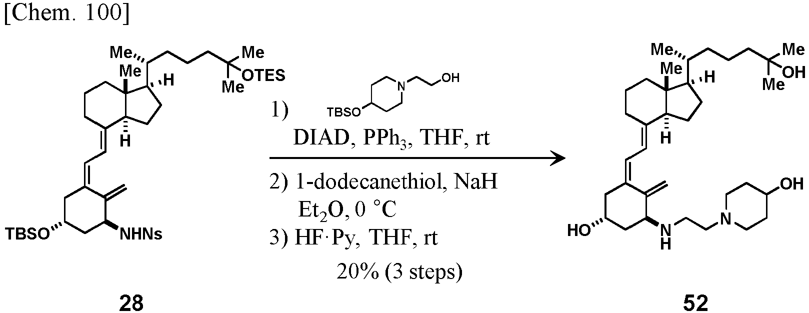

By following the similar procedure described for **47,** 1α-NH(2-(4-hydroxy piperidyl)ethyl) VD₃ **52** (4.22 mg, 20%) purified by flash chromatography (NH silica gel, chloroform/ethyl acetate/methanol; 18:1:1) was obtained from **28** (43.5 mg, 0.0525 mmol) as a solid.
**¹H NMR** (300 MHz, CDCl₃) δ 6.36 (d, *J* = 11.34 Hz, 1H), 6.01 (d, *J* = 11.34 Hz, 1H), 5.16 (d, *J=* 2.07 Hz, 1H), 4.98 (d, *J=* 2.07 Hz, 1H), 4.16-4.05 (m, 1H), 3.72-3.62 (m, 1H), 3.34 (t, *J=* 4.47 Hz, 1H), 2.83-1.15 (m, 34H), 1.21 (s, 6H), 0.93 (d, *J* = 5.82 Hz, 3H), 0.51 (s, 3H);
**HR-MS ESI** Calcd for C₃₄H₅₉N₂O₃ [M+H]⁺ : 543.45257, Found: 543.44912.

### Example 22

### 1α-NHMs VD₃ 53

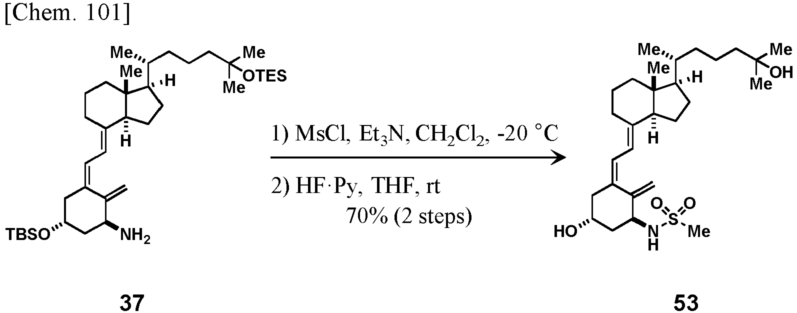

By following the similar procedure described for **38,** 1α-NHMs VD₃ **53** (1.49 mg, 70%) purified by flash chromatography (chloroform/methanol; 15:1) was obtained from **37** (2.8 mg, 4.347 µmol) as a solid.
**¹H NMR** (300 MHz, CDCl₃) δ 6.41 (d, *J* = 11.34 Hz, 1H), 5.95 (d, *J* = 11.34 Hz, 1H), 5.36 (s, 1H), 5.06 (s, 1H), 4.34-4.25 (m, 1H), 4.14-4.04 (m, 1H), 2.83 (d, *J=* 12.4 Hz, 1H), 2.98 (s, 3H), 2.83-1.15 (m, 23H), 1.21 (s, 6H), 0.93 (d, *J* = 6.18 Hz, 3H), 0.52 (s, 3H);
**HR-MS ESI** Calcd for C₂₈H₄₇N₁Na₁O₄S₁ [M+Na]⁺ : 516.31235, Found: 516.31391.

### Example 23

### 1α-NH(p-fluoroBz) VD₃ 54

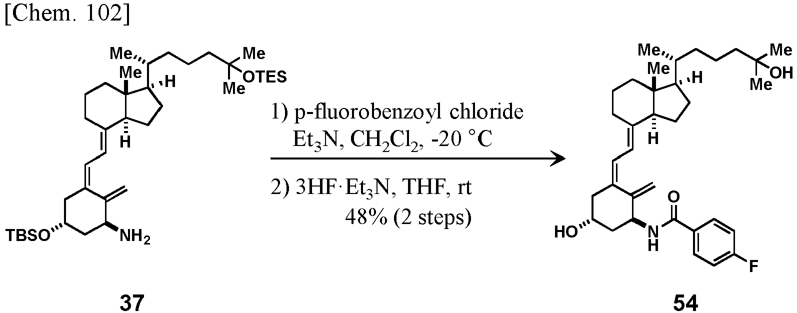

By following the similar procedure described for **38,** 1α-NH(*p*-fluoroBz) VD₃ **54** (1.89 mg, 48%) purified by flash chromatography (NH silica, hexane/ethyl acetate; 20:1) was obtained from **37** (4.7 mg, 7.296 µmol) as a solid.
**¹H NMR** (400 MHz, CDCl₃) δ 7.72 (dd, *J* = 8.72, 5.04 Hz,, 2H), 7.08 (t, *J* = 8.72 Hz, 2H), 6.45 (d, *J* = 11.00 Hz, 1H), 6.00 (d, *J* = 11.00 Hz, 1H), 5.94 (d, *J=* 7.80 Hz, 1H), 5.30 (s, 1H), 5.01 (s, 1H), 4.98-4.91 (m, 1H), 4.12-4.04 (m, 1H), 2.84 (dd, *J* = 12.84, 3.68 Hz, 1H), 2.64 (dd, *J=* 12.84, 3.68 Hz, 1H), 2.35 (dd, *J=* 12.84, 7.80 Hz, 1H), 2.21-1.15 (m, 20H), 1.21 (s, 6H), 0.94 (d, *J=* 5.96 Hz, 3H), 0.50 (s, 3H);
**HR-MS ESI** Calcd for C₃₄H₄₈F₁N₁Na₁O₃ [M+Na]⁺ : 560.35159, Found: 560.35351.

### Example 24

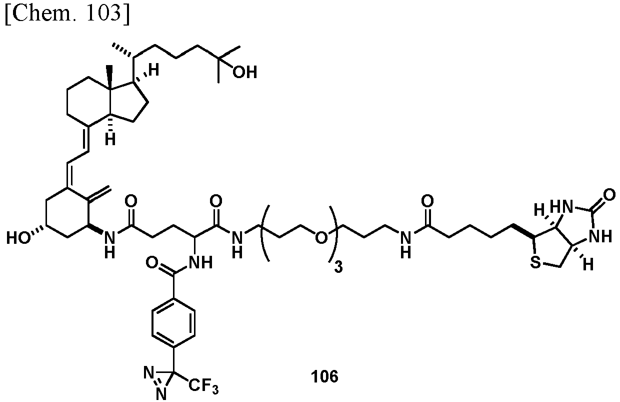

To a stirred solution of **105** (4.9 mg, 0.0062 mmol) and **19a** (1.4 mg, 0.0031 mmol) in methanol (1.0 mL) was added 4-(4,6-dimethoxy-1,3,5-triazin-2-yl)-4-methylmorpholinium chloride (1.7 mg, 0.0062 mmol) and *N*,*N-*diisopropylethylamine (0.54 µL, 0.0031 mmol) at 0 °C and the mixture was stirred at room temperature overnight. The solvent was evaporated and the residue was purified by HPLC to yield **106** (1.9 mg, 0.0016 mmol, 51%).
¹H NMR (CD₃OD) δ 0.51 (s, 3H), 0.94-3.36 (m, 47H), 3.48-3.64 (m, 20H), 4.03-4.81 (m, 4H), 4.88 (s, 1H), 5.15 (s, 1H), 5.96 (d, 1H), 6.32 (d, 1H), 7.35 (d, 2H), 8.01 (d, 2H);
HRMS (FAB) Calcd for C₆₁H₉₀F₃N₈NaO₁₀S [M+Na]⁺: 1207.6429, Found: 1207.6427.

### Example 25

### 19-nor 1β-NPhth-3β-OTBS VD₃ 68 and 1α-OTBS-3α-NPhth VD₃ 69

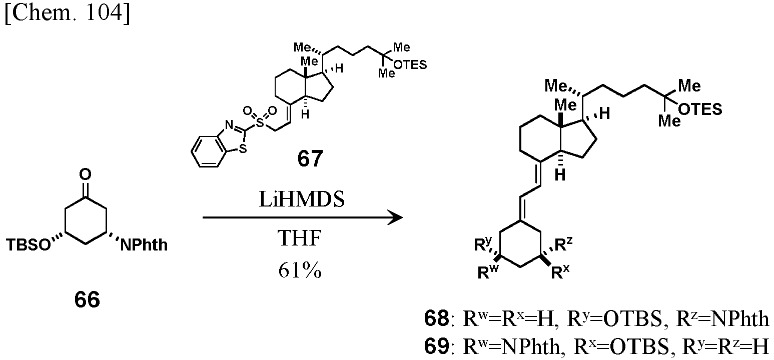

To a solution of benzothiazolyl sulfone **67** (61.9 mg, 0.102 mmol) in THF (1.0 mL) was added dropwise lithium bis(trimethylsilyl)amide (90 µL, 0.116 mmol, 1.3 M in THF) at -78 °C. After stirring for 1 h, a solution of ketone **66** (29.0 mg, 0.078 mmol) in THF (1.0 mL) was added. The mixture was stirred for 2 h at same temperature, then warmed up to room temperature and stirred for 3 h. The reaction mixture was quenched with water and the aqueous layer was extracted with ethyl acetate three times. The combined organic extracts were washed with brine, dried over MgSO₄, and concentrated *in vacuo.* The residue was chromatographed on silica gel (Hexane/ethyl acetate; 15:1) to give **68** (12.2 mg, 21%), **69** (24.0 mg, 40%) and starting material **67** (26.9 mg, 44% recovery).
**68; ¹H NMR** (300 MHz, CDCl₃) δ 7.85-7.81 (m, 2H), 7.73-7.69 (m, 2H), 6.23 (d, *J=* 11.45 Hz, 1H), 5.73 (d, *J* = 11.45 Hz, 1H), 4.20-4.10 (m, 1H), 3.67 (dddd, *J=* 10.99, 10.76, 5.95, 4.58 Hz, 1H), 2.82-1.15 (m, 25H), 1.18 (s, 6H), 0.95-0.91 (m, 12H), 0.88 (s, 9H), 0.57 (s, 3H), 0.55 (q, *J* = 7.79 Hz, 6H), 0.08 (s,3H), 0.05 (s, 3H).
**69; ¹H NMR** (300 MHz, CDCl₃) δ 7.84-7.80 (m, 2H), 7.72-7.68 (m, 2H), 6.23 (d, *J=* 11.45 Hz, 1H), 5.85 (d, *J=* 11.45 Hz, 1H), 4.14 (dddd, *J=* 12.82, 12.36, 4.12, 4.12 Hz, 1H), 3.58 (dddd, *J=* 10.99, 10.53, 6.42, 4.12 Hz, 1H), 3.03-2.97 (m, 2H), 2.79-2.74 (m, 1H), 2.49 (ddd, *J* = 12.36, 12.36, 10.99 Hz, 1H), 2.22 (dd, *J* = 12.36, 3.66 Hz, 1H), 2.02-1.15 (m, 20H), 1.19 (s, 6H), 0.95 (t, *J=* 7.79 Hz, 9H), 0.93 (d, *J=* 6.41 Hz, 3H), 0.89 (s, 9H), 0.56 (q, *J* = 7.79 Hz, 6H), 0.56 (s, 3H), 0.09 (s,3H), 0.06 (s, 3H).

### Example 26 (Comparative Example)

### 19-nor 1β-amino-3β-OTBS VD₃ 70 and 1α-OTBS-3α-amino VD₃ 71

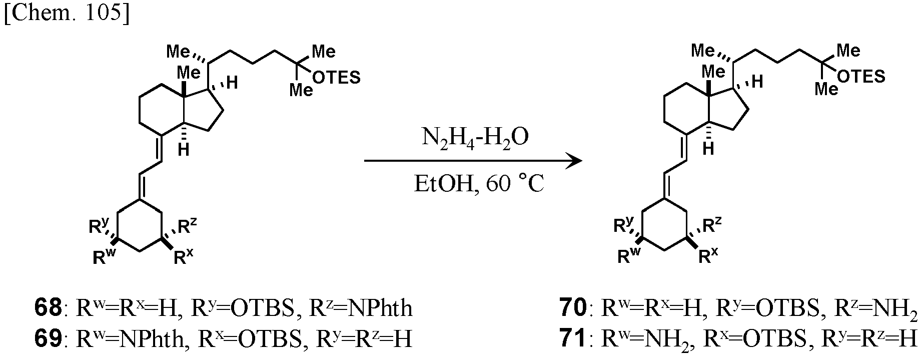

To a solution of **68** (16.7 mg, 0.0219 mmol) in ethanol (0.55 mL) was added hydrazine hydrate (5.3 µL, 0.110 mmol) at room temperature. The mixture was stirred for 1.5 h at 60 °C. The reaction mixture was filtered through a plug of cotton and filtrate was concentrated to give crude **70.**

By following the same procedure described for **70,** crude **71** was obtained from **37** (19.5 mg, 0.0256 mmol).

### Example 27

### 19-nor 1β-NHTs-3β-OH VD₃ 72 and 1α-OH-3α-NHTs VD₃ 73

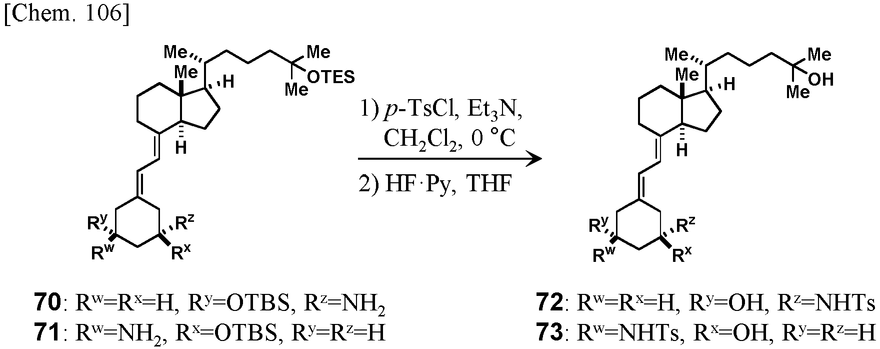

To a solution of crude **70** (ca. 0.011 mmol) in CH₂Cl₂ (0.60 mL) was added trimethylamine (4.0 µL, 0.028 mmol) andp-toluenesulfonyl chloride (2.5 mg, 0.013 mmol) at 0 °C. After stirring for 2.5 h, the reaction mixture was quenched with water and the aqueous layer was extracted with dichloromethane three times, dried over MgSO₄, and concentrated *in vacuo.* The residue was chromatographed on silica gel (NH silica gel, hexane/ethyl acetate; 30:1 to 4:1) to give 1β-NHTs-3β-OTBS VD₃ (6.4 mg, 73%) as a colorless oil. HF Py (121.0 mg, 1.221 mmol) was added to a solution of above 1β-NHTs-3β-OTBS VD₃ (6.4 mg, 8.139 µmol) in THF (0.60 mL) and the mixture was stirred for 20 h. The reaction mixture was quenched with sat. NaHCO₃ aq. and aqueous layer was extracted with ethyl acetate three times. The combined organic extracts were washed with brine, dried over MgSO₄, and concentrated *in vacuo.* The residue was chromatographed on silica gel (Hexane/ethyl acetate; 3:1 to 1:2) to give 1β-NHTs-3β-OH VD₃ **72** (4.29 mg, 94%) as a white solid.
**¹H NMR** (300 MHz, CDCl₃) δ 7.76 (d, *J=* 8.26 Hz, 2H), 7.29 (d, *J=* 8.26 Hz, 2H), 6.26 (d, *J* = 11.35 Hz, 1H), 5.69 (d, *J* = 11.35 Hz, 1H), 5.20 (d, *J =* 8.94 Hz, 1H), 3.95-3.87 (m, 1H), 3.54-3.44 (m, 1H), 2.76 (dd, *J =* 11.70, 3.78 Hz, 1H), 2.45-2.36 (m, 1H), 2.43 (s, 3H), 2.28 (dd, *J=* 13.42, 6.88 Hz, 1H), 2.15 (dd, *J=* 11.42, 6.54 Hz, 1H), 2.05-1.20 (m, 21H), 1.23 (s, 6H), 0.94 (d, *J=* 6.19 Hz, 3H), 0.54 (s, 3H).
**HR-MS ESI** Calcd for C₃₃H₅₁N₁Na₁O₄S₁ [M+Na]⁺ : 580.34365, Found: 580.34011.

By following the same procedure described for **72,** 1α-OTBS-3α-NHTs VD₃ 73 (131.4 mg, 83%) was obtained from **71** (ca. 0.283 mmol) as a white solid.
**¹H NMR** (300 MHz, CDCl₃) δ 7.76 (d, *J=* 8.26 Hz, 2H), 7.30 (d, *J=* 8.26 Hz, 2H), 6.10 (d, *J* = 11.01 Hz, 1H), 5.76 (d, *J* = 11.01 Hz, 1H), 5.20 (d, *J* = 8.60 Hz, 1H), 3.88-3.81 (m, 1H), 3.48-3.40 (m, 1H), 2.74-2.70 (m, 1H), 2.53 (dd, *J* = 13.07, 3.44 Hz, 1H), 2.43 (s, 3H), 2.34-1.20 (m, 23H), 1.22 (s, 6H), 0.94 (d, *J=* 6.19 Hz, 3H), 0.56 (s, 3H).
**HR-MS ESI** Calcd for C₃₃H₅₁N₁Na₁O₄S₁ [M+Na]⁺ : 580.34365, Found: 580.34781.

### Example 28

### 19-nor 1β-NH(p-SCF₃Bz)-3β-OH VD₃ 74 and 1α-OH-3α-NH(p-SCF₃Bz) VD₃ 75

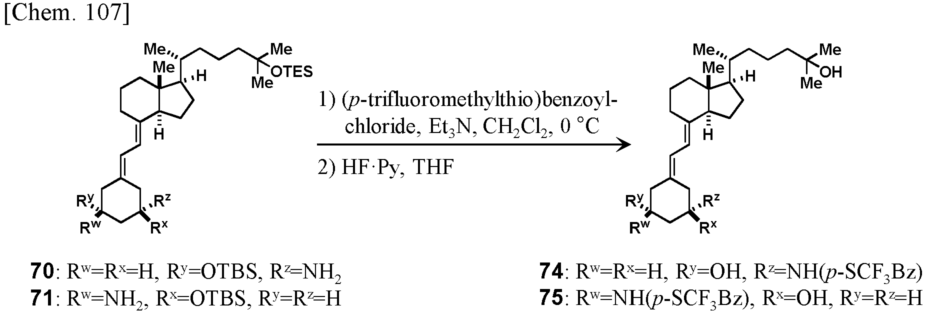

To a solution of crude **70** (ca. 0.011 mmol) in CH₂Cl₂ (0.60 mL) was added trimethylamine (4.0 µL, 0.028 mmol) and (p-trifluoromethylthio)benzoyl chloride (2.2 µL, 0.013 mmol) at 0 °C. After stirring for 1 h, the reaction mixture was quenched with water and the aqueous layer was extracted with dichloromethane three times, dried over MgSO₄, and concentrated *in vacuo.* The residue was chromatographed on silica gel (hexane/ethyl acetate; 10:1 to 6:1) to give 1β-NH(*p*-SCF₃Bz)-3β-OTBS VD₃ (7.6 mg, 82%) as a colorless oil. HF Py (129.3 mg, 1.304 mmol) was added to a solution of above 1β-NH(*p*-SCF₃Bz)-3β-OTBS VD₃ (7.6 mg, 9.087 µmol) in THF (0.60 mL) and the mixture was stirred for 20 h. The reaction mixture was quenched with sat. NaHCO₃ aq. and aqueous layer was extracted with ethyl acetate three times. The combined organic extracts were washed with brine, dried over MgSO₄, and concentrated *in vacuo.* The residue was chromatographed on silica gel (Hexane/ethyl acetate; 3:1 to 1:2) to give 1β-NH(*p*-SCF₃Bz)-3β-OH VD₃ **74** (3.24 mg, 59%) as a white solid.
**¹H NMR** (300 MHz, CDCl₃) δ 7.73-7.64 (m, 4H), 7.50 (d, *J=* 8.26 Hz, 1H), 6.34 (d, *J=* 11.35 Hz, 1H), 5.72 (d, *J=* 11.35 Hz, 1H), 4.51-4.46 (m, 1H), 3.31-3.27 (m, 1H), 2.94 (dd, *J=* 13.42, 3.78 Hz, 1H), 2.79-2.74 (m, 1H), 2.60-2.56 (m, 1H), 2.35-1.20 (m, 22H), 1.21 (s, 6H), 0.90 (d, *J* = 6.54 Hz, 3H), 0.50 (s, 3H).
**HR-MS ESI** Calcd for C₃₄H₄₈F₃N₁Na₁O₃S₁ [M+Na]⁺ : 630.32047, Found: 630.31864.

By following the same procedure described for **74,** 1α-OTBS-3α-NHTs VD₃ **75** (5.06 mg, 65%) was obtained from **71** (ca. 0.013 mmol) as a white solid.
**¹H NMR** (300 MHz, CDCl₃) δ 7.80-7.67 (m, 4H), 7.33 (d, *J =* 8.26 Hz, 1H), 6.29 (d, *J=* 11.01 Hz, 1H), 5.84 (d, *J=* 11.01 Hz, 1H), 4.40-4.32 (m, 1H), 4.23-4.18 (m, 1H), 2.76-2.68 (m, 1H), 2.60 (dd, *J=* 14.10, 5.85 Hz, 1H), 2.54-1.21 (m, 22H), 2.37 (dd, *J=* 14.10, 5.85 Hz, 1H), 1.22 (s, 6H), 0.94 (d, *J* = 6.19 Hz, 3H), 0.55 (s, 3H).
**HR-MS ESI** Calcd for C₃₄H₄₈F₃N₁Na₁O₃S₁ [M+Na]⁺ : 630.32047, Found: 630.32327.

### Example 29

### 19-nor 1β-NPhth-3β-OH VD₃ 76 and 1α-OH-3α-NHPhth VD₃ 77

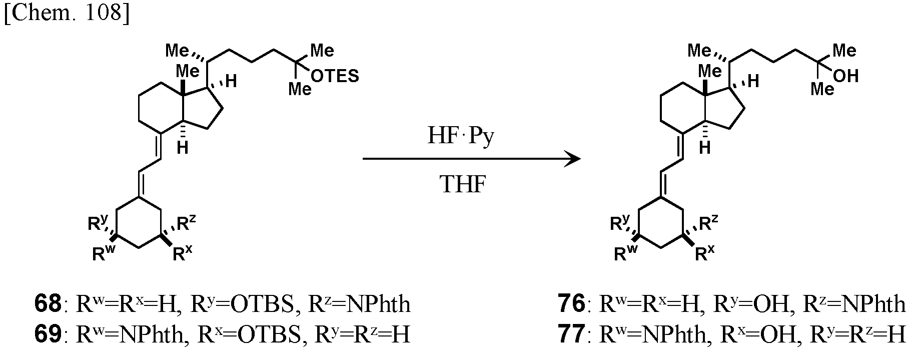

HF Py (150.0 mg, 1.513 mmol) was added to a solution of above 1β-NPhth-3β-OTBS VD₃ **68** (12.2 mg, 16.005 µmol) in THF (0.60 mL) and the mixture was stirred for 20 h. The reaction mixture was quenched with sat. NaHCO₃ aq. and aqueous layer was extracted with ethyl acetate three times. The combined organic extracts were washed with brine, dried over MgSO₄, and concentrated *in vacuo.* The residue was chromatographed on silica gel (Hexane/ethyl acetate; 3:1 to 1:1) to give 1β-NPhth-3β-OH VD₃ **76** (8.54 mg, 100%) as a white solid.
**¹H NMR** (300 MHz, CDCl₃) δ 7.86-7.70 (m, 4H), 6.27 (d, *J=* 11.35 Hz, 1H), 5.72 (d, *J=* 11.35 Hz, 1H), 4.24-4.13 (m, 1H), 3.72 (dddd, *J=* 11.01, 10.66, 6.88, 4.47 Hz, 1H), 2.81-2.74 (m, 2H), 2.61 (dd, *J=* 12.07, 4.13 Hz, 1H), 2.39 (ddd, *J* = 12.04, 11.70, 11.70 Hz, 1H), 2.25-1.19 (m, 21H), 1.20 (s, 6H), 0.93 (d, *J=* 6.54 Hz, 3H), 0.56 (s, 3H).
**HR-MS ESI** Calcd for C₃₄H₄₇N₁Na₁O₄ [M+Na]⁺ : 556.34028, Found: 556.34468.

By following the same procedure described for **76,** 1α-OH-3α-NHPhth VD₃ 77 (8.76 mg, 94%) was obtained from **69** (13.3 mg, 17.448 µmol) as a white solid.
**¹H NMR** (300 MHz, CDCl₃) δ 7.85-7.69 (m, 4H), 6.25 (d, *J =* 11.35 Hz, 1H), 5.86 (d, *J=* 11.35 Hz, 1H), 4.17 (dddd, *J* = 12.38, 12.38, 4.13, 3.78 Hz, 1H), 3.72 (dddd, *J* = 11.01, 11.01, 6.54, 4.13 Hz, 1H), 3.16 (dd, *J=* 12.73, 4.13 Hz, 2H), 3.00 (dd, *J=* 12.38, 12.38 Hz, 1H), 2.77 (dd, *J* = 12.04, 3.78 Hz, 1H), 2.46 (ddd, *J* = 12.04, 11.70, 11.70 Hz, 1H), 2.77 (dd, *J* = 12.38, 3.78 Hz, 1H), 2.17-1.21 (m, 19H), 1.22 (s, 6H), 0.94 (d, *J=* 6.19 Hz, 3H), 0.55 (s, 3H).
**HR-MS ESI** Calcd for C₃₄H₄₇N₁Na₁O₄ [M+Na]⁺ : 556.34028, Found: 556.34111.

### Example 30

### 19-nor 1α-NPhth-3β-OTBS VD₃ 82 and 1α-OTBS-3β-NPhth VD₃ 83

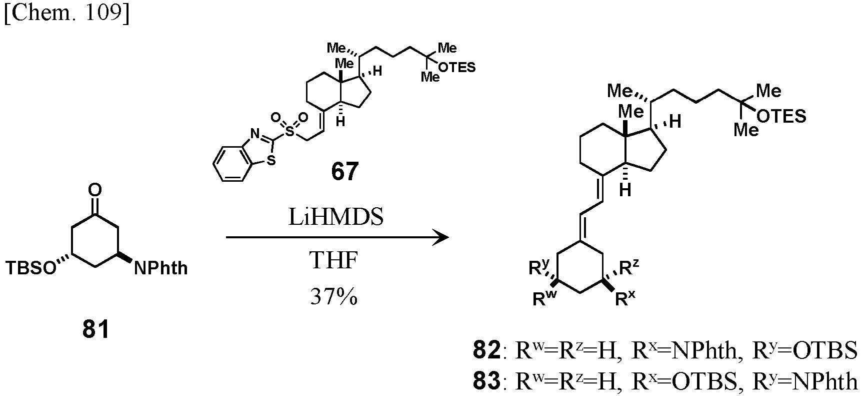

To a solution of benzothiazolyl sulfone **67** (202.0 mg, 0.334 mmol) in THF (2.0 mL) was added dropwise lithium bis(trimethylsilyl)amide (0.28 mL, 0.336 mmol, 1.3 M in THF) at - 78 °C. After stirring for 1 h, a solution of ketone **81** (72.4 mg, 0.194 mmol) in THF (1.0 mL) was added. The mixture was stirred for 2 h at same temperature, then warmed up to room temperature and stirred for 0.5 h. The reaction mixture was quenched with water and the aqueous layer was extracted with ethyl acetate three times. The combined organic extracts were washed with brine, dried over MgSO₄, and concentrated *in vacuo.* The residue was chromatographed on silica gel (Hexane/ethyl acetate; 30:1 to 15:1) to give **82** (21.5 mg, 15%), **83** (33.8 mg, 22%) and starting material **67** (98.7 mg, 49% recovery).
**82; ¹H NMR** (400 MHz, CDCl₃) δ 7.84-7.68 (m, 4H), 6.14 (d, *J=* 10.99 Hz, 1H), 5.77 (d, *J =* 10.99 Hz, 1H), 4.64 (dddd, *J* = 11.91, 11.91, 4.58, 44.12 Hz, 1H), 4.26-4.22 (m, 1H), 2.89-1.14 (m, 25H), 1.17 (s, 6H), 0.93(t, *J* = 10.99 Hz, 9H), 0.91 (d, *J=* 6.41 Hz, 3H), 0.89 (s, 9H), 0.54 (q, *J=* 8.24 Hz, 6H), 0.51 (s, 3H), 0.06 (s,3H), 0.05 (s, 3H).

### Example 31 (Comparative Example)

### 19-nor 1α-amino-3β-OTBS VD₃ 84

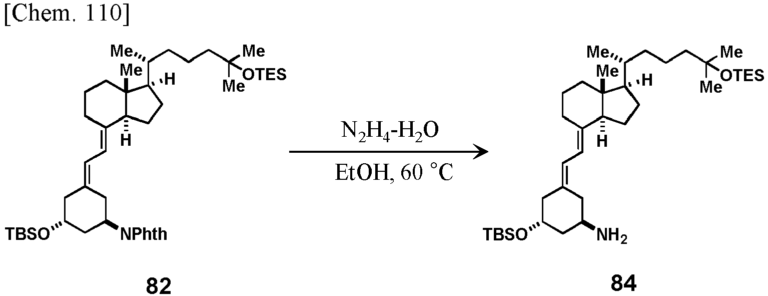

By following the same procedure described for **70,** crude **84** was obtained from **82** (3.8 mg, 4.985 µmol).

### Example 32

### 19-nor 1α-NHTs-3β-OH VD₃ 85

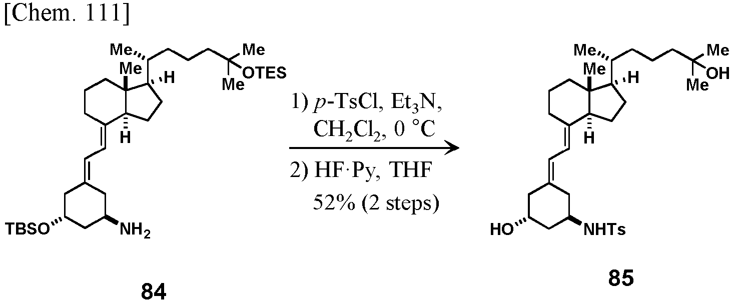

By following the same procedure described for **72,** 1α-NHTs-3β-OH VD₃ **85** (1.58 mg, 52%) was obtained from crude **84** (ca. 5.422 µmol) as a white solid.
**¹H NMR** (300 MHz, CDCl₃) δ 7.77-7.29 (m, 4H), 6.27 (d, *J=* 11.35 Hz, 1H), 5.59 (d, *J=* 11.35 Hz, 1H), 5.20 (d, *J* = 8.94 Hz, 1H), 4.00-3.93 (m, 1H), 3.69-3.60 (m, 1H), 2.44 (s, 3H), 2.28-1.20 (m, 24H), 1.23 (s, 6H), 0.95 (d, *J=* 6.19 Hz, 3H), 0.56 (s, 3H).
**HR-MS FAB** Calcd for C₃₃H₅₁N₁Na₁O₄S₁ [M+Na]⁺ : 580.3437 , Found: 580.3435.

### Example 33

### 19-nor 1α-NH(p-SCF₃Bz)-3β-OH VD₃ 86

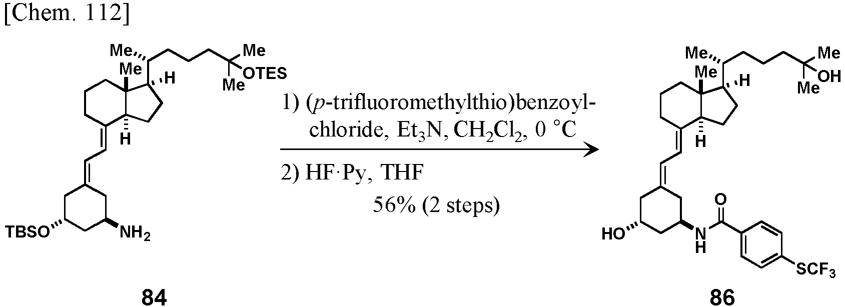

By following the same procedure described for **74,** 1α-NH(*p*-SCF₃Bz)-3β-OH VD₃ 86 (1.70 mg, 56%) was obtained from crude **84** (ca. 4.985 µmol) as a white solid.
**¹H NMR** (300 MHz, CDCl₃) δ 7.73-7.65 (m, 4H), 6.42 (d, *J=* 11.35 Hz, 1H), 6.03 (d, *J=* 8.26 Hz, 1H), 5.83 (d, *J* = 11.35 Hz, 1H), 4.55-4.48 (m, 1H), 3.97-3.88 (m, 1H), 2.85-1.20 (m, 25H), 1.21 (s, 6H), 0.93 (d, *J=* 5.85 Hz, 3H), 0.43 (s, 3H).
**HR-MS FAB** Calcd for C₃₄H₄₈F₃N₁Na₁O₃S₁ [M+Na]⁺ : 630.3205 , Found: 630.3207.

### Example 34

### 19-nor 1α-NPhth-3β-OH VD₃ 87

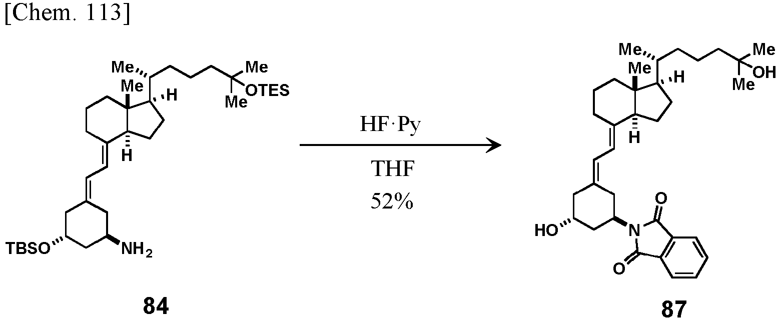

By following the same procedure described for **76,** 1α-NPhth-3β-OH VD₃ **87** (1.46 mg, 52%) was obtained from crude **84** (ca. 5.248 µmol) as a white solid.
**¹H NMR** (300 MHz, CDCl₃) δ 7.85-7.69 (m, 4H), 6.29 (d, *J=* 11.01 Hz, 1H), 5.80 (d, *J=* 11.01 Hz, 1H), 4.60-4.49 (m, 1H), 4.34-4.25 (m, 1H), 2.94-1.20 (m, 25H), 1.20 (s, 6H), 0.92 (d, *J =* 6.19 Hz, 3H), 0.51 (s, 3H).
**HR-MS FAB** Calcd for C₃₄H₄₈N₁O₄ [M+H]⁺ : 534.3583, Found: 534.3567.

### Example 35

### 19-nor 1α-NHAc-3β-OH VD₃ 88

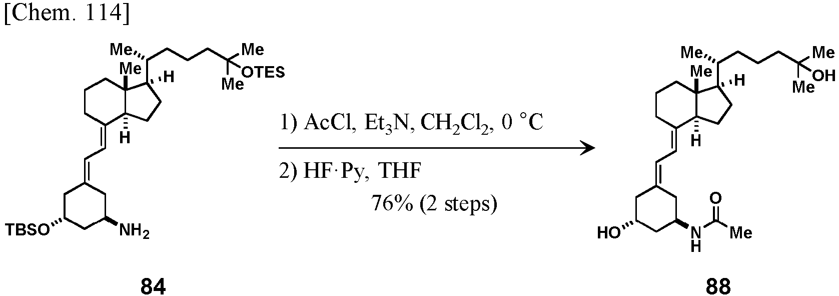

By following the similar procedure described for **76,** 1α-NHAc-3β-OH VD₃ **88** (2.15 mg, 76%) was obtained from crude **84** (ca. 5.422 µmol) as a white solid.
**¹H NMR** (300 MHz, CD₃OD) δ 6.25 (d, *J=* 10.32 Hz, 1H), 5.82 (d, *J=* 10.32 Hz, 1H), 4.70-4.55 (m, 1H), 4.06-3.96 (m, 1H), 2.87-1.15 (m, 28H), 1.16 (s, 6H), 0.97 (d, *J=* 5.50 Hz, 3H), 0.56 (s, 3H).
**HR-MS FAB** Calcd for C₂₈H₄₇N₁Na₁O₃ [M+Na]⁺ : 468.3454 , Found: 468.3454.

### Example 36

### 19-nor 1α-NHMs-3β-OH VD₃ 89

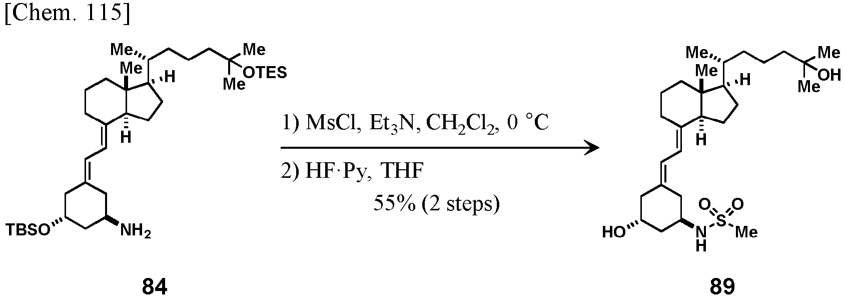

By following the similar procedure described for **76,** 1α-NHMs-3β-OH VD₃ **89** (1.64 mg, 55%) was obtained from crude **84** (ca. 5.422 µmol) as a white solid.
**¹H NMR** (300 MHz, CDCl₃) δ 6.32 (d, *J* = 11.01 Hz, 1H), 5.81 (d, *J* = 11.01 Hz, 1H), 4.08-4.00 (m, 1H), 3.84-3.76 (m, 1H), 2.98 (s, 3H), 2.78 (dd, *J=* 13.42, 4.13 Hz, 1H), 2.69 (dd, *J=* 13.42, 4.13 Hz, 1H), 2.49 (dd, *J* = 13.42, 4.13 Hz, 1H), 2.29 (dd, *J* = 13.07, 7.22 Hz, 1H), 2.19 (dd, *J* = 13.07, 7.22 Hz, 1H), 2.05-1.20 (m, 20H), 1.22 (s, 6H), 0.94 (d, *J=* 6.19 Hz, 3H), 0.54 (s, 3H).
**HR-MS FAB** Calcd for C₂₇H₄₇N₁Na₁O₄S₁ [M+Na]⁺ : 504.3124 , Found: 504.3121.

### Example 37

### 19-nor 1α-NH(trifluoroacetyl)-3β-OH VD₃ 90

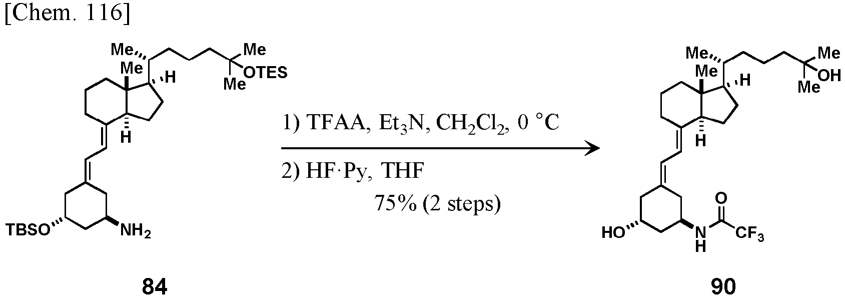

By following the similar procedure described for **76,** 1α-NH(trifluoroacetyl)-3β-OH VD₃ **90** (2.04 mg, 75%) was obtained from crude **84** (ca. 5.422 µmol) as a white solid.
**¹H NMR** (300 MHz, CDCl₃) δ 6.38 (d, *J* = 11.35 Hz, 1H), 6.13 (d, *J* = 7.91 Hz, 1H), 5.77 (d, *J* = 11.35 Hz, 1H), 4.37-4.25 (m, 1H), 3.95 (dddd, *J =* 7.91, 7.91, 4.13, 4.13 Hz, 1H), 2.80 (dd, *J* = 11.70, 3.78 Hz, 1H), 2.57-1.20 (m, 24H), 1.22 (s, 6H), 0.94 (d, *J=* 6.19 Hz, 3H), 0.51 (s, 3H).
**HR-MS FAB** Calcd for C₂₈H₄₅F₃N₁O₃ [M+H]⁺ : 500.3351 , Found: 500.3340.

### Example 38

### 19-nor 1α-NHDns-3β-OH VD₃ 91

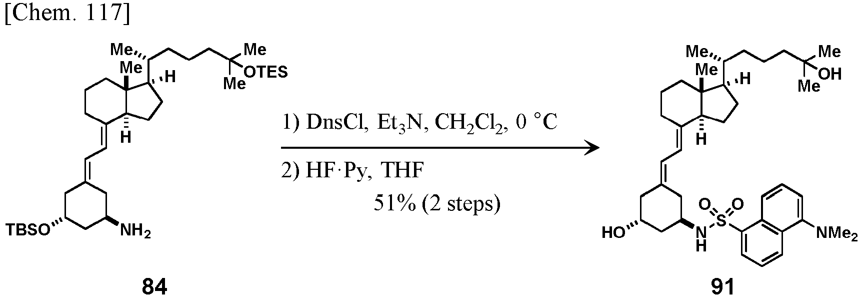

By following the similar procedure described for **76,** 1α-NHDns-3β-OH VD₃ **91** (1.72 mg, 51%) was obtained from crude **84** (ca. 5.422 µmol) as a white solid.
**¹H NMR** (300 MHz, CDCl₃) δ 8.56-7.17 (m, 6H), 6.23 (d, *J=* 11.70 Hz, 1H), 5.60 (d, *J=* 11.70 Hz, 1H), 4.54 (d, *J* = 8.26 Hz, 1H), 3.92-3.85 (m, 1H), 3.65-3.54 (m, 1H), 2.90 (s, 6H), 2.76-1.23 (m, 25H), 1.24 (s, 6H), 0.95 (d, *J=* 6.19 Hz, 3H), 0.53 (s, 3H).
**HR-MS FAB** Calcd for C₃₈H₅₆N₂Na₁O₄S₁ [M+Na]⁺ : 659.3859 , Found: 659.3860.

### Example 39 (Comparative Example)

### 19-nor 1-OBz-3-OTBS VD₃ 94

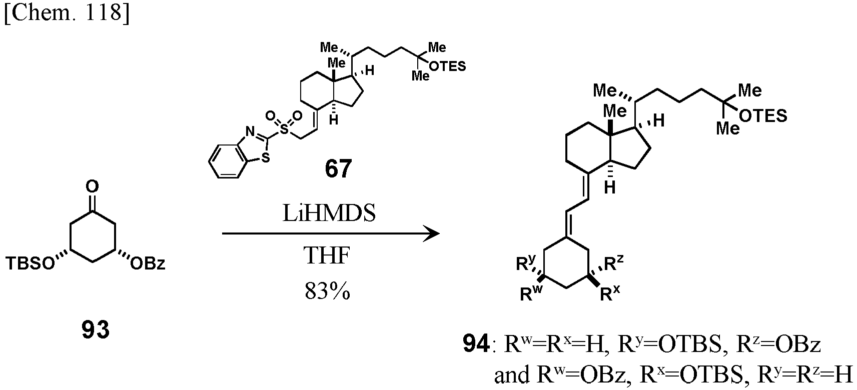

To a solution of benzothiazolyl sulfone **67** (558.1 mg, 0.924 mmol) and ketone **93** (214.7 mg, 0.616 mmol) in THF (12.3 mL) was added dropwise lithium bis(trimethylsilyl)amide (0.94 mL, 1.232 mmol, 1.3 M in THF) at -78 °C. The mixture was stirred for 1 h at same temperature, then warmed up to room temperature and stirred for 0.5 h. The reaction mixture was quenched with water and the aqueous layer was extracted with ethyl acetate three times. The combined organic extracts were washed with brine, dried over MgSO₄, and concentrated *in vacuo.* The residue was chromatographed on silica gel (Hexane/ethyl acetate; 75:1 to 10:1) to give **94** (379.1 mg, 83%) as isomer mixture and starting material **67** (112.9 mg, 20% recovery).

### Example 40 (Comparative Example)

### 19-nor 1β-OBz-3β-OTBS VD₃ 95 and 1α-OTBS-3α-OBz VD₃ 96

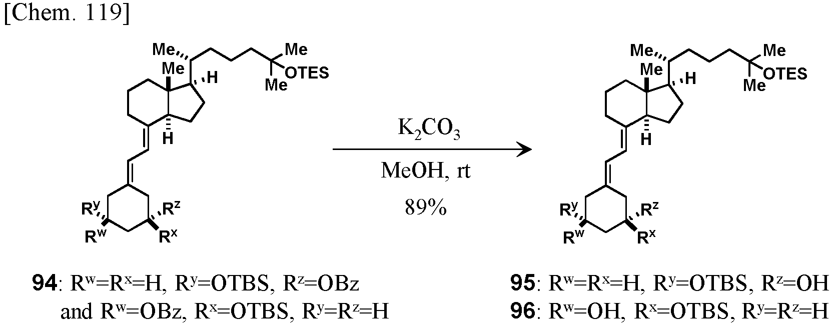

**94** (496.7 mg, 0.674 mmol) was dissolved in methanol (13.5 mL), then the solution was added potassium carbonate (372.5 mg, 2.695 mmol) at room temperature and stirred for 13 h. The reaction mixture was quenched with sat. NH₄Cl aq. and the aqueous layer was extracted with dichloromethane three times. The combined organic extracts were dried over MgSO₄, and concentrated *in vacuo.* The residue was chromatographed on silica gel (Hexane/ethyl acetate; 15:1 to 5:1) to give **95** (118.0 mg, 44%) and isomer **96** (192.6 mg, 45%).

### Example 41 (Comparative Example)

### 19-nor 1α-F-3β-OTBS VD₃ 97

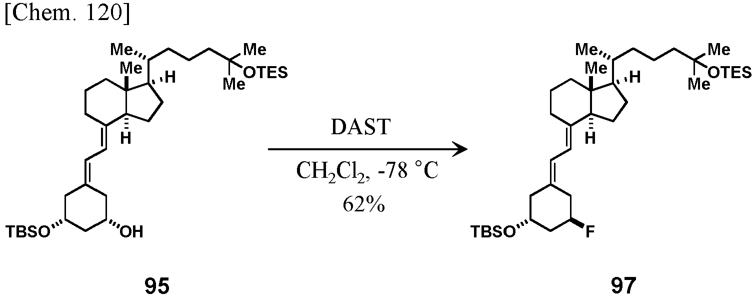

To a solution of **95** (87.2 mg, 0.138 mmol) in dichloromethane (2.8 mL) was added *N*,*N*-diethylaminosulfur trifluoride (27.3 µL, 0.207 mmol) at -78 °C. After stirring for 1 h, the reaction mixture was quenched with sat. NaHCO₃ aq. and the aqueous layer was extracted with ethyl acetate three times. The combined organic extracts were dried over MgSO₄, and concentrated *in vacuo.* The residue was chromatographed on silica gel (Hexane/ethyl acetate; 75:1 to 50:1) to give **97** (54.5 mg, 62%).

### Example 42 (Comparative Example)

### 19-nor 1α-F-3β-OH VD₃ 98

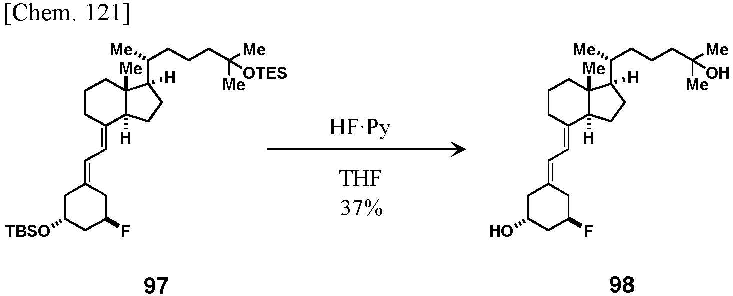

HF Py (120.3 mg, 1.213 mmol) was added to a solution of **97** (54.5 mg, 0.085 mmol) in THF (1.7 mL) and the mixture was stirred for 16 h. The reaction mixture was quenched with sat. NaHCO₃ aq. and aqueous layer was extracted with ethyl acetate three times. The combined organic extracts were washed with brine, dried over MgSO₄, and concentrated *in vacuo.* The residue was chromatographed on silica gel (Hexane/ethyl acetate; 10:1 to 1:1) to give 1α-F-3β-OH VD₃ **98** (12.9 mg, 37%) as a white solid.
**¹H NMR** (300 MHz, CDCl₃) δ 6.31 (d, *J* = 11.35 Hz, 1H), 5.80 (q, *J* = 11.35 Hz, 1H), 4.66 (ddddd, *J* = 48.16, 7.91,7.57, 3.78, 3.78 Hz, 1H), 3.77 (dddd, *J* = 7.91, 7.57, 3.78, 3.78 Hz, 1H) 2.79-1.18(m, 25H), 1.19 (s, 6H), 0.92 (d, *J* = 6.54 Hz, 3H), 0.53 (s, 3H).
HR-MS ESI Calcd for C₂₆H₄₃F₁Na₁O₂ [M+Na]⁺: 429.31448, Found: 429.31441.

### Experiment 1: PLAP-BP assay

The molecules in a chemical library which consists of 280 endogenous lipid related molecules were screened for their ability to inhibit the expression of a luciferase reporter gene. In this gene, three repeats of sterol regulatory elements (SREs) control expression of luciferase. Lipid depletion activates SREBPs, which bind to the SRE domain and work as transcription factors to express luciferase. The SREBP-responsive reporter construct was co-transfected into Chinese hamster ovary K1 (CHO-K1) cells, along with a control β-galactosidase (β-gal) reporter gene. A constitutively active actin promoter in the reporter gene drives the expression of β-gal. The levels of luciferase expression from the SREBP-responsive reporter gene were normalized to the levels of β-gal expression.

Eleven molecules that decreased the expression levels of the reporter gene by more than 40% were selected as hit molecules from the first screening. A reporter assay developed by Sakai et al. (Mol. Cell 1998, 2, 505-514) was used to determine whether the selected hit molecules affected the ER-Golgi translocation and proteolytic processing of SREBPs, and to eliminate molecules that gave false positive results. In this assay, a secreted alkaline phosphatase, fused with an SREBP-2 fragment lacking the NH₂-terminal DNA-binding domain (PLAP-BP2₅₁₃₋₁₁₄₁), was used to monitor translocation and processing, through changes in the fluorescence of a fluorogenic phosphatase substrate. When cells were co-transfected with plasmids encoding PLAP-BP2₅₁₃₋₁₁₄₁ and SREBP cleavage-activating protein (SCAP), PLAP phosphatase was secreted, and fluorescence signals were generated. Of the eleven hit molecules, vitamin D₃ (VD) derivatives caused a significant decrease in secretion, compared to the DMSO control (Fig. 1). Two molecules in particular, 25-hydroxyvitamin D₃ [25(OH)D] and 1α,25-dihydroxyvitamin D₃ [1,25(OH)₂D], caused a greater decrease in PLAP secretion than did cholesterol.

### Experiment 1-A: Procedures for cell culture

CHO-K1 cells were maintained in a medium A (1:1 mixture of Ham's F-12 medium and Dulbecco's modified Eagle medium, supplemented with 100 units/mL penicillin, 100 µg/mL streptomycin sulfate, and 5% (v/v) fetal bovine serum) at 37 °C under humidified 5% CO₂.

### Experiment 1-B: Procedures for PLAP-BP assay (Figure 1)

On day 0, CHO-K1 cells were added to 96-well plates at 2.0 × 10⁴ cells per well in medium A. On day 1, the cells were co-transfected with pCMV-PLAP-BP2₅₁₃₋₁₁₄₁ (0.1 µg/well), pCMV-SCAP (0-2.0 µg/well), and β-gal reporter, in which the expression of 0-galactosidase was controlled by an actin promoter (pAc-β-gal, 0.1 µg/well) using FuGENE(Registered trademark) HD transfection reagent (Promega), according to the manufacturer's protocol. After 5 h of incubation, the cells were washed with PBS and then incubated in medium B (1:1 mixture of Ham's F-12 medium and Dulbecco's modified Eagle medium, supplemented with 100 units/mL penicillin, 100 µg/mL streptomycin sulfate, 5% (v/v) lipid-depleted serum, 50 µM compactin, and 50 µM lithium mevalonate), in the absence or presence of VD derivatives (10 µM) or sterols (10 µg/mL of cholesterol and 1.0 µg/mL of 25-hydroxycholesterol). After 20 h of incubation, an aliquot of the medium was assayed for secreted alkaline phosphatase activity. The cells in each well were lysed and used for measurement of β-galactosidase activities. The alkaline phosphatase activity was normalized by the activity of β-galactosidase.

### Experiment 2: Luciferase reporter assay

25(OH)D inhibited the activation of SREBPs in a dose-dependent manner in the reporter assay, and had an IC₅₀ value of 1.0 µM (Fig. 2, Table 1). The inhibition of SREBP activation, mediated by the VD derivatives, was confirmed by Western blot analysis of SREBP-2 (Fig. 3). When cells were treated with 25-hydroxycholesterol [25-HC], the mature form of SREBP disappeared, and the precursor form of SREBP accumulated, indicating that 25-HC blocked the transportation of SREBP-SCAP complex from the ER to the Golgi apparatus. In contrast, treatment with 25(OH)D decreased levels of both the mature and precursor forms, suggesting that the VD derivatives reduced the levels of the precursor form of SREBP, which consequently decreased levels of the mature form, resulting in the inhibition of the SREBP activation.

### Experiment 2-A: Procedures for Luciferase reporter assay (Figure 2 and Table 1)

On day 0, CHO-K1 cells were added to 96-well plates at 1.0 × 10⁴ cells per well in medium A. On day 1, the cells were co-transfected with an SRE-1-driven luciferase reporter construct (pSRE-Luc) and pAc-β-gal at a 20/1 ratio, using FuGENE(Registered trademark) HD transfection reagent (Promega), according to the manufacturer's protocol. After 8-12 h of incubation, the cells were washed with PBS buffer, then incubated in medium B containing the specific test compounds. Stock solutions of each compound in DMSO were prepared and added to medium B to give a 200-fold (v/v) dilution (0.5% DMSO). After 20-24 h of incubation, the cells in each well were lysed by freeze-thaw with Reporter Lysis Buffer (Promega), and aliquots were used to measure luciferase and β-galactosidase activities. Luciferase activity was measured using the Steady-Glo(Registered trademark) Luciferase Assay System (Promega), and β-galactosidase activity was measured using the β-galactosidase Enzyme Assay System (Promega). Luciferase activity was normalized to β-galactosidase activity.

### Experiment 2-B: Procedures for Western-blot analysis (Figures 3, 4, 5, 6, 7, and 8)

On day 0, CHO-K1 cells were added to 6-well plates at 3.0 × 10⁵ cells per well in medium A. On day 1, the cells were washed with PBS, and then incubated in medium B in the absence or presence of specific test compounds (5 µM). After 16 h of incubation, the cells were washed three times with cold PBS, and lysed with a buffer A (50 mM Tris-HCl (pH 7.5), 150 mM NaCl, 1% (v/v) Nonidet P40, 0.5% (w/v) sodium deoxycholate, 8 M urea, and protease inhibitor cocktail (Nacalai)). The cell lysate were passed 16 times through a 25G needle and centrifuged at 7,000 g at 4 °C for 10 min. The supernatants were transferred to new tubes, and the pellets were extracted with a buffer A. The resulting buffer was centrifuged at 7,000 g at 4 °C for 10 min, and the supernatants were combined to the previous ones. The resulting lysate was mixed with 0.20 volume of 6× SDS sample buffer (Nacalai) and incubated at room temperature for 30 min. The samples were separated on a 10% SDS-PAGE gel and blotted, using mouse monoclonal antibodies against SREBP-2 (IgG-7D4), SCAP (IgG-9D5), c-Myc (IgG1-MC045), and actin (AC-40). The specific bands were visualized using enhanced chemiluminescence (ECL Prime Western Blotting Detection Reagent, GE Healthcare) on an ImageQuant LAS 500 (GE Healthcare).

### SREBP inhibitory mechanism

The effect of 25(OH)D on the levels of SCAP was also confirmed by Western blot analysis (Fig. 4). Treatment of cells with 25(OH)D caused reduced the levels of SCAP as well as that of SREBP. Furthermore, the decrease in the levels of SCAP, mediated by 25(OH)D, was cancelled by addition of MG-132, a general proteasome inhibitor (Fig. 5), and 25(OH)D accelerated the ubiquitination of SCAP (Fig. 6). When SREBP is not able to form a complex with SCAP, the precursor form of SREBP is rapidly degraded (R. B. Rawson, et al. J. Biol. Chem. 1999, 274, 28549.). These results suggested that 25(OH)D stimulates the ubiquitin-proteasome degradation of SCAP, resulting in the decrease in the levels of the precursor form of SREBP.

Despite the discovery of the SREBP inhibitory mechanism of hydroxylated VD derivatives, these endogenous molecules per se cannot be used for specific pharmacological intervention of SREBP due to their well-established roles in calcium homeostasis. For example, overdose administration 25(OH)D, which is converted to an active VDR agonist, 1,25(OH)₂D, increases serum calcium concentrations, often resulting in formation of kidney stones. One of possible ways to eliminate its VDR activity is to prevent metabolic hydroxylation of the C1 position. Example compounds **18a, 22a, 22b, 28, 32, 36, 38, 39** and **106** are unlikely to be VDR agonists or converted to VDR agonists.

Reporter assays showed that these Example compounds inhibited activation of SREBPs, with IC₅₀ values ranging from 0.5 to 16 µM (Table 1). The inhibition of SREBP activation mediated by Example compounds **22a, 22b, 28, 32, 36, 38,** and **39** (Fig. 7) and Example compounds **72-74, 76, 77, 85-87, 89-91,** and **98** (Fig. 8) was confirmed by Western-blot analysis of SREBP-2. The treatment of cells with the derivatives decreased the levels of the mature form of SREBP relative to treatment with DMSO.

### Table 1. Inhibitory effects of 1-N VD derivatives on the activation of SREBP.

**[Table 1]**

| **Compounds** | IC₅₀ (µM)*^{a}* |
|---|---|
| **25(OH)D** | 1.09 |
| **1,25(OH)₂D** | 1 |
| **36** | 1.38 |
| **18a*** | 0.82 |
| **22a** | 3.24 |
| **39** | 0.97 |
| **38** | 1.76 |
| **32** | 0.61 |
| **28** | 0.68 |
| **49** | 16 |
| **22b** | 3.56 |
| **72** | 1.69 |
| **73** | 2.71 |
| **74** | 1.11 |
| **76** | 2.05 |
| **77** | 1.55 |
| **85** | 0.52 |
| **86** | 0.53 |
| **87** | 0.52 |
| **89** | 3.34 |
| **90** | 0.83 |
| **91** | 1.17 |
| **98*** | 1.59 |

| | |
|---|---|
| *^{a}* All values are the averages of three independent experiments. * Comparative compounds | |

### [Industrial Applicability]

The compound of Formula (I) or a pharmaceutically acceptable salt thereof may be useful for treating a disease such as metabolic disease including non-alcoholic steatohepatitis (NASH), liver disease including fatty liver, diabetes, cancer, obesity, cardiovascular disease, etc.

## Claims

1. A compound of the following general formula (I): 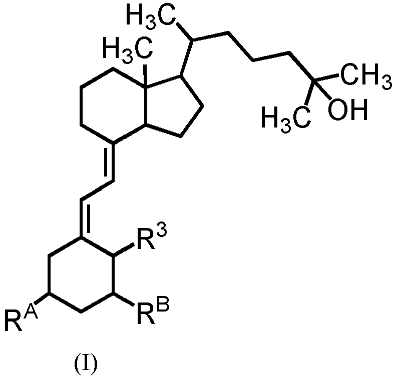 wherein one of R^{A} and R^{B} is hydroxyl and the other is NR¹R²;
R¹ and R² are each independently selected from hydrogen; C₁₋₄ alkyl; C₁₋₄ alkylcarbonyl optionally substituted with at least one halogen which are the same or different; C₁₋₄ alkylsulfonyl; benzyloxycarbonyl; 3 to 6-membered cycloalkyl-C₁₋₄ alkyl; C₆₋₁₀ arylcarbonyl optionally substituted with at least one group independently selected from the group consisting of halogen, halo-C₁₋₄ alkyl, -S-halo-C₁₋₄ alkyl, C₁₋₄ alkoxy, halo-C₁₋₄ alkoxy, nitro, cyano, C₁₋₄ alkoxycarbonyl and C₆₋₁₀ aryl; C₆₋₁₀ arylsulfonyl optionally substituted with at least one group independently selected from the group consisting of C₁₋₄ alkyl, nitro, and di-(C₁₋₄ alkyl)amino; 5 to 6-membered saturated heterocyclyl-C₁₋₄ alkyl optionally substituted with at least one group independently selected from the group consisting of halogen and hydroxyl; 5 to 6-membered heteroaryl; and a group of the following formula: 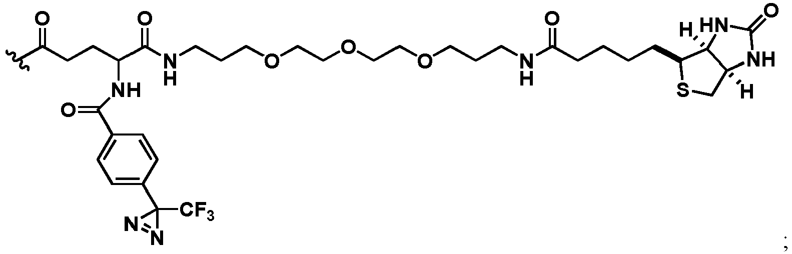 or
R¹ and R² may optionally combine together with the nitrogen atom to which they attach to form a nitrogen-containing oxo-substituted saturated 5 to 6-membered heterocyclic ring which may be optionally fused with a C₆₋₁₀ aryl ring; and
R³ is hydrogen or =CH₂; or a pharmaceutically acceptable salt thereof;
provided that R¹ and R² are not concurrently hydrogen.

2. The compound of claim 1, wherein R³ is =CH₂, or a pharmaceutically acceptable salt thereof.

3. The compound of claim 1, wherein R³ is hydrogen, or a pharmaceutically acceptable salt thereof.

4. The compound of any one of claims 1 to 3, having any one of the following formulae: 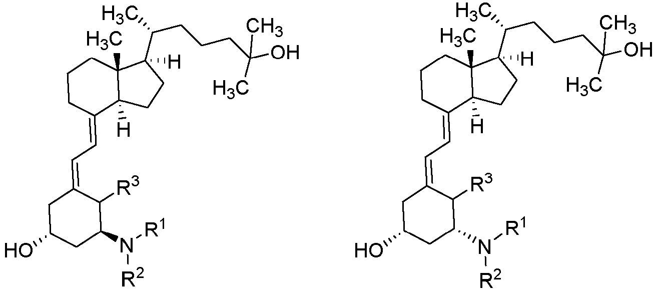 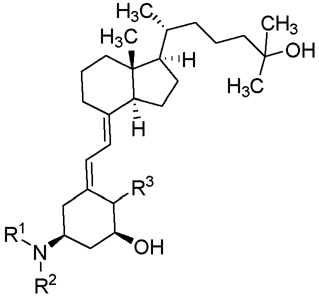 wherein R¹ and R² are as defined in claim 1, and R³ is as defined in any one of claims 1 to 3.

5. The compound of any one of claims 1 to 4, wherein R² is hydrogen, or a pharmaceutically acceptable salt thereof.

6. The compound of claim 1, having a structure:

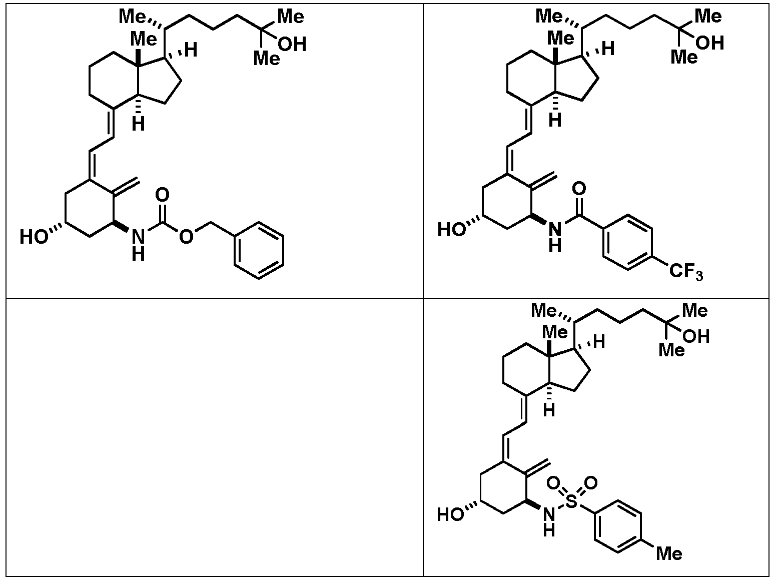

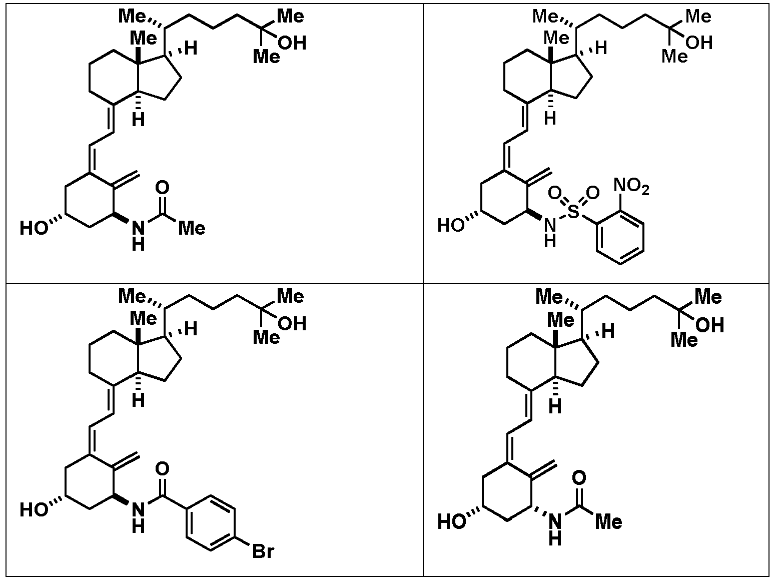

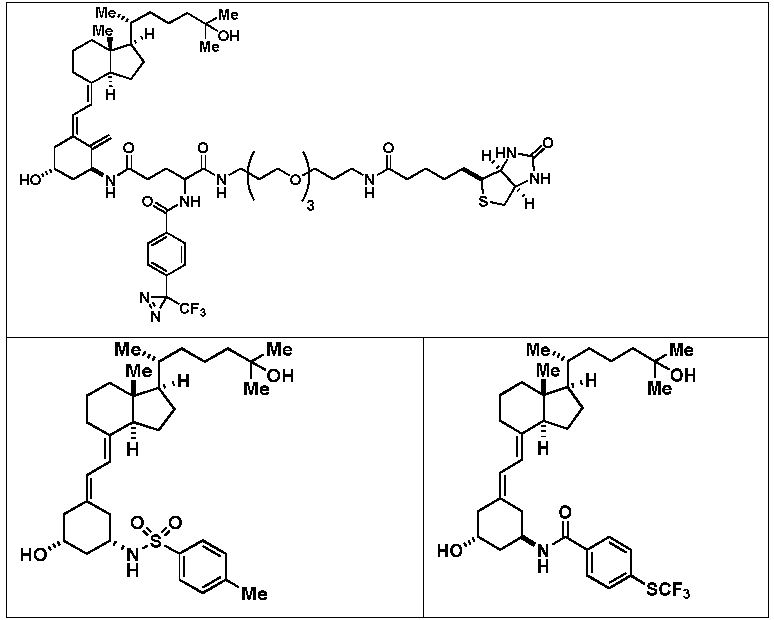

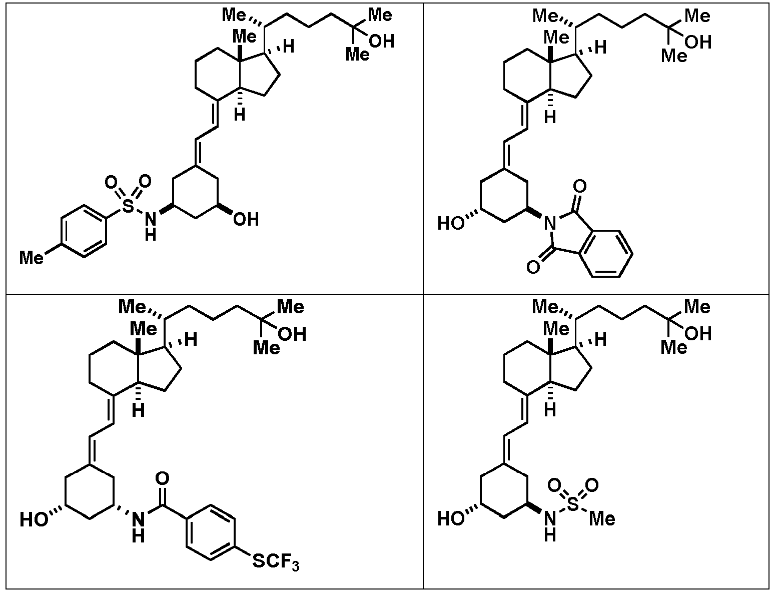

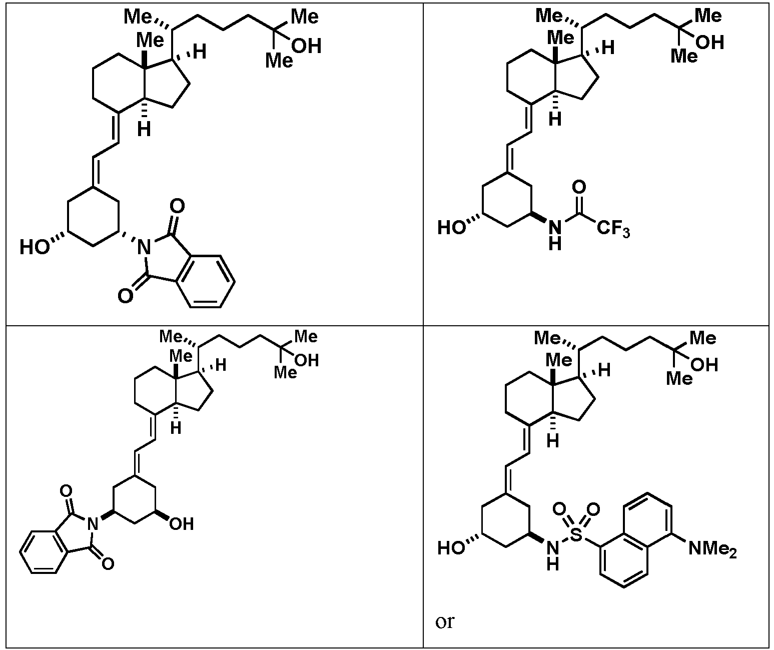

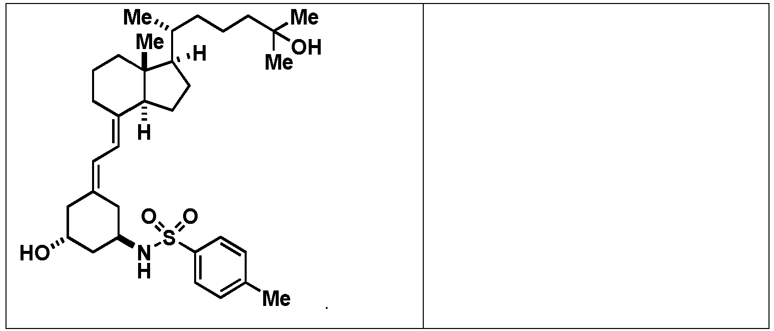

7. A pharmaceutical composition, comprising as the active ingredient the compound of any one of claims 1 to 6 or a pharmaceutically acceptable salt thereof and a pharmaceutically acceptable carrier.

8. A compound of any one of claims 1 to 6 or a pharmaceutically acceptable salt thereof, or a pharmaceutical composition of claim 7, for use in treating a metabolic disease, liver disease, obesity, diabetes, cardiovascular disease, or cancer in a subject.

9. The compound or a pharmaceutically acceptable salt thereof, or the pharmaceutical composition for use of claim 8, wherein the disease is obesity, non-alcoholic steatohepatitis (NASH), fatty liver, or cancer.

10. The compound or a pharmaceutically acceptable salt thereof, or the pharmaceutical composition for use of claim 8 or 9, wherein the compound or the compound comprised in the pharmaceutical composition is selected from

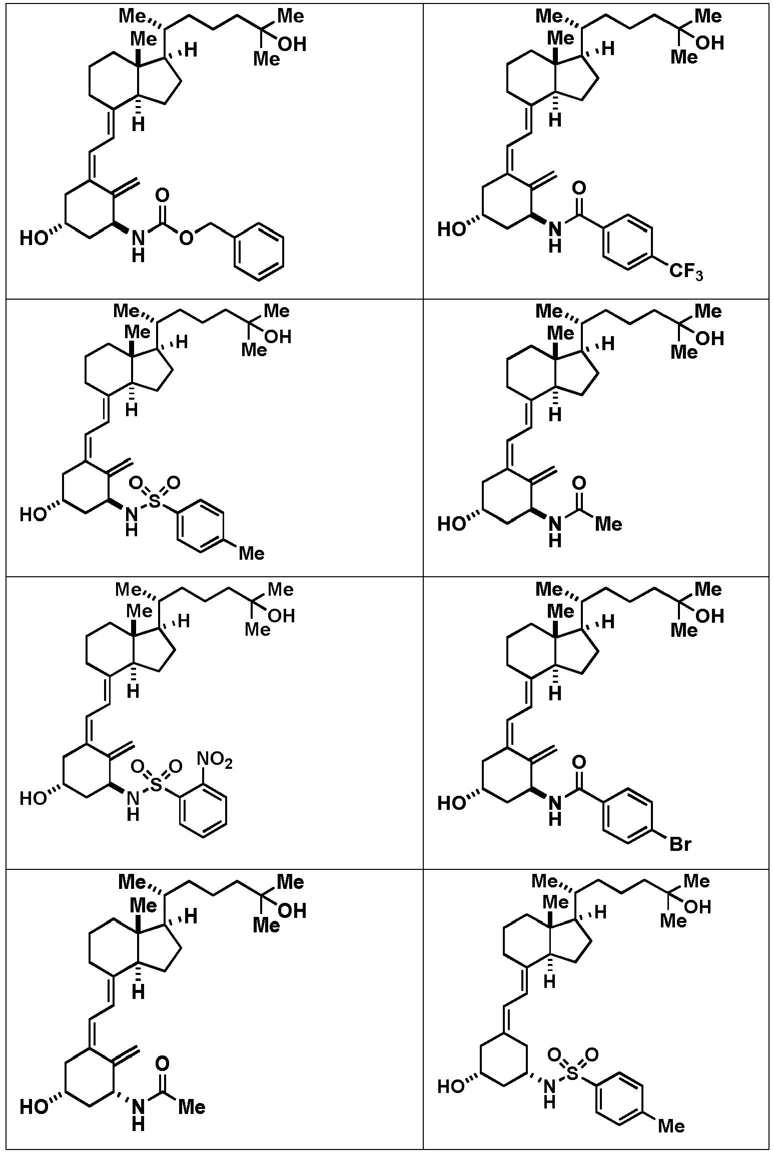

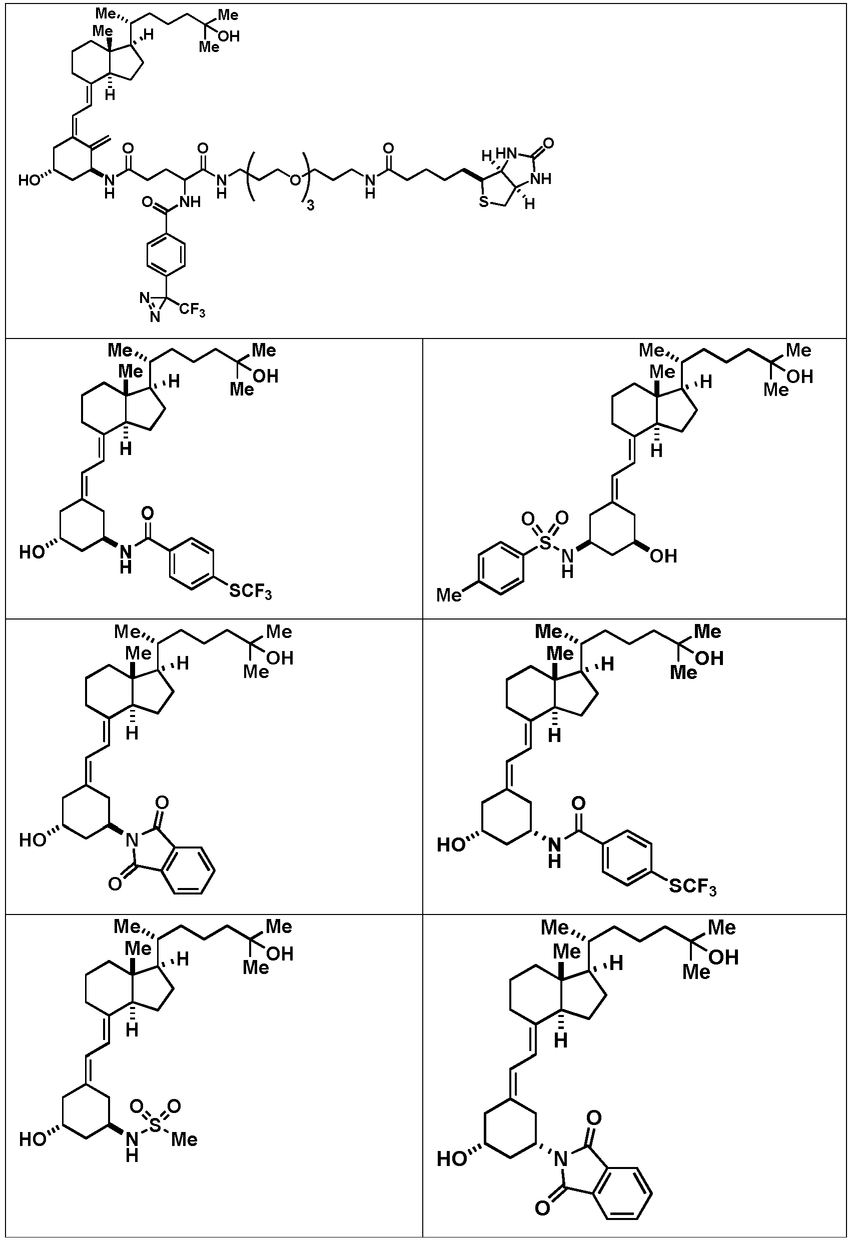

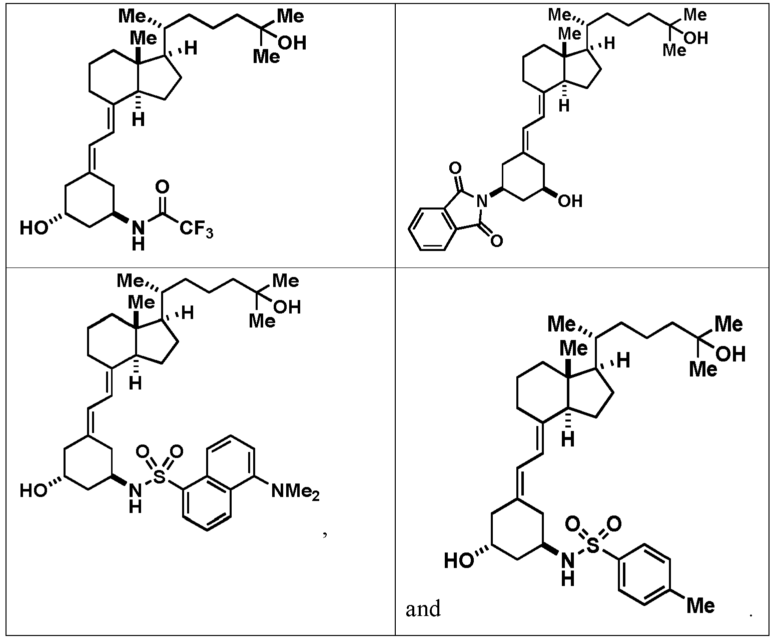

11. A compound of any one of claims 1 to 6 or a pharmaceutically acceptable salt thereof for use in inhibiting an SREBP in a subject.

12. The compound or a pharmaceutically acceptable salt thereof for use of claim 11, wherein the SREBP inhibition is for treating a metabolic disease, liver disease, obesity, diabetes, cardiovascular disease, or cancer in the subject.

13. The compound or a pharmaceutically acceptable salt thereof for use of claim 12, wherein the SREBP inhibition is for treating obesity, non-alcoholic steatohepatitis (NASH), fatty liver, or cancer in the subject.

## Patentansprüche

1. Verbindung der folgenden allgemeinen Formel (I): 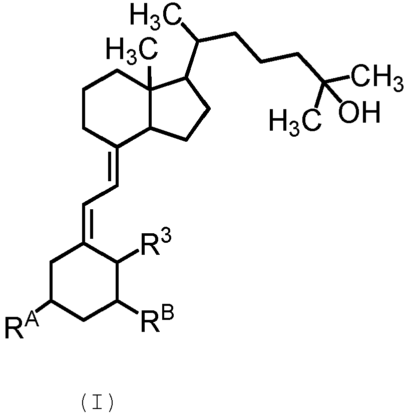
worin einer der Reste R^{A} und R^{B} Hydroxyl ist und der andere NR¹R² ist;
R¹ und R² jeweils unabhängig voneinander aus Wasserstoff; C₁₋₄-Alkyl; C₁₋₄-Alkylcarbonyl, optional substituiert mit mindestens einem Halogen, die gleich oder verschieden sind; C₁₋₄-Alkylsulfonyl; Benzyloxycarbonyl; 3- bis 6-gliedrigem Cycloalkyl-C₁₋₄-alkyl; C₆₋₁₀-Arylcarbonyl, optional substituiert mit mindestens einer Gruppe, unabhängig voneinander ausgewählt aus der Gruppe, bestehend aus Halogen, Halogen-C₁₋₄-alkyl, -S-Halogen-C₁₋₄-alkyl, C₁₋₄-Alkoxy, Halogen-C₁₋₄-alkoxy, Nitro, Cyano, C₁₋₄-Alkoxycarbonyl und C₆₋₁₀-Aryl; C₆₋₁₀-Arylsulfonyl, optional substituiert mit mindestens einer Gruppe, unabhängig voneinander ausgewählt aus der Gruppe, bestehend aus C₁₋₄-Alkyl, Nitro und Di-(C₁₋₄-alkyl)amino; 5- bis 6-gliedrigem gesättigtem Heterocyclyl-C₁₋₄-alkyl, optional substituiert mit mindestens einer Gruppe, unabhängig voneinander ausgewählt aus der Gruppe, bestehend aus Halogen und Hydroxyl; 5- bis 6-gliedrigem Heteroaryl;
und einer Gruppe der folgenden Formel: 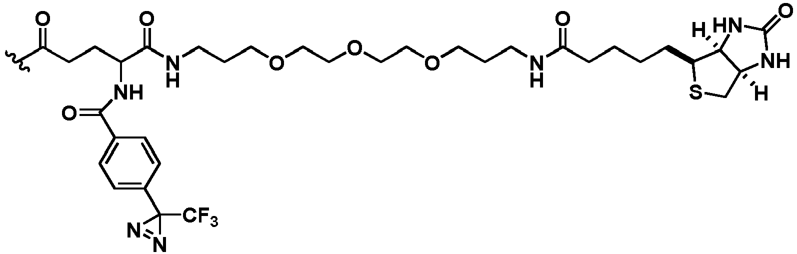
ausgewählt sind; oder
R¹ und R² optional zusammen mit dem Stickstoffatom, an das sie gebunden sind, unter Bildung eines stickstoffhaltigen oxosubstituierten gesättigten 5- bis 6-gliedrigen heterocyclischen Rings, der optional mit einem C₆₋₁₀-Arylring kondensiert sein kann, kombiniert sein können; und
R³ Wasserstoff oder =CH₂ ist; oder ein pharmazeutisch verträgliches Salz davon;
mit der Maßgabe, dass R¹ und R² nicht gleichzeitig Wasserstoff sind.

2. Verbindung gemäß Anspruch 1, wobei R³ =CH₂ ist, oder ein pharmazeutisch verträgliches Salz davon.

3. Verbindung gemäß Anspruch 1, wobei R³ Wasserstoff ist, oder ein pharmazeutisch verträgliches Salz davon.

4. Verbindung gemäß einem der Ansprüche 1 bis 3, die eine der folgenden Formeln aufweist: 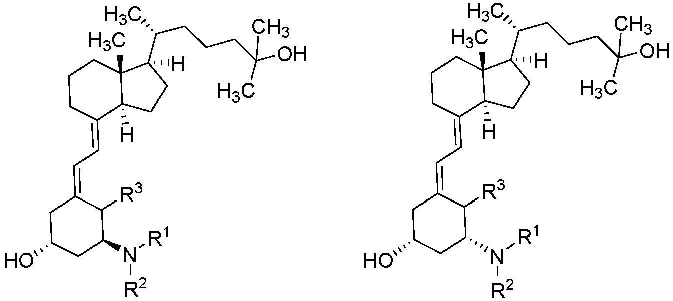 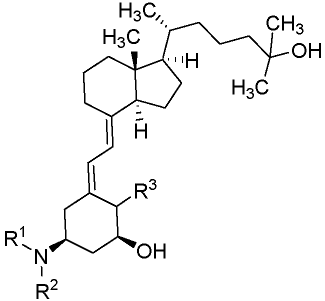 worin R¹ und R² wie in Anspruch 1 definiert sind und R³ wie in einem der Ansprüche 1 bis 3 definiert ist.

5. Verbindung gemäß einem der Ansprüche 1 bis 4, wobei R² Wasserstoff ist, oder ein pharmazeutisch verträgliches Salz davon.

6. Verbindung gemäß Anspruch 1 mit einer Struktur:

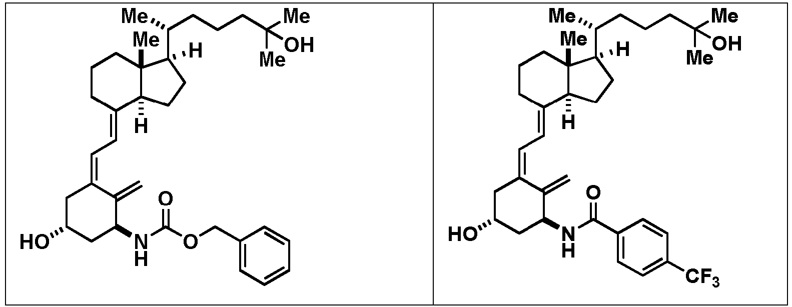

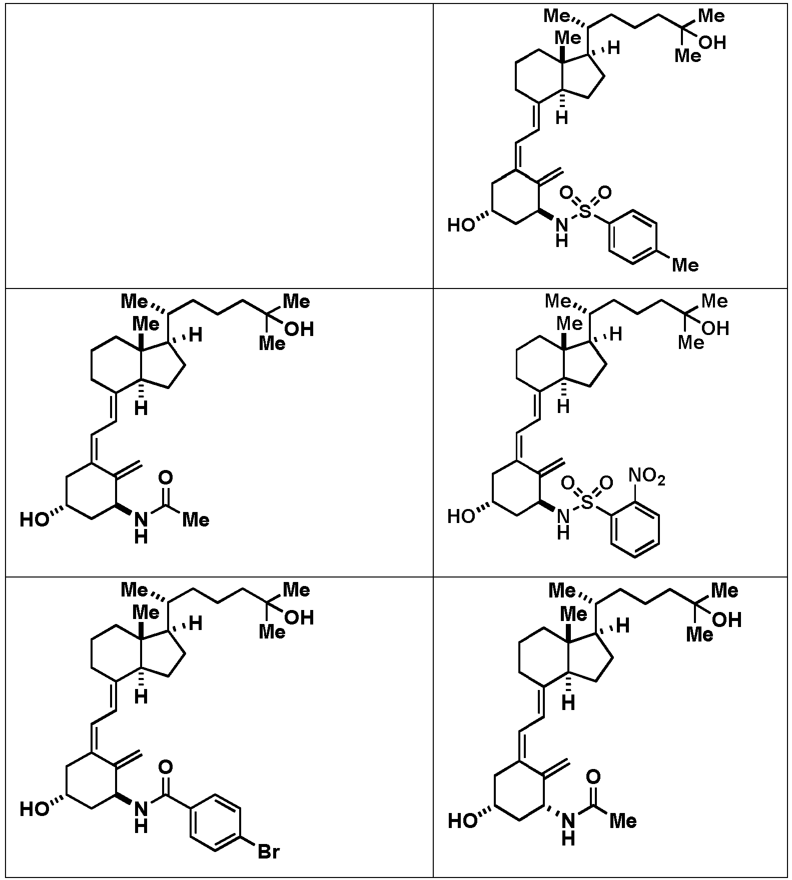

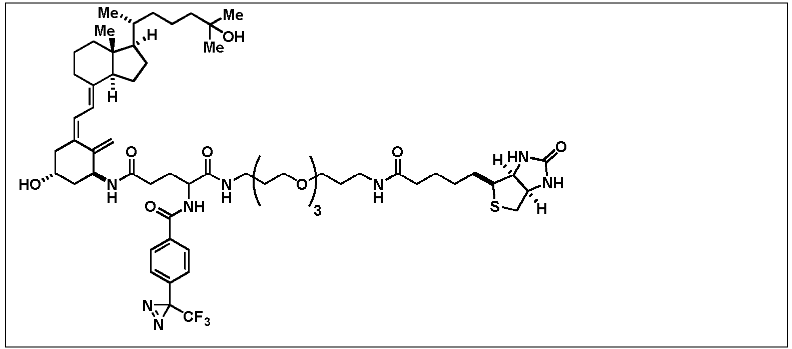

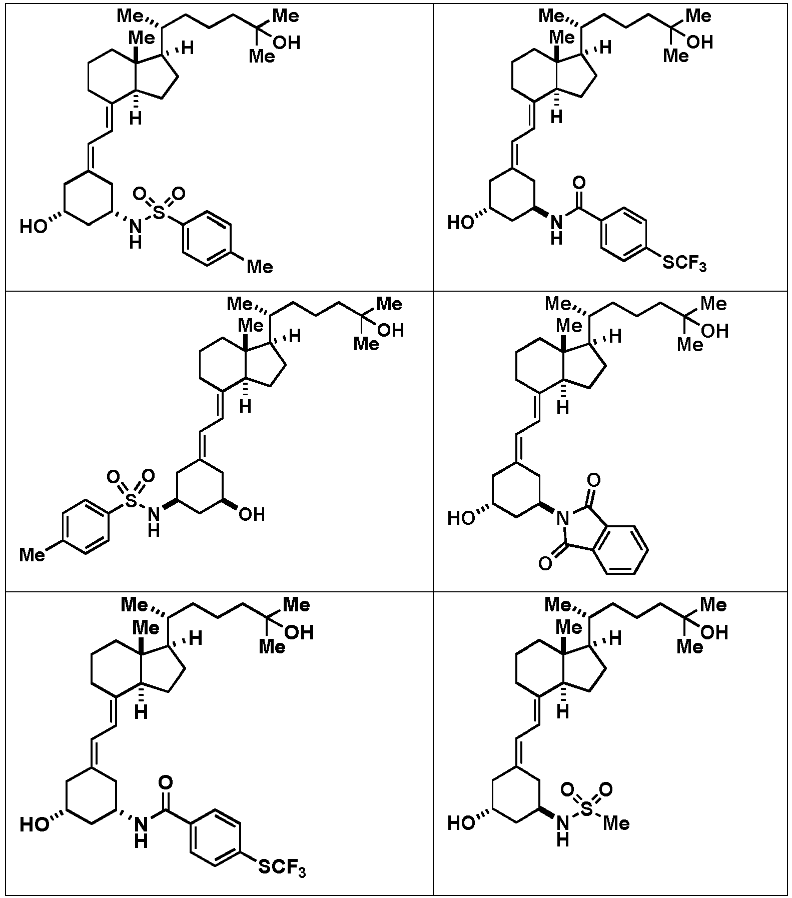

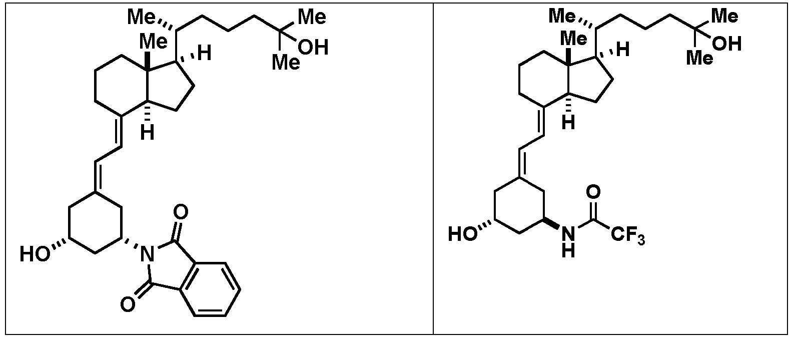

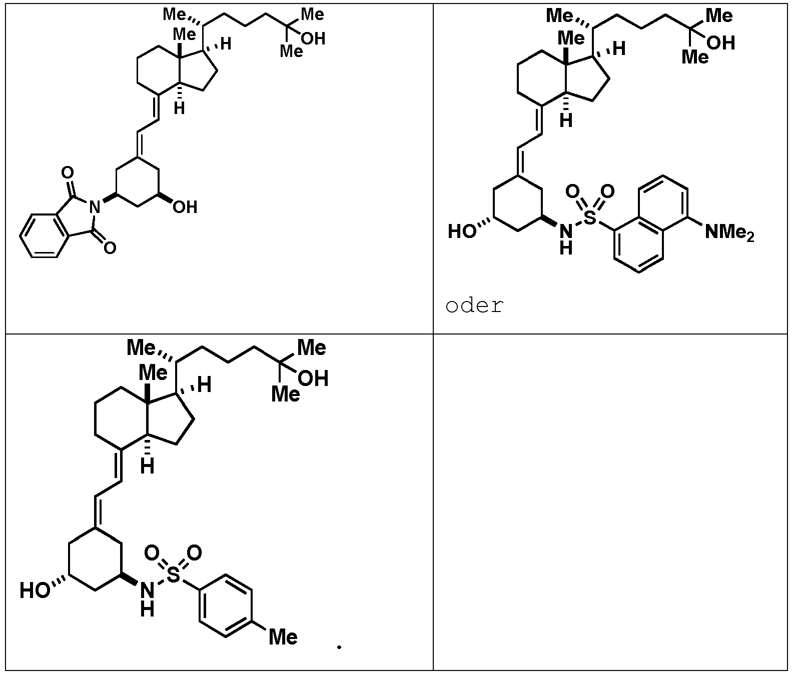

7. Pharmazeutische Zusammensetzung, umfassend die Verbindung gemäß einem der Ansprüche 1 bis 6 oder ein pharmazeutisch verträgliches Salz davon als Wirkstoff und einen pharmazeutisch verträglichen Träger.

8. Verbindung gemäß einem der Ansprüche 1 bis 6 oder ein pharmazeutisch verträgliches Salz davon oder eine pharmazeutische Zusammensetzung gemäß Anspruch 7 zur Verwendung bei der Behandlung von einer Stoffwechselkrankheit, einer Leberkrankheit, Fettleibigkeit, Diabetes, einer Herz-Kreislauf-Krankheit oder Krebs bei einem Subjekt.

9. Verbindung oder ein pharmazeutisch verträgliches Salz davon oder die pharmazeutische Zusammensetzung zur Verwendung gemäß Anspruch 8, wobei die Krankheit Fettleibigkeit, nichtalkoholische Steatohepatitis (NASH), Fettleber oder Krebs ist.

10. Verbindung oder ein pharmazeutisch verträgliches Salz davon oder die pharmazeutische Zusammensetzung zur Verwendung gemäß Anspruch 8 oder 9, wobei die Verbindung oder die in der pharmazeutischen Zusammensetzung enthaltene Verbindung aus 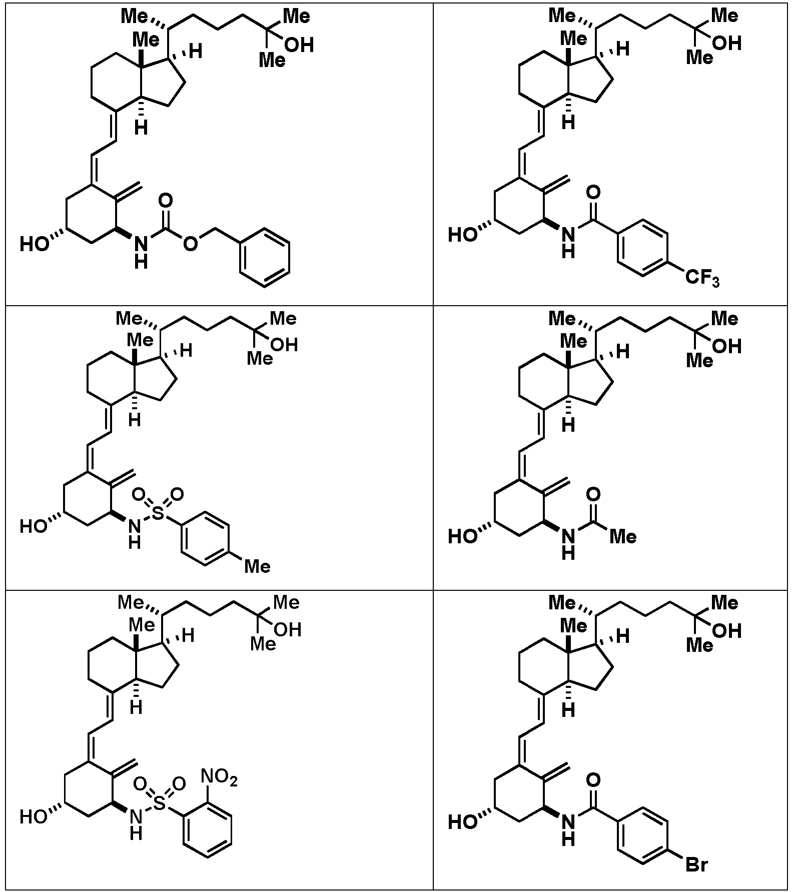 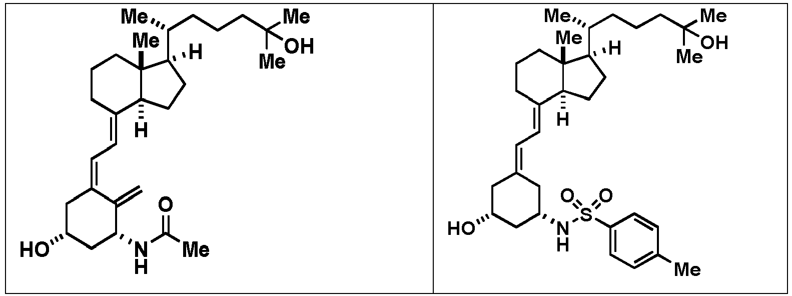 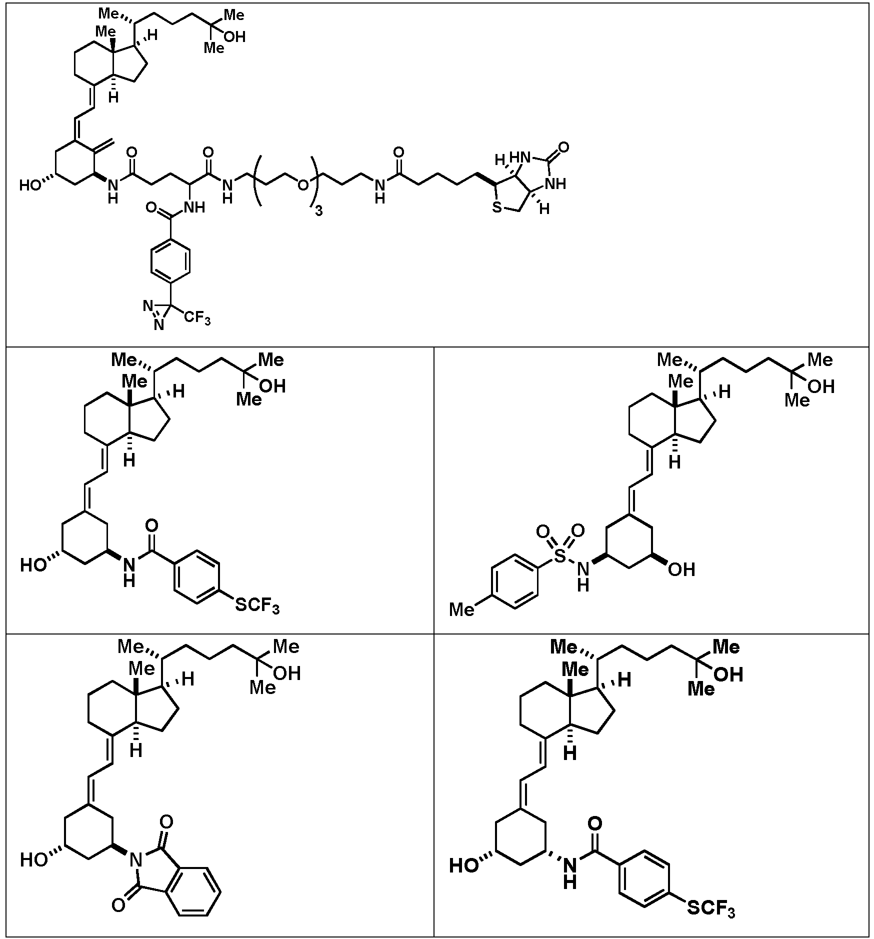 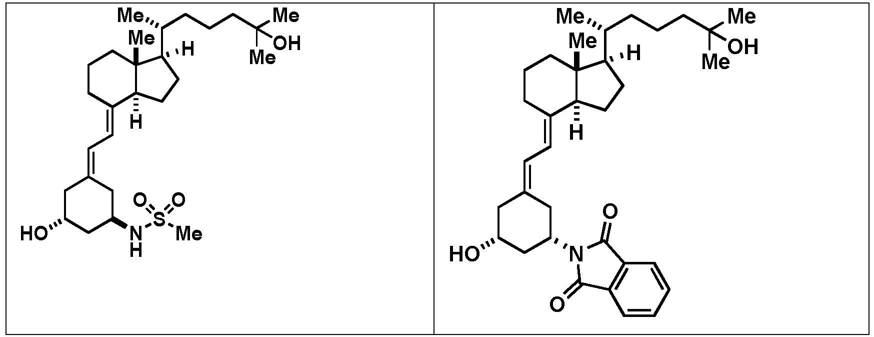 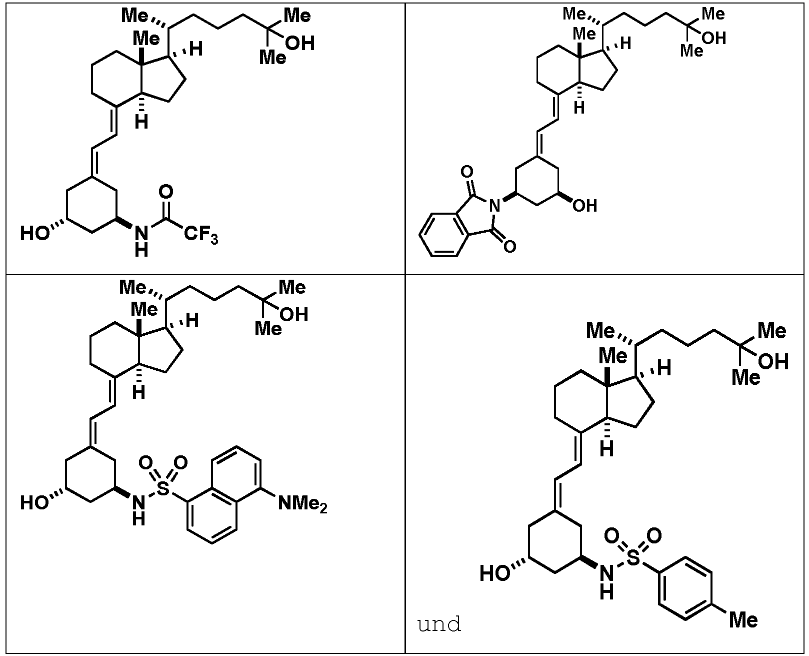 ausgewählt ist.

11. Verbindung gemäß einem der Ansprüche 1 bis 6 oder ein pharmazeutisch verträgliches Salz davon zur Verwendung bei der Hemmung eines SREBP in einem Subjekt.

12. Verbindung oder ein pharmazeutisch verträgliches Salz davon zur Verwendung gemäß Anspruch 11, wobei die SREBP-Hemmung zur Behandlung von einer Stoffwechselkrankheit, einer Leberkrankheit, Fettleibigkeit, Diabetes, einer Herz-Kreislauf-Krankheit oder Krebs bei dem Subjekt ist.

13. Verbindung oder ein pharmazeutisch verträgliches Salz davon zur Verwendung gemäß Anspruch 12, wobei die SREBP-Hemmung zur Behandlung von Fettleibigkeit, nichtalkoholischer Steatohepatitis (NASH), Fettleber oder Krebs bei dem Subjekt ist.

## Revendications

1. Composé de la formule générale (I) suivante : 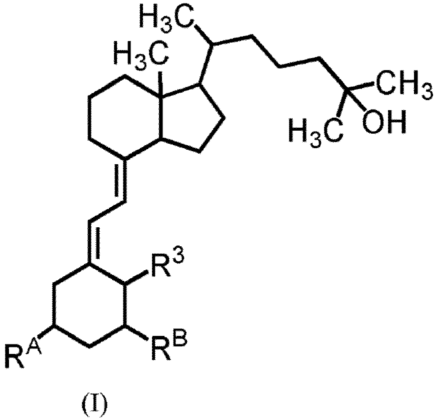 dans lequel un élément parmi R^{A} et de R^{B} est un hydroxyle et l'autre élément est NR¹R² ;
R¹ et R² sont chacun indépendamment sélectionnés parmi un hydrogène ; un alkyle en C₁₋₄ ; un alkylcarbonyle en C₁₋₄ facultativement substitué par au moins un halogène qui est identique ou différent ; un alkylsulfonyle en C₁₋₄ ; un benzyloxycarbonyle ; un cycloalkyl-alkyle en C₁₋₄ à 3 à 6 éléments ; un arylcarbonyle en C₆₋₁₀ facultativement substitué par au moins un groupe indépendamment sélectionné parmi le groupe consistant en un halogène, un halo-alkyle en C₁₋₄, un -S-halo-alkyle en C₁₋₄, un alcoxy en C₁₋₄, un halo-alcoxy en C₁₋₄, un nitro, un cyano, un alcoxycarbonyle en C₁₋₄ et un aryle en C₆₋₁₀ ; un arylsulfonyle en C₆₋₁₀ facultativement substitué par au moins un groupe indépendamment sélectionné parmi le groupe consistant en un alkyle en C₁₋₄, un nitro, et un di-(alkyle en C₁₋₄)amino ; un hétérocyclyl-alkyle en C₁₋₄ saturé à 5 à 6 éléments facultativement substitué par au moins un groupe indépendamment sélectionné parmi le groupe consistant en un halogène et un hydroxyle ; un hétéoaryle à 5 à 6 éléments ; et un groupe de la formule suivante : 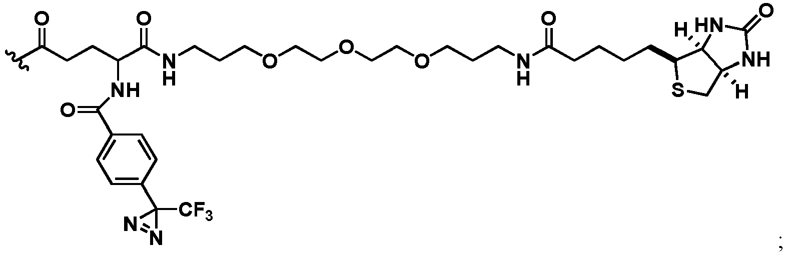 ou
R¹ et R² peuvent facultativement se combiner conjointement avec l'atome d'azote auquel ils sont attachés pour former un cycle hétérocyclique à 5 ou 6 éléments saturé oxo-substitué contenant un azote qui peut être facultativement fusionné avec un cycle aryle en C₆₋₁₀ ; et
R³ est un hydrogène ou =CH₂ ; ou un sel pharmaceutiquement acceptable de celui-ci ;
à condition que R¹ et R² ne soient pas simultanément un hydrogène.

2. Composé selon la revendication 1, dans lequel R³ est =CH₂, ou un sel pharmaceutiquement acceptable de celui-ci.

3. Composé selon la revendication 1, dans lequel R³ est un hydrogène, ou un sel pharmaceutiquement acceptable de celui-ci.

4. Composé selon l'une quelconque des revendications 1 à 3, présentant l'une quelconque des formules suivantes : 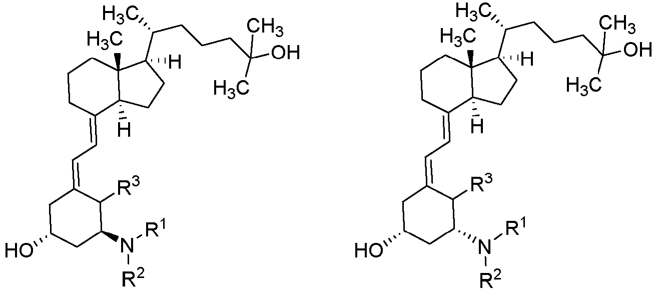 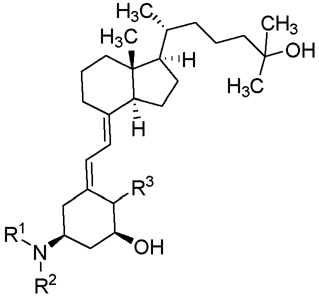 dans lequel R¹ et R² sont tels que définis dans la revendication 1, et R³ est tel que défini dans l'une quelconque des revendications 1 à 3.

5. Composé selon l'une quelconque des revendications 1 à 4, dans lequel R² est un hydrogène, ou un sel pharmaceutiquement acceptable de celui-ci.

6. Composé selon la revendication 1, présentant une structure :

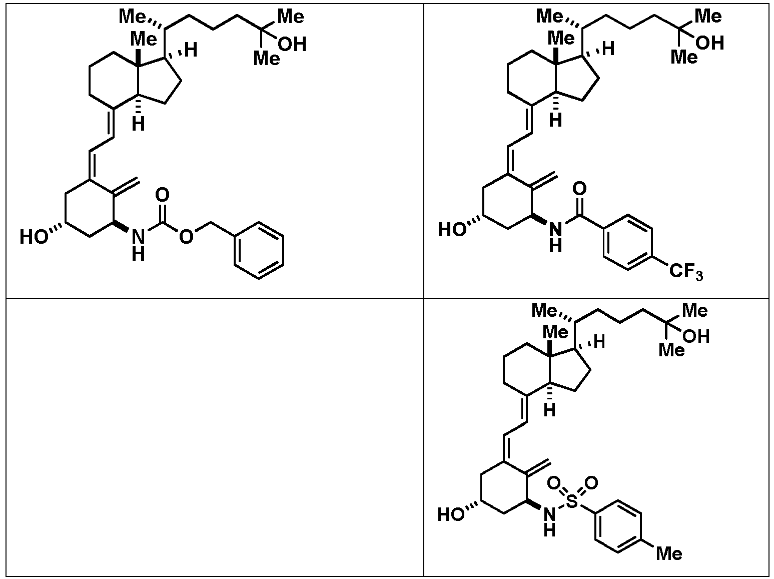

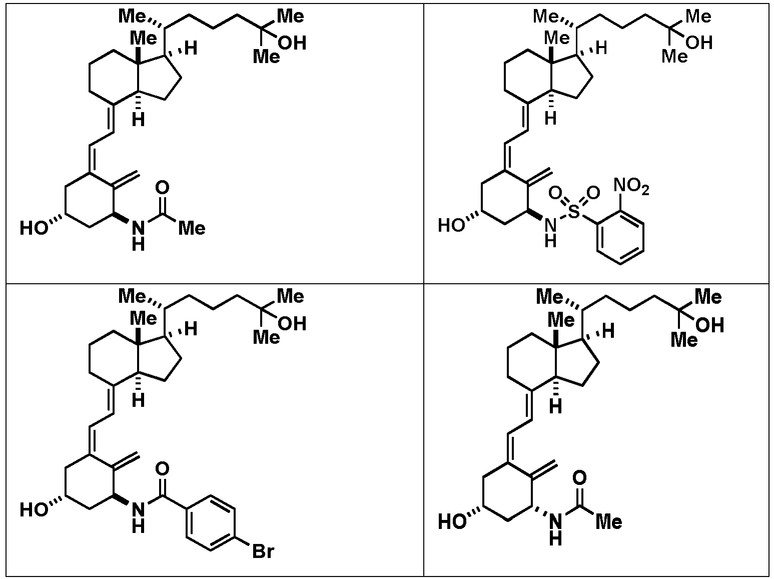

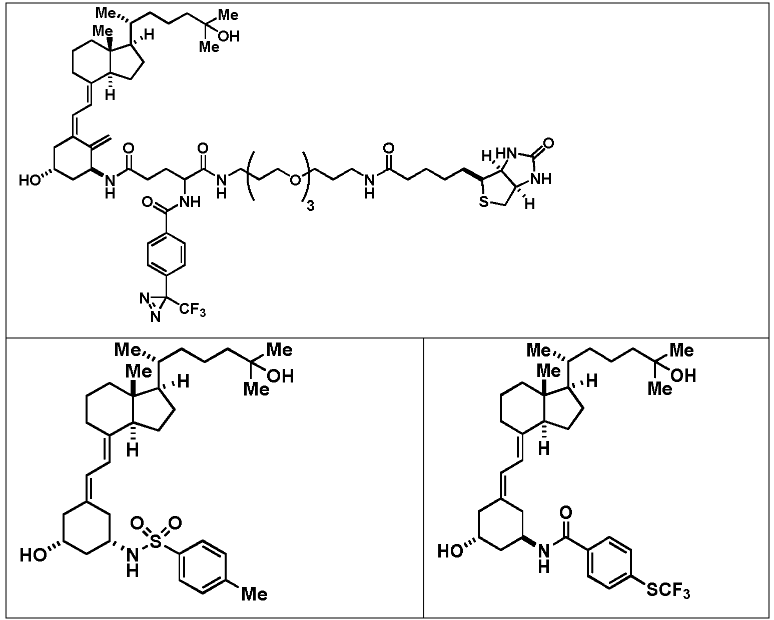

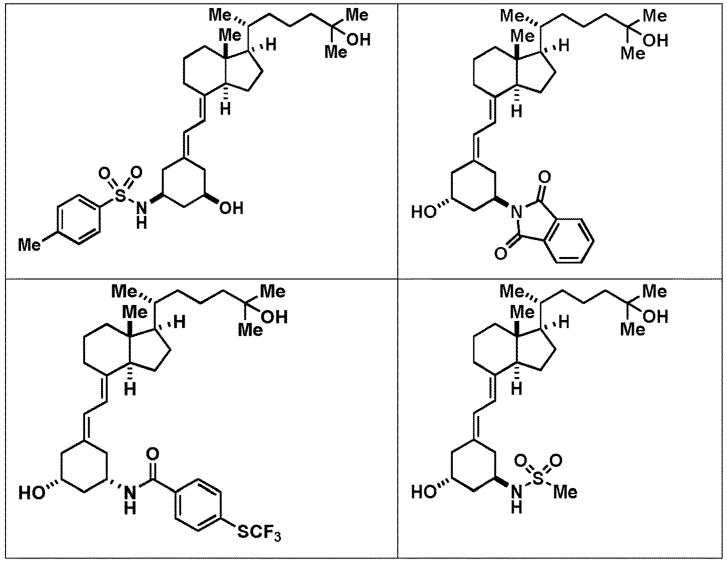

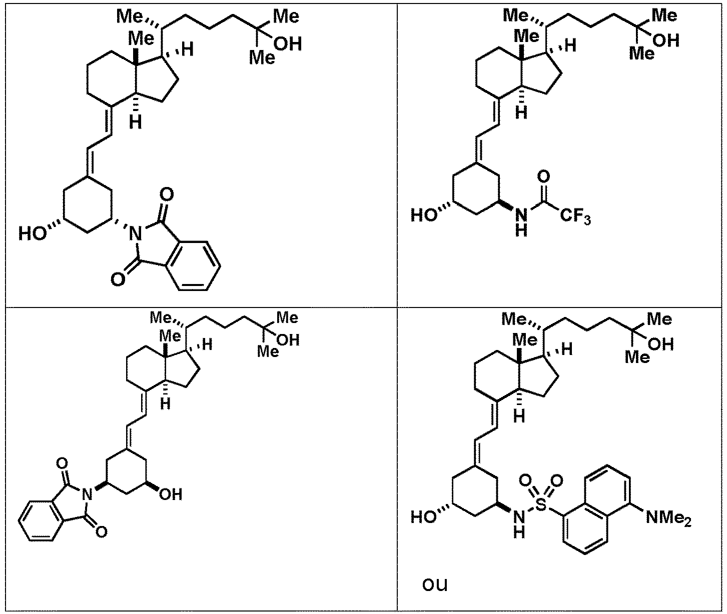

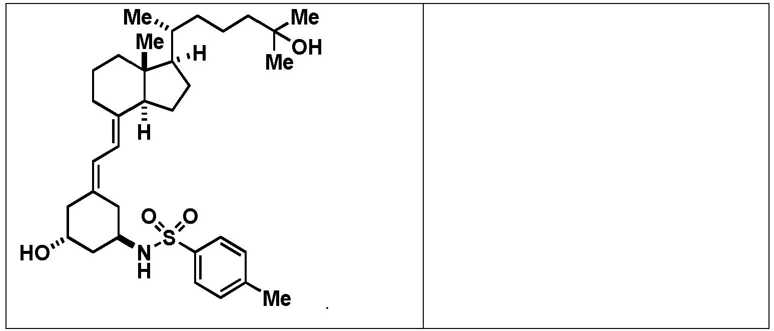

7. Composition pharmaceutique, comprenant en tant que le principe actif le composé selon l'une quelconque des revendications 1 à 6 ou un sel pharmaceutiquement acceptable de celui-ci et un vecteur pharmaceutiquement acceptable.

8. Composé selon l'une quelconque des revendications 1 à 6 ou sel pharmaceutiquement acceptable de celui-ci, ou composition pharmaceutique selon la revendication 7, pour utilisation dans un traitement d'une maladie métabolique, d'une maladie hépatique, d'une obésité, d'un diabète, d'une maladie cardiovasculaire, ou d'un cancer chez un sujet.

9. Composé ou sel pharmaceutiquement acceptable de celui-ci, ou composition pharmaceutique pour utilisation selon la revendication 8, dans lequel ou laquelle la maladie est une obésité, une stéatohépatite non alcoolique (NASH), une stéatose hépatique, ou un cancer.

10. Composé ou sel pharmaceutiquement acceptable de celui-ci, ou composition pharmaceutique pour utilisation selon la revendication 8 ou 9, dans lequel ou laquelle le composé ou le composé compris dans la composition pharmaceutique est sélectionné parmi

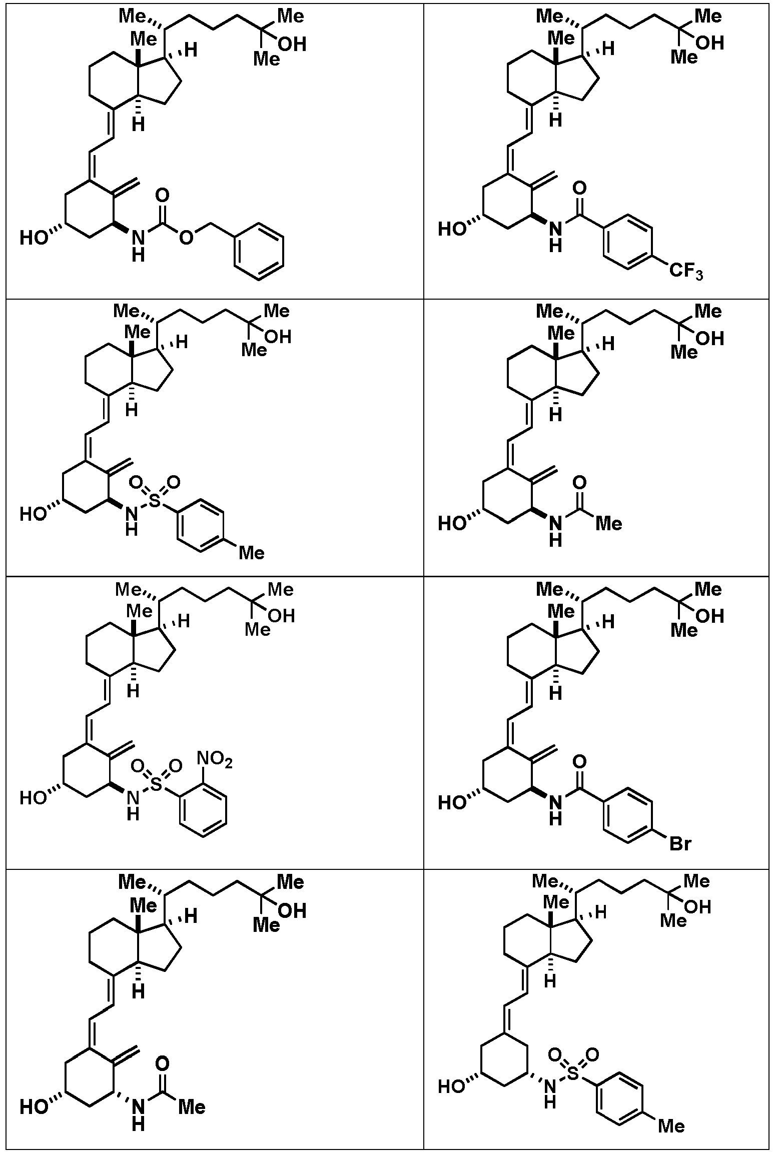

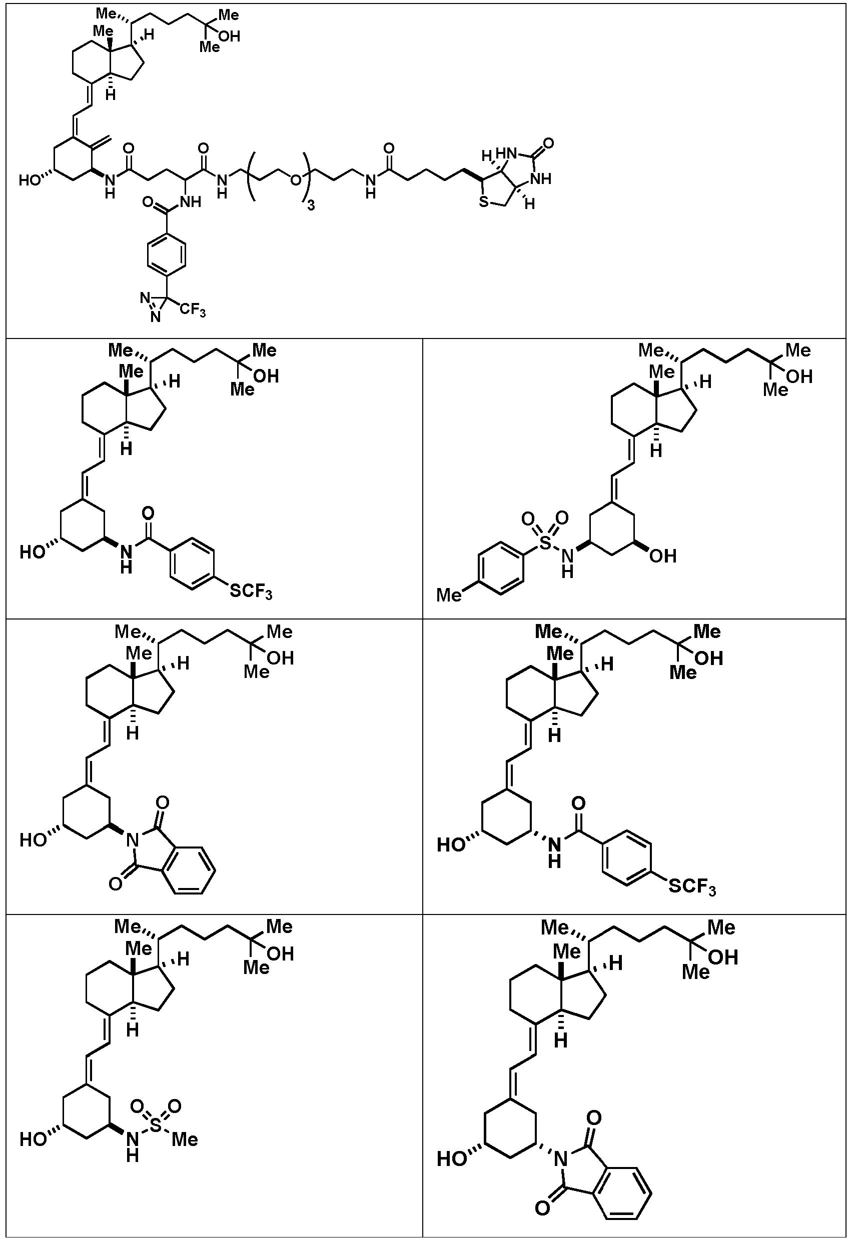

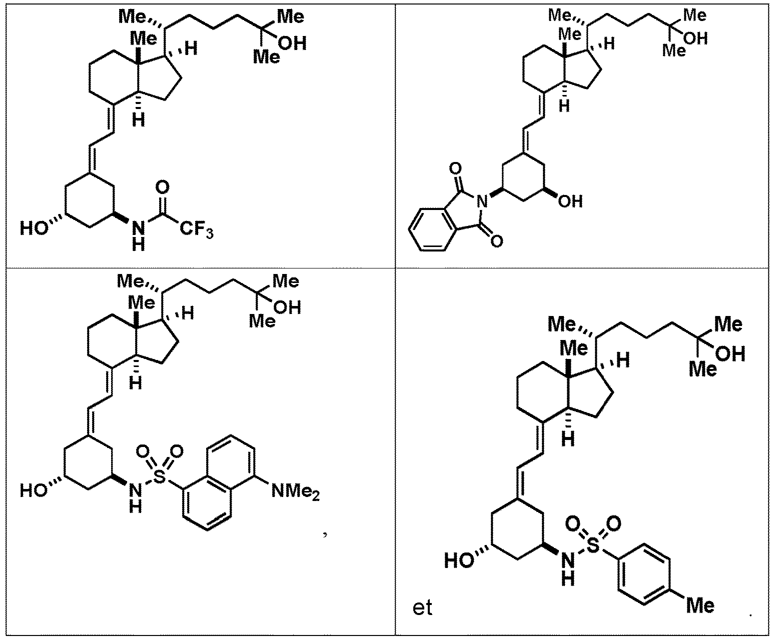

11. Composé selon l'une quelconque des revendications 1 à 6 ou sel pharmaceutiquement acceptable de celui-ci pour utilisation dans l'inhibition d'une SREBP chez un sujet.

12. Composé ou sel pharmaceutiquement acceptable de celui-ci pour utilisation selon la revendication 11, dans lequel l'inhibition de la SREBP est destinée à traiter une maladie métabolique, une maladie hépatique, une obésité, un diabète, une maladie cardiovasculaire, ou un cancer chez le sujet.

13. Composé ou sel pharmaceutiquement acceptable de celui-ci pour utilisation selon la revendication 12, dans lequel l'inhibition de la SREBP est destinée à traiter une obésité, une stéatohépatite non alcoolique (NASH), une stéatose hépatique, ou un cancer chez le sujet.
